# EUROPEAN PATENT APPLICATION

(11) **EP 1 829 980 A1**
(43) Date of publication of application: **05.09.2007**
(21) Application number: 07250838.5
(22) Date of filing: 28.02.2007
(51) Int. Cl.: C12Q 1/68

(54) **Detection of lymph node metastasis from gastric carcinoma**

(30) Priority: 01.03.2006 US 365630
(71) Applicant: Veridex, LLC, Warren, NJ 07059 (US)
(72) Inventor: Xu, Ming, Berkeley Heights, NJ 07922 (US); Markiewicz, Jadwiga, Kearny, NJ 07032 (US); Zieba, Renata T., Garfield, NJ 07026 (US); Green, George, Newton, NJ 07860 (US); Cao, Sean Wuxiong, Three Bridges, NJ 08887 (US)
(74) Representative: Mercer, Christopher Paul

(57) **Abstract**

Methods, compositions and articles are directed to diagnosing the lymph node metastasis from gastric carcinoma, differentiating between negative lymph nodes and lymph nodes with metastasis from gastric carcinoma, testing tissue samples of for determining gastric carcinoma staging status.

### Annex to the application documents - subsequently filed sequences listing

SEQUENCE LISTING

<110>  Ming, Xu

<120>  DETECTION OF LYMPH NODE METASTASIS FROM GASTRIC CARCINOMA

<130>  VDX5032USNP

<160>  39

<170>  PatentIn version 3.3

<210>  1
<211>  1339
<212>  DNA
<213>  Homo sapiens

<220>
<221>  misc_feature
<223>  Gene ID: ANXA3

<400>  1
gaattccgat tagtgtgatc tcagctcaag gcaaaggtgg gatatcatgg catctatctg      60
ggttggacac cgaggaacag taagagatta tccagacttt agcccatcag tggatgctga     120
agctattcag aaagcaatca gaggaattgg aactgatgag aaaatgctca tcagcattct     180
gactgagagg tcaaatgcac agcggcagct gattgttaag gaatatcaag cagcatatgg     240
aaaggagctg aaagatgact tgaagggtga tctctctggc cactttgagc atctcatggt     300
ggccctagtg actccaccag cagtctttga tgcaaagcag ctaaagaaat ccatgaaggg     360
cgcgggaaca aacgaagatg ccttgattga aatcttaact accaggacaa gcaggcaaat     420
gaaggatatc tctcaagcct attatacagt atacaagaag agtcttggag atgacattag     480
ttccgaaaca tctggtgact tccggaaagc tctgttgact ttggcagatg gcagaagaga     540
tgaaagtctg aaagtggatg agcatctggc caaacaagat gcccagattc tctataaagc     600
tggtgagaac agatggggca cggatgaaga caaattcact gagatcctgt gtttaaggag     660
ctttcctcaa ttaaaactaa catttgatga atacagaaat atcagccaaa aggacattgt     720
ggacagcata aaaggagaat tatctgggca ttttgaagac ttactgttgg ccatagttaa     780
ttgtgtgagg aacacgccgg cctttttagc cgaaagactg catcgagcct tgaagggtat     840
tggaactgat gagtttactc tgaaccgaat aatggtgtcc agatcagaaa ttgacctttt     900
ggacattcga acagagttca agaagcatta tggctattcc ctatattcag caattaaatc     960
ggatacttct ggagactatg aaatcacact cttaaaaatc tgtggtggag atgactgaac    1020
caagaagata atctccaaag gtccacgatg ggctttccca acagctccac cttacttctt    1080
ctcatactat ttaagagaac aagcaaatat aaacagcaac ttgtgttcct aacaggaatt    1140
ttcattgttc tataacaaca acaacaaaag cgattattat tttagagcat ctcatttata    1200
atgtagcagc tcataaatga aattgaaaat ggtattaaag atctgcaact actatccaac    1260
ttatatttct gctttcaaag ttaagaatct ttatagttct actccattaa atataaagca    1320
agataataaa acggaattc                                                 1339

<210>  2
<211>  1749
<212>  DNA
<213>  Homo sapiens

<220>
<221>  misc_feature
<223>  Gene ID:  AP1M2

<400>  2
ggcgcttccg caggaagaag gaagcggcgc cgccatcgcc tcccggcgct ccctccccga      60
ctcctaagtc cttcggccgc caccatgtcc gcctcggctg tcttcattct ggacgttaag     120
ggcaagccat tgatcagccg caactacaag ggcgatgtgg ccatgagcaa gattgagcac     180
ttcatgcctt tgctggtaca gcgggaggag gaaggcgccc tggccccgct gctgagccac     240
ggccaggtcc acttcctatg gatcaaacac agcaacctct acttggtggc caccacatcg     300
aagaatgcca atgcctccct ggtgtactcc ttcctgtata agacaataga ggtattctgc     360
gaatacttca aggagctgga ggaggagagc atccgggaca actttgtcat cgtctacgag     420
ttgctggacg agctcatgga ctttggcttc ccgcagacca ccgacagcaa gatcctgcag     480
gagtacatca ctcagcagag caacaagctg gagacgggca agtcacgggt gccacccact     540
gtcaccaacg ctgtgtcctg gcgctccgag ggtatcaagt ataagaagaa cgaggtcttc     600
attgatgtca tagagtctgt caacctgctg gtcaatgcca acggcagcgt ccttctgagc     660
gaaatcgtcg gtaccatcaa gctcaaggtg tttctgtcag gaatgccaga gctgcggctg     720
ggcctcaatg accgcgtgct cttcgagctc actggccgca gcaagaacaa atcagtagag     780
ctggaggatg taaaattcca ccagtgcgtg cggctctctc gctttgacaa cgaccgcacc     840
atctccttca tcccgcctga tggtgacttt gagctcatgt cataccgcct cagcacccag     900
gtcaagccac tgatctggat tgagtctgtc attgagaagt tctcccacag ccgcgtggag     960
atcatggtca aggccaaggg gcagtttaag aaacagtcag tggccaacgg tgtggagata    1020
tctgtgcctg tacccagcga tgccgactcc cccagattca agaccagtgt gggcagcgcc    1080
aagtatgtgc cggagagaaa cgtcgtgatt tggagtatta agtctttccc ggggggcaag    1140
gagtacttga tgcgagccca ctttggcctc cccagtgtgg aaaaggaaga ggtggagggc    1200
cggcccccca tcggggtcaa gtttgagatc ccctacttca ccgtctctgg gatccaggtc    1260
cgatacatga agatcattga gaaaagtggt taccaggccc tgccctgggt tcgctacatc    1320
acccagagtg gcgattacca acttcgtacc agctagaagg gagaagagat gggggcttga    1380
acacggggct tccttacagc cccggatgca gattttagag ggagggcagg tgcgggctgt    1440
gtgtgtctgt gtgagggcag gtcctggact tggcagtttc ttgctcccag cacccgcccc    1500
ttcctcacct cttccttatt ccataggctg ggagagaaac tctctgcttc cctcgccctt    1560
ggagctttcc ccatccccct gattttatat gaagaaatag aagaggggct tgaagtcccc    1620
ctcgcgagtg ccttcttgca attacctgcc ttagcgggtg ttgcgggtcc ctccttcaca    1680
gccgctgagc ccagaggtcc cgctggcccc tcctctgaat tttaggatgt cattaaaaag    1740
atgaatcta                                                            1749

<210>  3
<211>  1568
<212>  DNA
<213>  Homo sapiens

<220>
<221>  misc_feature
<223>  Gene ID: ATP1B1

<400>  3
cagcggcgcg tcctgcctgc agagagccag gccggagaag ccgagcggcg cagaggacgc      60
cagggcgcgc gccgcagcca cccaccctcc ggaccgcggc agctgctgac ccgccatcgc     120
catggcccgc gggaaagcca aggaggaggg cagctggaag aaattcatct ggaactcaga     180
gaagaaggag tttctgggca ggaccggtgg cagttggttt aagatccttc tattctacgt     240
aatattttat ggctgcctgg ctggcatctt catcggaacc atccaagtga tgctgctcac     300
catcagtgaa tttaagccca catatcagga ccgagtggcc ccgccaggat taacacagat     360
tcctcagatc cagaagactg aaatttcctt tcgtcctaat gatcccaaga gctatgaggc     420
atatgtactg aacatagtta ggttcctgga aaagtacaaa gattcagccc agaggatgga     480
catgattttt gaagattgtg gcgatgtgcc cagtgaaccg aaagaacgag gagactttaa     540
tcatgaacga ggagagcgaa aggtctgcag attcaagctt gaatggctgg gaaattgctc     600
tggattaaat gatgaaactt atggctacaa agagggcaaa ccgtgcatta ttataaagct     660
caaccgagtt ctaggcttca aacctaagcc tcccaagaat gagtccttgg agacttaccc     720
agtgatgaag tataacccaa atgtccttcc cgttcagtgc actggcaagc gagatgaaga     780

taaggataaa gttggaaatg tggagtattt tggactgggc aactcccctg gttttcctct 840
gcagtattat ccgtactatg gcaaactcct gcagcccaaa tacctgcagc ccctgctggc 900
cgtacagttc accaatctta ccatggacac tgaaattcgc atagagtgta aggcgtacgg 960
tgagaacatt gggtacagtg agaaagaccg ttttcaggga cgttttgatg taaaaattaa 1020
attttaagtg acactacaga aaaacacaaa aaggtgatgg gttgtgttat gcttgtattg 1080
aatgctgtct tgacatctct tgccttgtcc tccggtatgt tctaaagctg tgtctgagat 1140
ctggatctgc ccatcacttt ggctagtgac agggctaatt aatttgcttt atacattttc 1200
ttttactttc cttttttcct ttctggaggc atcacatgct ggtgctgtgt ctttatgaat 1260
gttttaacca ttttcatggt ggaagaattt tatatttatg cagttgtaca attttatttt 1320
tttctgcaag aaaaagtgta atgtatgaaa taaaccaaag tcacttgttt gaaaataaat 1380
ctttattttg aactttataa aaagcaatgc agtaccccat agactggtgt taaatgttgt 1440
ctacagtgca aaatccatgt tctaacatat gtaataattg ccaggagtac agtgctcttg 1500
ttgatcttgt attcagtcag gttaaaacaa cggacaataa aagaatgaac acattcaaaa 1560
aaaaaaaa 1568

<210> 4
<211> 5511
<212> DNA
<213> Homo sapiens

<220>
<221> misc_feature
<223> Gene ID: C11orf9

<400> 4
   acacacacac acccctcccc tgccatccct ccccggactc cggctccggc tccgattgca 60
   atttgcaacc tccgctgccg tcgccgcagc agccaccaat tcgccagcgg ttcaggtggc 120
   tcttgcctcg atgtcctagc ctagggcccc ccgggccgga cttggctggg ctcccttcac 180
   cctctgcgga gtcatgaggg cgaacgacgc tctgcaggtg ctgggcttgc ttttcagcct 240
   ggcccggggc tccgaggtgg gcaactctca ggcagtgtgt cctgggactc tgaatggcct 300
   gagtgtgacc ggcgatgctg agaaccaata ccagacactg tacaagctct acgagaggtg 360
   tgaggtggtg atggggaacc ttgagattgt gctcacggga cacaatgccg acctctcctt 420
   cctgcagtgg attcgagaag tgacaggcta tgtcctcgtg gccatgaatg aattctctac 480
   tctaccattg cccaacctcc gcgtggtgcg agggacccag gtctacgatg ggaagtttgc 540
   catcttcgtc atgttgaact ataacaccaa ctccagccac gctctgcgcc agctccgctt 600
   gactcagctc accgagattc tgtcaggggg tgtttatatt gagaagaacg ataagctttg 660
   tcacatggac acaattgact ggagggacat cgtgagggac cgagatgctg agatagtggt 720
   gaaggacaat ggcagaagct gtcccccctg tcatgaggtt tgcaaggggc gatgctgggg 780
   tcctggatca gaagactgcc agacattgac caagaccatc tgtgctcctc agtgtaatgg 840
   tcactgcttt gggcccaacc ccaaccagtg ctgccatgat gagtgtgccg ggggctgctc 900
   aggccctcag gacacagact gctttgcctg ccggcacttc aatgacagtg gagcctgtgt 960
   acctcgctgt ccacagcctc ttgtctacaa caagctaact ttccagctgg aacccaatcc 1020
   ccacaccaag tatcagtatg gaggagtttg tgtagccagc tgtccccata actttgtggt 1080
   ggatcaaaca tcctgtgtca gggcctgtcc tcctgacaag atggaagtag ataaaaatgg 1140
   gctcaagatg tgtgagcctt gtgggggact atgtcccaaa gcctgtgagg gaacaggctc 1200
   tgggagccgc ttccagactg tggactcgag caacattgat ggatttgtga actgcaccaa 1260
   gatcctgggc aacctggact ttctgatcac cggcctcaat ggagacccct ggcacaagat 1320
   ccctgccctg gacccagaga agctcaatgt cttccggaca gtacgggaga tcacaggtta 1380
   cctgaacatc cagtcctggc cgccccacat gcacaacttc agtgtttttt ccaatttgac 1440
   aaccattgga ggcagaagcc tctacaaccg gggcttctca ttgttgatca tgaagaactt 1500
   gaatgtcaca tctctgggct tccgatccct gaaggaaatt agtgctgggc gtatctatat 1560
   aagtgccaat aggcagctct gctaccacca ctctttgaac tggaccaagg tgcttcgggg 1620
   gcctacggaa gagcgactag acatcaagca taatcggccg cgcagagact gcgtggcaga 1680
   gggcaaagtg tgtgacccac tgtgctcctc tgggggatgc tggggcccag gccctggtca 1740
   gtgcttgtcc tgtcgaaatt atagccgagg aggtgtctgt gtgacccact gcaactttct 1800
   gaatggggag cctcgagaat ttgcccatga ggccgaatgc ttctcctgcc acccggaatg 1860
   ccaacccatg gagggcactg ccacatgcaa tggctcgggc tctgatactt gtgctcaatg 1920
   tgcccatttt cgagatgggc cccactgtgt gagcagctgc ccccatggag tcctaggtgc 1980
   caagggccca atctacaagt acccagatgt tcagaatgaa tgtcggccct gccatgagaa 2040
   ctgcacccag gggtgtaaag gaccagagct tcaagactgt ttaggacaaa cactggtgct 2100
   gatcggcaaa acccatctga caatggcttt gacagtgata gcaggattgg tagtgatttt 2160
   catgatgctg ggcggcactt ttctctactg gcgtgggcgc cggattcaga ataaaagggc 2220
   tatgaggcga tacttggaac ggggtgagag catagagcct ctggacccca gtgagaaggc 2280
   taacaaagtc ttggccagaa tcttcaaaga gacagagcta aggaagctta aagtgcttgg 2340
   ctcgggtgtc tttggaactg tgcacaaagg agtgtggatc cctgagggtg aatcaatcaa 2400
   gattccagtc tgcattaaag tcattgagga caagagtgga cggcagagtt ttcaagctgt 2460
   gacagatcat atgctggcca ttggcagcct ggaccatgcc cacattgtaa ggctgctggg 2520
   actatgccca gggtcatctc tgcagcttgt cactcaatat ttgcctctgg gttctctgct 2580
   ggatcatgtg agacaacacc ggggggcact ggggccacag ctgctgctca actggggagt 2640
   acaaattgcc aagggaatgt actaccttga ggaacatggt atggtgcata gaaacctggc 2700
   tgcccgaaac gtgctactca agtcacccag tcaggttcag gtggcagatt ttggtgtggc 2760
   tgacctgctg cctcctgatg ataagcagct gctatacagt gaggccaaga ctccaattaa 2820
   gtggatggcc cttgagagta tccactttgg gaaatacaca caccagagtg atgtctggag 2880
   ctatggtgtg acagtttggg agttgatgac cttcggggca gagccctatg cagggctacg 2940
   attggctgaa gtaccagacc tgctagagaa gggggagcgg ttggcacagc cccagatctg 3000
   cacaattgat gtctacatgg tgatggtcaa gtgttggatg attgatgaga acattcgccc 3060
   aacctttaaa gaactagcca atgagttcac caggatggcc cgagacccac cacggtatct 3120
   ggtcataaag agagagagtg ggcctggaat agcccctggg ccagagcccc atggtctgac 3180
   aaacaagaag ctagaggaag tagagctgga gccagaacta gacctagacc tagacttgga 3240
   agcagaggag gacaacctgg caaccaccac actgggctcc gccctcagcc taccagttgg 3300
   aacacttaat cggccacgtg ggagccagag ccttttaagt ccatcatctg gatacatgcc 3360
   catgaaccag ggtaatcttg gggagtcttg ccaggagtct gcagtttctg ggagcagtga 3420
   acggtgcccc cgtccagtct ctctacaccc aatgccacgg ggatgcctgg catcagagtc 3480
   atcagagggg catgtaacag gctctgaggc tgagctccag gagaaagtgt caatgtgtag 3540
   gagccggagc aggagccgga gcccacggcc acgcggagat agcgcctacc attcccagcg 3600
   ccacagtctg ctgactcctg ttaccccact ctccccaccc gggttagagg aagaggatgt 3660
   caacggttat gtcatgccag atacacacct caaaggtact ccctcctccc gggaaggcac 3720
   cctttcttca gtgggtctca gttctgtcct gggtactgaa gaagaagatg aagatgagga 3780
   gtatgaatac atgaaccgga ggagaaggca cagtccacct catcccccta ggccaagttc 3840
   ccttgaggag ctgggttatg agtacatgga tgtggggtca gacctcagtg cctctctggg 3900
   cagcacacag agttgcccac tccaccctgt acccatcatg cccactgcag gcacaactcc 3960
   agatgaagac tatgaatata tgaatcggca acgagatgga ggtggtcctg ggggtgatta 4020
   tgcagccatg ggggcctgcc cagcatctga gcaagggtat gaagagatga gagcttttca 4080
   ggggcctgga catcaggccc cccatgtcca ttatgcccgc ctaaaaactc tacgtagctt 4140
   agaggctaca gactctgcct ttgataaccc tgattactgg catagcaggc ttttccccaa 4200
   ggctaatgcc cagagaacgt aactcctgct ccctgtggca ctcagggagc atttaatggc 4260
   agctagtgcc tttagagggt accgtcttct ccctattccc tctctctccc aggtcccagc 4320
   cccttttccc cagtcccaga caattccatt caatctttgg aggcttttaa acattttgac 4380
   acaaaattct tatggtatgt agccagctgt gcactttctt ctctttccca accccaggaa 4440
   aggttttcct tattttgtgt gctttcccag tcccattcct cagcttcttc acaggcactc 4500
   ctggagatat gaaggattac tctccatatc ccttcctctc aggctcttga ctacttggaa 4560
   ctaggctctt atgtgtgcct ttgtttccca tcagactgtc aagaagagga aagggaggaa 4620
   acctagcaga ggaaagtgta attttggttt atgactctta accccctaga aagacagaag 4680
   cttaaaatct gtgaagaaag aggttaggag tagatattga ttactatcat aattcagcac 4740
   ttaactatga gccaggcatc atactaaact tcacctacat tatctcactt agtcctttat 4800
   catccttaaa acaattctgt gacatacata ttatctcatt ttacacaaag ggaagtcggg 4860
   catggtggct catgcctgta atctcagcac tttgggaggc tgaggcagaa ggattacctg 4920
   aggcaaggag tttgagacca gcttagccaa catagtaaga cccccatctc tttaaaaaaa 4980
   aaaaaaaaaa aaaaaaaaaa actttagaac tgggtgcagt ggctcatgcc tgtaatccca 5040
   gccagcactt tgggaggctg agatggaaag atcacttgag cccagaatta gagataagcc 5100
   tatggaaaca tagcaagaca ctgtctctac aggggaaaaa aaaaaaagaa actgagcctt 5160
   aaagagatga aataaattaa gcagtagatc caggatgcaa aatcctccca attcctgtgc 5220
   atgtgctctt attgtaaggt gccaagaaaa actgatttaa gttacagccc ttgtttaagg 5280
   ggcactgttt cttgtttttg cactgaatca agtctaaccc caacagccac atcctcctat 5340
   acctagacat ctcatctcag gaagtggtgg tgggggtagt cagaaggaaa aataactgga 5400
   catctttgtg taaaccataa tccacatgtg ccgtaaatga tcttcactcc ttatccgagg 5460
   gcaaattcac aaggatcccc aagatccact tttagaagcc attctcatcc a 5511

<210> 5
<211> 4098
<212> DNA
<213> Homo sapiens

<220>
<221> misc_feature
<223> Gene ID: C1orf106

<400> 5
atgctgcaaa tgccgaagtt aaatgaaata cctccgggga gggcaggccg cagggaggct 60
cggggggagg gaagatggcc tggacaaaca ggtcctgaag ctgcgaggct ggagtggagg 120
gcgcaggggc aggcgggcgg cgccagagct ccatgggaca gctggggaag ctccaggcta 180
cctacacaac ctggcccagg ctggtcacgg tgtcccccct ccctgctctg tgccctctcc 240
ttccagaaat ccaccatgga gagtaaggat gaggtcagcg acaccgacag tggcatcatc 300
ctgcagtctg gccccgacag cccggtctcc ccaatgaagg agctgaccca tgcagtgcac 360
aagcagcaga gggccctgga agcgaggctg gaggcctgcc tggaggagct gaggagactc 420
tgccttcggg aagcggagct gacgggcacc ttgccagcgg agtatcccct caaaccaggg 480
gaaaaggccc ccaaggttcg ccgcaggatc ggagcggctt acaaactgga tgactgggcc 540
ttgcacagag aggaccccct aagcagcctg gagcgccagc tggccctgca gctgcagatc 600
acagaggcag cccgtcggct gtgcctggag gagaacctca gcaggcaggc tcggcggcag 660
cggaagcact ccatgctgca ggaggagaag aagctgcagg agctccagcg ctgcctggtc 720
gagcggcggc gcaatagcga gccacctccg gctgctgctc tccccctggg ccgagagctc 780
agtgcctctg atgacagctc cctgtcagat gggctcctcc tggaggaaga ggaatcccaa 840
gtgccaaaac ctcctccaga gtctccagcc ccaccttctc ggcctctccc accccaaacc 900
cttgagggtc tgcagccaac aggacctgag gctgggagcc cagaacgggc tccagtccag 960
aacagcccct ggaaggaaac cagcctggac cacccctatg agaagcccag gaagtcttct 1020
gagccctgga gcgagtccag cagcccagcc accacaccac aggatgggcc cagtgcctcc 1080
agcctgtggc ttctggagcc tgcctcctac cacgtggttc ccatccgtgg tgttcctggc 1140
cagtggcagg gccgcaccag tgccccagcc acccctgaga tacaggggag gaggggccag 1200
tcgcagtctc tgagggtgga ttccttccgg gcgggtcctg agggccgagg tcgcagcgcc 1260
tttccccgcc gccgccccac tcactacacg gtgacagtgc cagattcctg ctttcccgcg 1320
accaagcccc cgctgcccca cgccgcctgc cactcctgct cagaagacag tggctctgac 1380
gtctccagca tctcccaccc cacttcgccg ggcagcagca gccccgacat ctcctttctg 1440
cagcctctct cccctcccaa gacccatcgt caccgcgggg cctgggtccc agccggcagc 1500
agagagctgg tcgcccacca ccccaagcta ctgctgccgc ctggctattt cccggcgggg 1560
cggtacgtgg tggtggctga gagccccctg ccgcctggcg agtgggagct gtgccgcgca 1620
gccccgggcc ctgcttacga ggaggagggc actcccctgc gctaccagcg tctggtgccc 1680
tcccgcagcc gcatcgtgcg gacgccctcc ctgaaggaca gcccggcagg ccgggggctc 1740
agcaaggccg ccgtgtccga ggagctcaag tggtggcacg agcgtgcacg cctccggagc 1800
acccgacccc actcactgga ccgccaagga gctttccggg tcaggagcct gccccttggg 1860
agagagggct tcggacgagc cctgggaccc cgggcacagg tgcccacagt ttgtgtgctg 1920
tggagatcgc ctgatggggc ccctgtgcaa gtctttgtac ctgaaaaagg agagatcatc 1980
agccaggtgt aactctgcgc cccacgctgg aaaaaactgt ttcatagagg ggctgggctg 2040
agaccccccc acccctgagt gcctctttca gctcccccat ccccatcgca ggccgatgac 2100
ctggagctaa gaccttttat tatttttttt ttacacgact tttttcagaa gccctgacct 2160
aaggatttat atatgtggat tgtcctcaat acccctgtga tatgattatg ttttatcccc 2220

cagagtttgg cctactggac ttaaggcctt gcctgtctga ctgacagcct ctatctcctt 2280
atataagaca agtggcaggg gacgagtgaa gcagagtgag ccaccttggg agttctccaa 2340
cgctctgtgc tctggttcta agaaattccc tggggaactg cccctggccc tcctgtccca 2400
ctattgctgg aggctggaca tggtacatac tcatgcacat gactctcccc catttcccag 2460
gtctctgggt accccagcct gggctggggg agaatctctt cccccttttc taatgtgctc 2520
tgtgatgcac acaccaagtg gtaggtcaaa ggtcagtata tcccggtggt gtattgtctt 2580
gctagaccct gctattttcc tgacccccta aatcctcttt agggacccag tcactatacc 2640
ctgtctatgc cctgtgggct cccagacccc tgagctttga gtcagtggca tcacagtttg 2700
tagcctcagg gggtctggct gggggctggt ccatgcttgt ggttagtgga cagcagccac 2760
cctttgacag ctacctctgg acatctcaag ggcttgcagc cccactgctc cttctaacat 2820
tttgtttgtt tttgagatgg agtctcgctc tgtcgcccag gctggaatgc tgtagcaaga 2880
tttcggctca ctgcaacccc cgtctcccgg gttcaagcga atctcctgcc tcagccttct 2940
gagtagctgg gattacaggc aagcaccacc atgcctgcta atttttattt tttagtagag 3000
atggggtttc atcatgttgg ccaggctggt ctcgaactcc tgacctcaag tgatccacct 3060
gccttggcct ttcaaagtgc tgggattaca ggcatgagcc accgcacctg gcccttctaa 3120
cgttttttca tcatagtccc aaaaaccaat actttacaag tggtttttga aaggcaccac 3180
ttttgtggca tgttctggtt gggagaggga gtcacagttc ctactccccc caccagctat 3240
gcttctgctc tgagaaggtg gttatttata caaacatgga catactcact cccaagggct 3300
gatgagatgc tgaattttct ttgggggcat tcattaattg tcccagctgc agcgactgga 3360
gcaagtctgg aagctgcctg tgctaagacc acccagctgt ccctgggttc tcatcctagg 3420
gccttctttg cttccaggtc aggggacctg cttcaatgag aaagcaactg aattgaggct 3480
aggagaggta gggagagctg agttctgact tcacctgtgc agaactctct gcccccatgt 3540
tacctggact ggaacagact gtgaatatag cagaaggttc caagaactct ggtgtctgac 3600
ctagaagagg cacagttctc tctactggaa agaaaacgat gtagccgatt gcacaagggt 3660
gccaagggaa gacccaggat ggcccatcaa aggaacctgg gggaggatgc aggaggctga 3720
agggatgcac ctggcatttc tctcactgtg ctcttaccgc atcagcaacc cccaactttt 3780
gggcctactc tgccccccat gcgtgaatac cctgcttgga tgctgtgctt ttccggtttg 3840
tctctaagcc cctttctcca gggcatgttg gtttccctgg cctctcagtg tcctaactgg 3900
agcccagagt gccttgttct gagccaggag acggctgagc actggccctc cacacctaag 3960
cgtcctttac attaacttat tggtcttgta taacacctgg tgccattgcc aagtggctgt 4020
gtcctcagct acagagctgg aattgtgtgg ggtttagtgc taaatacttc aataaagtct 4080
gtttttgtg attggctg 4098

<210> 6
<211> 4828
<212> DNA
<213> Homo sapiens

<220>
<221> misc_feature
<223> Gene ID: CDH1

<400> 6
agtggcgtcg gaactgcaaa gcacctgtga gcttgcggaa gtcagttcag actccagccc 60
gctccagccc ggcccgaccc gaccgcaccc ggcgcctgcc ctcgctcggc gtccccggcc 120
agccatgggc ccttggagcc gcagcctctc ggcgctgctg ctgctgctgc aggtctcctc 180
ttggctctgc caggagccgg agccctgcca ccctggcttt gacgccgaga gctacacgtt 240
cacggtgccc cggcgccacc tggagagagg ccgcgtcctg ggcagagtga attttgaaga 300
ttgcaccggt cgacaaagga cagcctattt ttccctcgac acccgattca aagtgggcac 360
agatggtgtg attacagtca aaaggcctct acggtttcat aacccacaga tccatttctt 420
ggtctacgcc tgggactcca cctacagaaa gttttccacc aaagtcacgc tgaatacagt 480
ggggcaccac caccgccccc cgccccatca ggcctccgtt tctggaatcc aagcagaatt 540
gctcacattt cccaactcct ctcctggcct cagaagacag aagagagact gggttattcc 600
tcccatcagc tgcccagaaa atgaaaaagg cccatttcct aaaaacctgg ttcagatcaa 660
atccaacaaa gacaaagaag gcaaggtttt ctacagcatc actggccaag gagctgacac 720

accccctgtt ggtgtcttta ttattgaaag agaaacagga tggctgaagg tgacagagcc 780
tctggataga gaacgcattg ccacatacac tctcttctct cacgctgtgt catccaacgg 840
gaatgcagtt gaggatccaa tggagatttt gatcacggta accgatcaga atgacaacaa 900
gcccgaattc acccaggagg tctttaaggg gtctgtcatg gaaggtgctc ttccaggaac 960
ctctgtgatg gaggtcacag ccacagacgc ggacgatgat gtgaacacct acaatgccgc 1020
catcgcttac accatcctca gccaagatcc tgagctccct gacaaaaata tgttcaccat 1080
taacaggaac acaggagtca tcagtgtggt caccactggg ctggaccgag agagtttccc 1140
tacgtatacc ctggtggttc aagctgctga ccttcaaggt gaggggttaa gcacaacagc 1200
aacagctgtg atcacagtca ctgacaccaa cgataatcct ccgatcttca atcccaccac 1260
gtacaagggt caggtgcctg agaacgaggc taacgtcgta atcaccacac tgaaagtgac 1320
tgatgctgat gcccccaata ccccagcgtg ggaggctgta tacaccatat tgaatgatga 1380
tggtggacaa tttgtcgtca ccacaaatcc agtgaacaac gatggcattt tgaaaacagc 1440
aaagggcttg gattttgagg ccaagcagca gtacattcta cacgtagcag tgacgaatgt 1500
ggtacctttt gaggtctctc tcaccacctc cacagccacc gtcaccgtgg atgtgctgga 1560
tgtgaatgaa gcccccatct ttgtgcctcc tgaaaagaga gtggaagtgt ccgaggactt 1620
tggcgtgggc caggaaatca catcctacac tgcccaggag ccagacacat ttatggaaca 1680
gaaaataaca tatcggattt ggagagacac tgccaactgg ctggagatta atccggacac 1740
tggtgccatt tccactcggg ctgagctgga cagggaggat tttgagcacg tgaagaacag 1800
cacgtacaca gccctaatca tagctacaga caatggttct ccagttgcta ctggaacagg 1860
gacacttctg ctgatcctgt ctgatgtgaa tgacaacgcc cccataccag aacctcgaac 1920
tatattcttc tgtgagagga atccaaagcc tcaggtcata aacatcattg atgcagacct 1980
tcctcccaat acatctccct tcacagcaga actaacacac ggggcgagtg ccaactggac 2040
cattcagtac aacgacccaa cccaagaatc tatcattttg aagccaaaga tggccttaga 2100
ggtgggtgac tacaaaatca atctcaagct catggataac cagaataaag accaagtgac 2160
caccttagag gtcagcgtgt gtgactgtga aggggccgcc ggcgtctgta ggaaggcaca 2220
gcctgtcgaa gcaggattgc aaattcctgc cattctgggg attcttggag gaattcttgc 2280
tttgctaatt ctgattctgc tgctcttgct gtttcttcgg aggagagcgg tggtcaaaga 2340
gcccttactg cccccagagg atgacacccg ggacaacgtt tattactatg atgaagaagg 2400
aggcggagaa gaggaccagg actttgactt gagccagctg cacaggggcc tggacgctcg 2460
gcctgaagtg actcgtaacg acgttgcacc aaccctcatg agtgtccccc ggtatcttcc 2520
ccgccctgcc aatcccgatg aaattggaaa ttttattgat gaaaatctga aagcggctga 2580
tactgacccc acagccccgc cttatgattc tctgctcgtg tttgactatg aaggaagcgg 2640
ttccgaagct gctagtctga gctccctgaa ctcctcagag tcagacaaag accaggacta 2700
tgactacttg aacgaatggg gcaatcgctt caagaagctg gctgacatgt acggaggcgg 2760
cgaggacgac taggggactc gagagaggcg ggccccagac ccatgtgctg ggaaatgcag 2820
aaatcacgtt gctggtggtt tttcagctcc cttcccttga gatgagtttc tggggaaaaa 2880
aaagagactg gttagtgatg cagttagtat agctttatac tctctccact ttatagctct 2940
aataagtttg tgttagaaaa gtttcgactt atttcttaaa gctttttttt ttttcccatc 3000
actctttaca tggtggtgat gtccaaaaga tacccaaatt ttaatattcc agaagaacaa 3060
ctttagcatc agaaggttca cccagcacct tgcagatttt cttaaggaat tttgtctcac 3120
ttttaaaaag aaggggagaa gtcagctact ctagttctgt tgttttgtgt atataatttt 3180
ttaaaaaaaa tttgtgtgct tctgctcatt actacactgg tgtgtccctc tgcctttttt 3240
ttttttttta agacagggtc tcattctatc ggccaggctg gagtgcagtg gtgcaatcac 3300
agctcactgc agccttgtcc tcccaggctc aagctatcct tgcacctcag cctcccaagt 3360
agctgggacc acaggcatgc accactacgc atgactaatt ttttaaatat ttgagacggg 3420
gtctccctgt gttacccagg ctggtctcaa actcctgggc tcaagtgatc ctcccatctt 3480
ggcctcccag agtattggga ttacagacat gagccactgc acctgcccag ctccccaact 3540
ccctgccatt ttttaagaga cagtttcgct ccatcgccca ggcctgggat gcagtgatgt 3600
gatcatagct cactgtaacc tcaaactctg gggctcaagc agttctccca ccagcctcct 3660
ttttattttt ttgtacagat ggggtcttgc tatgttgccc aagctggtct taaactcctg 3720
gcctcaagca atccttctgc cttggccccc caaagtgctg ggattgtggg catgagctgc 3780
tgtgcccagc ctccatgttt taatatcaac tctcactcct gaattcagtt gctttgccca 3840
agataggagt tctctgatgc agaaattatt gggctctttt agggtaagaa gtttgtgtct 3900
ttgtctggcc acatcttgac taggtattgt ctactctgaa gacctttaat ggcttccctc 3960
tttcatctcc tgagtatgta acttgcaatg ggcagctatc cagtgacttg ttctgagtaa 4020

gtgtgttcat taatgtttat ttagctctga agcaagagtg atatactcca ggacttagaa 4080
tagtgcctaa agtgctgcag ccaaagacag agcggaacta tgaaaagtgg gcttggagat 4140
ggcaggagag cttgtcattg agcctggcaa tttagcaaac tgatgctgag gatgattgag 4200
gtgggtctac ctcatctctg aaaattctgg aaggaatgga ggagtctcaa catgtgtttc 4260
tgacacaaga tccgtggttt gtactcaaag cccagaatcc ccaagtgcct gcttttgatg 4320
atgtctacag aaaatgctgg ctgagctgaa cacatttgcc caattccagg tgtgcacaga 4380
aaaccgagaa tattcaaaat tccaaatttt ttcttaggag caagaagaaa atgtggccct 4440
aaagggggtt agttgagggg tagggggtag tgaggatctt gatttggatc tctttttatt 4500
taaatgtgaa tttcaacttt tgacaatcaa agaaaagact tttgttgaaa tagctttact 4560
gtttctcaag tgttttggag aaaaaaatca accctgcaat cactttttgg aattgtcttg 4620
atttttcggc agttcaagct atatcgaata tagttctgtg tagagaatgt cactgtagtt 4680
ttgagtgtat acatgtgtgg gtgctgataa ttgtgtattt tctttggggg tggaaaagga 4740
aaacaattca agctgagaaa agtattctca aagatgcatt tttataaatt ttattaaaca 4800
attttgttaa accataaaaa aaaaaaaa 4828

<210> 7
<211> 5289
<212> DNA
<213> Homo sapiens

<220>
<221> misc_feature
<223> Gene ID: COBL

<400> 7
ccagcccctt ccgtccgccg cgctccgccg ctgtcctccg ccggcccgcg tgggaggacg 60
atgggtcagc gcggaaaaag tgaatgaggg cggcagcggt ggcggcggta gccagcgcct 120
gactcggcgg gcccggagca ccgcccgcgt cccggccccc ggcaccatgg acgcgccgcg 180
cgcctcggcg gccaagcccc cgaccgggag gaagatgaag gctcgtgctc ccccacctcc 240
tggaaaggct gccactctgc atgtgcacag tgaccagaag cccccccacg atggggccct 300
cgggtcgcag cagaacttgg ttcgcatgaa ggaggcgctg agggccagca ccatggacgt 360
caccgtggtc ctgcctagcg ggctggagaa gaggagcgtg ctcaatggga gccatgcgat 420
gatggaccta ctggttgaac tttgccttca gaaccacctg aatccatccc accatgccct 480
tgaaattcgg tcttcagaaa cccaacaacc tttgagtttt aagccaaata ctttgattgg 540
gaccctgaat gtgcatactg tgtttctgaa agaaaaagtt cctgaagaga aggttaagcc 600
tggtccccct aaggtgcctg agaaatctgt gcgtttggtc gtgaattacc tgcggacaca 660
aaaagctgtt gtgcgtgtga gccctgaggt tcctctccag aatattctcc cagtcatttg 720
tgcaaagtgt gaggtcagcc cagagcacgt ggttctcctc agggacaaca ttgccggaga 780
ggagctggag ctgtccaagt ccctgaacga gctcgggata aaggagctct acgcgtggga 840
caacagaaga gaaaccttta ggaaatcatc acttggcaat gatgagacag ataaagagaa 900
gaaaaaattt ctgggatttt tcaaagttaa taaaagaagc aatagtaagg gctgtttaac 960
gacccccaac tccccatcca tgcactcacg ttctcttacg ctgggtccat ccctctcgct 1020
gggcagcatc tcaggggtgt ccgtgaagtc ggagatgaag aagcgccgag cccctcctcc 1080
tccaggttca gggccacctg tgcaagacaa ggcatcggaa aaggtatctc ttgggtcaca 1140
gattgattta cagaagaaga agcggcgagc gccagctccc cctccaccac agccaccacc 1200
accgagtccc ctgatcccca accgcactga ggataaggag gagaacagga agagcacgat 1260
ggtaagcctg cccctggggt ctggcagcca ctgcagcccg gatggagccc cgcaggtgct 1320
gtcagaggcg gaggagaccg tgtcagttgg cagctgtttt gcgtcggagg acacgaccga 1380
ggactcagga gtgatgagtt ccccctcaga catcgtctct ctggactcgc agcaggacag 1440
catgaaatac aaagacaagt gggccacaga ccaggaagac tgcagtgacc aggacctcgc 1500
tggaacccca gacctgggtc cccagaagag ccccttgtgg gagaagaatg gctctgagaa 1560
ctcacatctg agaactgaaa aagctgtcac agcctcaaat gatgaagaag acctattaat 1620
tgctggagag ttccgtaaaa cccttgcaga acttgatgaa gacctggaag aaatggaaga 1680
cagctatgaa acagacacca gctccctcac cagctccatc catggtgcat ccaaccactg 1740
cccacaggat gccatgatcc ctcacggcga cacagatgca atcccagtaa cattcatagg 1800

ggaagtttca gatgatcctg tggattcggg gttgttttcc aatagaaaca acaatgctgg 1860
gtctttcgac tcggagggtg ttgccagcag gagagactcc ctggcgccac ttcaagctga 1920
gcacagccag ccccatgaaa aggcaaggga ggaagtacct gccctgcacc ccgcttccca 1980
tgacgtcgga aaaggaatcc gtgtggcctt atctaacatc tctaaagatg ggaatctaat 2040
ggaaacggcg cccagggtga cttcttttgc ttcgaatctt cacacagaca acctaaatgc 2100
aaaagtgaaa gacaaagtgt atggctgtgc tgacggggag aggactcaag ccacagagag 2160
agtgaattcc caaccggtga atgaaaagga cagcaacgat aaaaacgctg ccttggcacc 2220
aacatcatgg caccaacgag gccaaaaccc agggaaaagc tacagactta agcacggcct 2280
cacaacgtat aaaatcattc ctccaaagtc agagatgcga tgttacgaca gagatgtgtc 2340
cctctccacc ggagccatta agattgacga gctggggaac ttggtgagtc ctcacgccac 2400
cggcatcagg atcatttccc tgtcttcgtc tgtgcctgaa gcagaatctc agcccattgg 2460
gaaagtcaga gagttctgga ggtgcaactc tgtggaaaaa cacctgggcc gaccctcaga 2520
gagctctgcc aggggacccc cctccacccc tgtgcccacg cagacgcaga atccagagag 2580
cagactccaa gcagacccca agccaatatc gccccagcag aagtctgccc accatgaggg 2640
ccggaacccc ctaggggagg ggagaaacca gccccccacc atgggcatgg gtcacgtgag 2700
ggtgccagca gctcacacca cagaagtcac atttctcaag cctcagagaa gaacgtccag 2760
ccagtatgtg gcctctgcca ttgccaagcg cataggagcc ccaaaagtcc atgctgatgt 2820
ggtgaggcca cacggttatg ccgagaaggg ttatgcaggc aaggcgccag tgctggctgc 2880
accacctgtc actgtgaaag atgacaggac atccagccca cactcagaga cacaaggatg 2940
gaaggatggt gcccagtggc cctgtgtcac tcctcccaac aaccacgggg aagatttggc 3000
agtgggagcc cctcctaggg gggaggtcat tggcccacac aggaagttgt ctactcagga 3060
cagacctgct gctatccaca gaagctcctg tttctcactg gttcagtctt cccagaggga 3120
tcgtgtttct gtgggacaga gctgtggttt cagtggaaag caaagcacaa gtagccagga 3180
ggccagctca gcatctgagc ccagacgcgc acctgatggt acagacccac cccctccaca 3240
cacatctgac actcaggcct gcagcaggga gctggtcaat ggctctgtgc gcgccccagg 3300
ccacggcgag ccttcccacc ctccaggagg gtctggcaca gaaagccaca ttctcctaga 3360
aagagaggaa aagcccagtg tgttctctac agatggaaat gaaacagaca gtatttggcc 3420
acccagcatt tttgggccga agaaaaaatt caaacctgtt gtccagagac cagtcccaaa 3480
agacacatcc ctgcactctg ccctgatgga agccatccac tctgcgggag ggaaggacag 3540
actacgcaag acggcagaac acaccgggga gggcaggcca gcgaaactgt cctacacgga 3600
ggcagagggc gaacgatctg cactgctggc agctatccgc gggcacagcg gcacctgcag 3660
cctgaggaag gtggcatcct ctgcttctga ggagctccag agcttccgag atgccgcact 3720
ctctgctcag ggctcggaaa gtcctctgct agaagacctt ggtcttctgt ccccaccagc 3780
cattccccca ccaccccctc caccctccca ggctctctct gcaccaagga cggcctccag 3840
gttcagcacg ggcaccctca gcaacaccgc agacgcaagg caagccttga tggacgccat 3900
ccgctccggc acaggggctg cgagactgag aaaggtgccc ttgctcgtgt gaatgaacag 3960
aggccagaga attgcagaaa cccacctgta atatgcccac aggcgtcact caaggagtct 4020
gcatagccag gaacatcaag ttttggtgtt actttcttgt ctacaggcca aatgcgcaca 4080
gttgattttc ggtgtgttcc tgtataacgg cttgaaagga aaggagaaga tgctgtgtcg 4140
ctgcgcagct cgtgtgccaa ggaaatgtgt tttgcctctg aatatcttaa gttgctaata 4200
aacgattctt gttggcaggt taatgggact ataaatgctg gagcccagca ctcacactta 4260
attatattta tgggcaaagt aaaaagaatc atgccttaga ctgattttgg acctttgaga 4320
cttttaccac atacagatgg atgaaactac ttactgattt aagatctttt gaattttaga 4380
aaaaacatat gaatcagatg gaacaatctg aatgtgatat gctccatgat gctgtgtgtt 4440
ttggtagtaa gaaaaaaaat tgggaggctt ttcaaaggtg gctttgttag gtagtaaaac 4500
tgctggattc ttcttcctca aatatttggg gctaacactt cacagagggt cacagtccat 4560
gatctgacca actaagcttt taaaaatcaa ctattaccac ccagaaaaca gtcctacgtg 4620
atcatgtggc cagggcatgc ccgagtcctg caagggtggc gggtggctga ggtcacacca 4680
ccactggagt ggatgggcag tgccccacat ggacagccat tccccaggct atgaagtgac 4740
ataggcctcc cccacggtgc ctgtgtacgg agcctgatga cttcactggg agccttctgg 4800
aatcctgcag agggtcaagg agcagggatg ttggatgccc acctgtcaag agttcagatc 4860
aaagttgcgc tgagagcatc acaattttgg ttcagccttg acgcgtttgt ccaacagctc 4920
attggctccc tttgtatgat atcgtggtct tctcacatgg tgcccagtca ccaatattta 4980
taatgaggtc taactacagc agtagttttt catatatatc tctaaaacat tttgttatat 5040
tgaaaaaagt aatagaaatc aagatgtgtt gatgaaataa aatgtgtatc tgagtgagaa 5100

aacaagtatg gtgaggtcac tttaatgttt cacagcgatc tcagatctag gcctcaggta 5160
gaatggaagc tgttctgcat tcactgatta acgttgctaa actcttggtg aggcacgagc 5220
taccagccaa ttgctcttca tcacagctat ctgtctttta gtgccacaaa taaaagaaaa 5280
tcaaatctt 5289

<210> 8
<211> 2080
<212> DNA
<213> Homo sapiens

<220>
<221> misc_feature
<223> Gene ID: CYP3A7

<400> 8
tcactgctgt gcagggcagg aaagctccac acacacagcc cagcaaacag cagcacgctg 60
ctgaaaaaaa gactcagagg agagagataa ggaaggaaag tagtgatgga tctcatccca 120
aacttggccg tggaaacctg gcttctcctg gctgtcagcc tgatactcct ctatctatat 180
ggaacccgta cacatggact ttttaagaag cttggaattc cagggcccac acctctgcct 240
tttttgggaa atgctttgtc cttccgtaag ggctattgga cgtttgacat ggaatgttat 300
aaaaagtata gaaaagtctg ggggtattat gactgtcaac agcctatgct ggctatcaca 360
gatcccgaca tgatcaaaac agtgctagtg aaagaatgtt attctgtctt cacaaaccgg 420
aggcctttcg ggccagtggg atttatgaaa aatgccatct ctatagctga ggatgaagaa 480
tggaagagaa tacgatcatt gctgtctcca acattcacca gcggaaaact caaggagatg 540
gtccctatca ttgcccagta tggagatgtg ttggtgagaa atctgaggcg ggaagcagag 600
acaggcaagc ctgtcacctt gaaacacgtc tttggggcct acagcatgga tgtgatcact 660
agcacatcat ttggagtgag catcgactct ctcaacaatc cacaagaccc ctttgtggaa 720
aacaccaaga agcttttaag atttaatcca ttagatccat tcgttctctc aataaaagtc 780
tttccattcc ttaccccaat tcttgaagca ttaaatatca ctgtgtttcc aagaaaagtt 840
ataagttttc taacaaaatc tgtaaaacag ataaaagaag gtcgcctcaa agagacacaa 900
aagcaccgag tggatttcct tcagctgatg attgactctc agaattcaaa agactctgag 960
acccacaaag ctctgtctga tctggagctc atggcccaat caattatctt tatttttgct 1020
ggctatgaaa ccacgagcag tgttctctcc ttcattatat atgaactggc cactcaccct 1080
gatgtccagc agaaagtgca gaaggaaatt gatacagttt tacccaataa ggcaccaccc 1140
acctatgata ctgtgctaca gttggagtat cttgacatgg tggtgaatga aacactcaga 1200
ttattcccag ttgctatgag acttgagagg gtctgcaaaa aagatgttga aatcaatggg 1260
atgtttattc ccaaaggggt ggtggtgatg attccaagct atgttcttca tcatgaccca 1320
aagtactgga cagagcctga gaagttcctc cctgaaaggt tcagtaaaaa gaacaaggac 1380
aacatagatc cttacatata cacacccttt ggaagtggac ccagaaactg cattggcatg 1440
aggtttgctc tcgtgaacat gaaacttgct ctagtcagag tccttcagaa cttctccttc 1500
aaaccttgta aagaaacaca gatccccctg aaattacgct ttggaggact tcttctaaca 1560
gaaaaaccca ttgttctaaa ggctgagtca agggatgaga ccgtaagtgg agcctgattt 1620
ccctaaggac ttctggtttg ctctttaaga aagctgtgcc ccagaacacc agagacctca 1680
aattacttta caaatagaac cctgaaatga agacgggctt catccaatgt gctgcataaa 1740
taatcaggga ttctgtacgt gcattgtgct ctctcatggt ctgtatagag tgttatactt 1800
ggtaatatag aggagatgac caaatcagtg ctggggaagt agatttggct tctctgcttc 1860
tcataggact atctccacca cccccagtta gcaccattaa ctcctcctga gctctgataa 1920
cataattaac atttctcaat aatttcaacc acaatcatta ataaaaatag gaattatttt 1980
gatggctcta acagtgacat ttatatcatg tgttatatct gtagtattct atagtaagct 2040
ttatattaag caaatcaata aaaacctctt tacaaaagta 2080

<210> 9
<211> 3516
<212> DNA
<213> Homo sapiens

<220>
<221> misc_feature
<223> Gene ID: DSG2

<400> 9
tcgagggtgc gatggcgcgg acgcgggacc gcgtacgcct gctgcttctc ctgatctgct 60
ttaacgttgg aagtggactt cacttacagg tcttaagcac aagaaatgaa aataagctgc 120
ttcctaaaca tcctcattta gtgcggcaaa agcgcgcctg gatcaccgcc cccgtggctc 180
ttcgggaggg agaggatctg tccaagaaga atccaattgc caagatacat tctgatcttg 240
cagaagaaag aggactcaaa attacttaca aatacactgg aaaagggatt acagagccac 300
cttttggtat atttgtcttt aacaaagata ctggagaact gaatgttacc agcattcttg 360
atcgagaaga aacaccattt tttctgctaa caggttacgc tttggatgca agaggaaaca 420
atgtagagaa acccttagag ctacgcatta aggttcttga tatcaatgac aacgaaccag 480
tgttcacaca ggatgtcttt gttgggtctg ttgaagagtt gagtgcagca catactcttg 540
tgatgaaaat caatgcaaca gatgcagatg agcccaatac cctgaattcg aaaatttcct 600
atagaatcgt atctctggag cctgcttatc ctccagtgtt ctacctaaat aaagatacag 660
gagagattta tacaaccagt gttaccttgg acagagagga acacagcagc tacactttga 720
cagtagaagc aagagatggc aatggagaag ttacagacaa acctgtaaaa caagctcaag 780
ttcagattcg tattttggat gtcaatgaca atatacctgt agtagaaaat aaagtgcttg 840
aagggatggt tgaagaaaat caagtcaatg tagaagttac gcgcataaaa gtgttcgatg 900
cagatgaaat aggttctgat aattggctgg caaattttac atttgcatca ggaaatgaag 960
gaggttattt ccacatagaa acagatgctc aaactaacga aggaattgtg acccttatta 1020
aggaagtaga ttatgaagaa atgaagaatc ttgacttcag tgttattgtc gctaataaag 1080
cagcttttca caagtcgatt aggagtaaat acaagcctac acccattccc atcaaggtca 1140
aagtgaaaaa tgtgaaagaa ggcattcatt ttaaaagcag cgtcatctca atttatgtta 1200
gcgagagcat ggatagatca agcaaaggcc aaataattgg aaattttcaa gcttttgatg 1260
aggacactgg actaccagcc catgcaagat atgtaaaatt agaagataga gataattgga 1320
tctctgtgga ttctgtcaca tctgaaatta aacttgcaaa acttcctgat tttgaatcta 1380
gatatgttca aaatggcaca tacactgtaa agattgtggc catatcagaa gattatccta 1440
gaaaaaccat cactggcaca gtccttatca atgttgaaga catcaacgac aactgtccca 1500
cactgataga gcctgtgcag acaatctgtc acgatgcaga gtatgtgaat gttactgcag 1560
aggacctgga tggacaccca aacagtggcc ctttcagttt ctccgtcatt gacaaaccac 1620
ctggcatggc agaaaaatgg aaaatagcac gccaagaaag taccagtgtg ctgctgcaac 1680
aaagtgagaa aaagcttggg agaagtgaaa ttcagttcct gatttcagac aatcagggtt 1740
ttagttgtcc tgaaaagcag gtccttacac tcacagtttg tgaggttctg catggcagcg 1800
gctgcaggga agcacagcat gactcctatg tgggcctggg acccgcagca attgcgctca 1860
tgattttggc ctttctgctc ctgctattgg taccactttt actgctgatg tgccattgcg 1920
gaaagggcgc caaagcgttt acccccatac ctggcaccat agagatgctg catccttgga 1980
ataatgaagg agcaccacct gaagacaagg tggtgccatc atttctgcca gtggatcaag 2040
ggggcagtct agtaggaaga aatggagtag gaggtatggc caaggaagcc acgatgaaag 2100
gaagtagctc tgcttccatt gtcaaagggc aacatgagat gtccgagatg gatggaaggt 2160
gggaagaaca cagaagcctg ctttctggta gagctaccca gtttacaggg gccacaggcg 2220
ctatcatgac cactgaaacc acgaagaccg caagggccac aggggcttcc agagacatgg 2280
ccggagctca ggcagctgct gttgcactga acgaagaatt cttaagaaat tatttcactg 2340
ataaagcggc ctcttacact gaggaagatg aaaatcacac agccaaagat tgccttctgg 2400
tttattctca ggaagaaact gaatcgctga atgcttctat tggttgttgc agttttattg 2460
aaggagagct agatgaccgc ttcttagatg atttgggact taaattcaag acgctagctg 2520
aagtttgcct gggtcaaaaa atagatataa ataaggaaat tgagcagaga caaaaacctg 2580
ccacagaaac aagtatgaac acagcttcac attcactctg tgagcaaact atggttaatt 2640
cagagaatac ctactcctct ggcagtagct tcccagttcc aaaatctttg caagaagcca 2700
atgcagagaa agtaactcag gaaatagtca ctgaaagatc tgtgtcttct aggcaggcgc 2760
aaaaggtagc tacacctctt cctgacccaa tggcttctag aaatgtgata gcaacagaaa 2820
cttcctatgt cacagggtcc actatgccac caaccactgt gatcctgggt cctagccagc 2880
cacagagcct tattgtgaca gagagggtgt atgctccagc ttctaccttg gtagatcagc 2940

cttatgctaa tgaaggtaca gttgtggtca ctgaaagagt aatacagcct catgggggtg 3000
gatcgaatcc tctggaaggc actcagcatc ttcaagatgt accttacgtc atggtgaggg 3060
aaagagagag cttccttgcc cccagctcag gtgtgcagcc tactctggcc atgcctaata 3120
tagcagtagg acagaatgtg acagtgacag aaagagttct agcacctgct tccactctgc 3180
aatccagtta ccagattccc actgaaaatt ctatgacggc taggaacacc acggtgtctg 3240
gagctggagt ccctggccct ctgccagatt ttggtttaga ggaatctggt cattctaatt 3300
ctaccataac cacatcttcc accagagtca ccaagcatag cactgtacag cattcttact 3360
cctaaacagc agtcagccac aaactgaccc agagtttaat tagcagtgac taatttcatg 3420
tttccaatgt acctgatttt tcatgagcct tacagacaca cagagacaca tacacattga 3480
tcttaaaatt tttctcagtc actgatatgc aaagga 3516

<210> 10
<211> 5036
<212> DNA
<213> Homo sapiens

<220>
<221> misc_feature
<223> Gene ID: ELF3

<400> 10
   gtgctgccgt gtgggcaagc tctgtgggga gaggggaggc cagtgggttg tttttgccat 60
   cacagaatac tgggaagccc ctggcatcct gctccatagc tctcttcacc actatcctgg 120
   aaccttctcc ccacccccat ccccatgcct ccaaggcact gacctcaaat ccaagtcttt 180
   cccacttatc tcaagctgcc agcctgtagg gattccttat ctcagctcca tgtcagcggt 240
   gaggaagccc caagaaggca agggagctga cagccttctc atttttctcg tacatcctcc 300
   tgttcacccc gccatcccgg gagccccagc cagatgctct tcagggcagg gagcacgtga 360
   gcagccctgg ggctagaagc cggttctccc acattcctgg gtgagggact gggtggaggg 420
   tgtgcctgcc tcaggctcct tgggggaggc ccctgaaggg ctggggaaaa tcctactgag 480
   ccccaggctc tcctgcctgc actggcccag tgcgggggcg ggggggaggg gggatcctgg 540
   acattaactc tctcctaggc acctgggagc ttcagagagg aagaaggtaa ggtaagaagc 600
   agattctctg cgcaggactt tcttagtcct caggctatag agttaaagaa agaagtatct 660
   gtagaaggtg gagtcacctt ttgtttgagt aaccgtttca cccaccctgg ctgagggtgg 720
   ccaggaactc cccaagttgt gcattaatgg aggtgggtca cacagatcct acattttttt 780
   gtttttgttt tttttttttg agacgcagtg attacaggtg tgagccaccg gatcctacat 840
   ttcaaatgca taaaaatcta gatatgggct gggcgcagta gctcatgcct gtaatcccag 900
   cacattggga ggctgaggca ggcagatcat gaggtcagga gatcgagtcc atcctggcta 960
   acatggtgaa accctgtctc tactaaaaat acaaaaagcc gggcatggca gcgggcgcct 1020
   gtaatcccag ctactcggaa agctgaggca ggagaatcgc ttgaacccag gagtcagagg 1080
   ttgcagtgag cagagatcac gccactgcac tccaacctgg gcgacagagc gagactccac 1140
   ctcaaaacaa aataaacaaa atactagatc tggaagagat cttagggatt attaaattca 1200
   gacaacctca ttttttatag atggggaaac aagcacagac tccaagggcc tcatccaaga 1260
   tcacacagtt gcagatgctg gctacaagtc tcctgcctca accacctgta ttaccccatt 1320
   cagggtctca agaagggtct ataagacact atccattgtg cttcgggctg agtccataga 1380
   gacaaccaca gacatggggg actctgccca cagggaaggc aagggctctg gccatggagc 1440
   tggatgggaa gactctgaag cccgaagaca ttgaatcctg tgcagggaaa gagcgagggt 1500
   tttgtgtaca acacacctgc atacctggat gtgaatctca gctccacccc ttcaccaact 1560
   ctgtgtggcc tgggcaagcc attctaaggg aaccctccac actgcaactt tcatgtctat 1620
   aaaatgggaa taaccatgca ttccttacag gacttttttg gtgtgaggat taaatgagag 1680
   aatatgttga aaagtgcttg gtaaatatat taatactatg cattccctct tctttgaatg 1740
   acgtgaccca ggtagtcagg cttctgacca ctagagggca gcagaaggta ctggaaaact 1800
   gggccgagtg aaccagagat tagatggggt ccagagagca gggatgaact tacccgtgtg 1860
   gattctggca actccggcag ggagggctcc agcaggcgct gagggaagaa ctttcaagca 1920
   gagccgggtc tcttcaggag cgactgcagc aaccctgatg cttggatgga gtccaggcag 1980
   gtgatggtag tgaagacctt gccaacagag tgggcgctgg agaaggagcc ctttagtggg 2040
   gaccctgggg ccacgactag gctggcaggc ccagccagca ccaattaatc catgagtatt 2100
   gcccagcatt gagcctggag caccttccag cccctggcca gagtcctggg tgttctggga 2160
   aaaaccccta aacctagtaa ctcctctccc tactaggcct ctttgttgct gaatctctgg 2220
   aatttagggg ccagcagctt tctgactcag gtcagccagg ggttcatgtt ccctcacttg 2280
   ccctccccct gcctggccca tctctggcct ggcccctggg aggaatttcc tgggccagag 2340
   ggcagccgaa agcacagatg cccaccccag caacgttccc gccacctgcc caggccagtg 2400
   ccccgtgccc aaccccagag ggtgcgggat gacagactct gacaatcatt aaaccagccg 2460
   ggcctgattt cccagcactg cctgctaaga tccgggccaa gtggcactga atatgcaaat 2520
   cacctggggc caggagccca gtctaaaggc caggaaatcc cctccatcca atgagacacc 2580
   agctcaggtt actgcagggg acacactata aagccctgag ctcagggagg agctccctcc 2640
   aggctctatt tagagccggg taggggagcg cagcggccag atacctcagc gctacctggc 2700
   ggaactggat ttctctcccg cctgccggcc tgcctgccac agccggactc cgccactccg 2760
   gtaggattcc ccgcctgtca ttccctagcc cagctcttgg gaaactgcag aggggtccag 2820
   aggatttgca gttctgaacc tgcacactcc agtctaggat ctccgagcaa gagcgtaggt 2880
   gtcctgaggg tcaaagaaca gagagagatt gtctctggga aggcagaatg gccatgacgc 2940
   cgctagcctg gctccagggc cccagagatc tgaggaggga agcccagctg gaggctcctg 3000
   tggtcctgcc ctggtctgag atcttggagc ccttcttgaa gagagggtgt ccgcagagtt 3060
   gctgatcttc ctgcccctgg gggctactct tgcccagggt tgggcaaagc agagtagctg 3120
   ggagtgtaag gagaggaccc tcgtcccctc accaacctca tcctctctcc ccctacccac 3180
   gggtagcctc atggctgcaa cctgtgagat tagcaacatt tttagcaact acttcagtgc 3240
   gatgtacagc tcggaggact ccaccctggc ctctgttccc cctgctgcca cctttggggc 3300
   cgatgacttg gtactgaccc tgagcaaccc ccagatgtca ttggagggta cagagaaggc 3360
   cagctggttg ggggaacagc cccagttctg gtcgaagacg caggttctgg actggatcag 3420
   ctaccaagtg gagaagaaca agtacgacgc aagcgccatt gacttctcac gatgtgacat 3480
   ggatggcgcc accctctgca attgtgccct tgaggagctg cgtctggtct ttgggcctct 3540
   gggggaccaa ctccatgccc agctgcgaga cctcacttcc agctcttctg atgagctcag 3600
   ttggatcatt gagctgctgg agaaggatgg catggccttc caggaggccc tagacccagg 3660
   gccctttgac cagggcagcc cctttgccca ggagctgctg gacgacggtc agcaagccag 3720
   cccctaccac cccggcagct gtggcgcagg agccccctcc cccggcagct ctgacgtctc 3780
   caccgcaggg actggtgctt ctcggagctc ccactcctca gactccggtg gaagtgacgt 3840
   ggacctggat cccactgatg gcaagctctt ccccagcgat ggttttcgtg actgcaagaa 3900
   gggggatccc aagcacgggg agcggaaacg aggccggccc cgaaagctga gcaaagagta 3960
   ctgggactgt ctcgagggca agaagagcaa gcacgcgccc agaggcaccc acctgtggga 4020
   gttcatccgg gacatcctca tccacccgga gctcaacgag ggcctcatga agtgggagaa 4080
   tcggcatgaa ggcgtcttca agttcctgcg ctccgaggct gtggcccaac tatggggcca 4140
   aaagaaaaag aacagcaaca tgacctacga gaagctgagc cgggccatga ggtactacta 4200
   caaacgggag atcctggaac gggtggatgg ccggcgactc gtctacaagt ttggcaaaaa 4260
   ctcaagcggc tggaaggagg aagaggttct ccagagtcgg aactgagggt tggaactata 4320
   cccgggacca aactcacgga ccactcgagg cctgcaaacc ttcctgggag gacgggcagg 4380
   ccagatggcc cctccactgg ggaatgctcc cagctgtgct gtggagagaa gctgatgttt 4440
   tggtgtattg tcagccatcg tcctgggact cggagactat ggcctcgcct ccccaccctc 4500
   ctcttggaat tacaagccct ggggtttgaa gctgacttta tagctgcaag tgtatctcct 4560
   tttatctggt gcctcctcaa acccagtctc agacactaaa tgcagacaac accttcctcc 4620
   tgcagacacc tggactgagc caaggaggcc tggggaggcc ctaggggagc accgtgatgg 4680
   agaggacaga gcaggggctc cagcaccttc tttctggact ggcgttcacc tccctgctca 4740
   gtgcttgggc tccacgggca ggggtcagag cactccctaa tttatgtgct atataaatac 4800
   gtcagatgta catagagatc tattttttct aaaacattcc cctccccact cctctcccac 4860
   agagtgctgg actgttccag gccctccagt gggctgatgc tgggaccctt aggatggggc 4920
   tcccagctcc tttctcctgt gaatggaggc agagacctcc aataaagtgc cttctgggct 4980
   ttttctaaaa aaaaaaaaaa aaaaaaaaaa aaagaaaaaa aaaaaaaaaa aaaaca 5036

<210> 11
<211> 5511
<212> DNA
<213> Homo sapiens

<220>
<221> misc_feature
<223> Gene ID: ERBB3

<400> 11
   acacacacac acccctcccc tgccatccct ccccggactc cggctccggc tccgattgca 60
   atttgcaacc tccgctgccg tcgccgcagc agccaccaat tcgccagcgg ttcaggtggc 120
   tcttgcctcg atgtcctagc ctaggggccc ccgggccgga cttggctggg ctcccttcac 180
   cctctgcgga gtcatgaggg cgaacgacgc tctgcaggtg ctgggcttgc ttttcagcct 240
   ggcccggggc tccgaggtgg gcaactctca ggcagtgtgt cctgggactc tgaatggcct 300
   gagtgtgacc ggcgatgctg agaaccaata ccagacactg tacaagctct acgagaggtg 360
   tgaggtggtg atggggaacc ttgagattgt gctcacggga cacaatgccg acctctcctt 420
   cctgcagtgg attcgagaag tgacaggcta tgtcctcgtg gccatgaatg aattctctac 480
   tctaccattg cccaacctcc gcgtggtgcg agggacccag gtctacgatg ggaagtttgc 540
   catcttcgtc atgttgaact ataacaccaa ctccagccac gctctgcgcc agctccgctt 600
   gactcagctc accgagattc tgtcaggggg tgtttatatt gagaagaacg ataagctttg 660
   tcacatggac acaattgact ggagggacat cgtgagggac cgagatgctg agatagtggt 720
   gaaggacaat ggcagaagct gtcccccctg tcatgaggtt tgcaaggggc gatgctgggg 780
   tcctggatca gaagactgcc agacattgac caagaccatc tgtgctcctc agtgtaatgg 840
   tcactgcttt gggcccaacc ccaaccagtg ctgccatgat gagtgtgccg ggggctgctc 900
   aggccctcag gacacagact gctttgcctg ccggcacttc aatgacagtg gagcctgtgt 960
   acctcgctgt ccacagcctc ttgtctacaa caagctaact ttccagctgg aacccaatcc 1020
   ccacaccaag tatcagtatg gaggagtttg tgtagccagc tgtccccata actttgtggt 1080
   ggatcaaaca tcctgtgtca gggcctgtcc tcctgacaag atggaagtag ataaaaatgg 1140
   gctcaagatg tgtgagcctt gtgggggact atgtcccaaa gcctgtgagg gaacaggctc 1200
   tgggagccgc ttccagactg tggactcgag caacattgat ggatttgtga actgcaccaa 1260
   gatcctgggc aacctggact ttctgatcac cggcctcaat ggagacccct ggcacaagat 1320
   ccctgccctg gacccagaga agctcaatgt cttccggaca gtacgggaga tcacaggtta 1380
   cctgaacatc cagtcctggc cgccccacat gcacaacttc agtgtttttt ccaatttgac 1440
   aaccattgga ggcagaagcc tctacaaccg gggcttctca ttgttgatca tgaagaactt 1500
   gaatgtcaca tctctgggct tccgatccct gaaggaaatt agtgctgggc gtatctatat 1560
   aagtgccaat aggcagctct gctaccacca ctctttgaac tggaccaagg tgcttcgggg 1620
   gcctacggaa gagcgactag acatcaagca taatcggccg cgcagagact gcgtggcaga 1680
   gggcaaagtg tgtgacccac tgtgctcctc tgggggatgc tggggcccag gccctggtca 1740
   gtgcttgtcc tgtcgaaatt atagccgagg aggtgtctgt gtgacccact gcaactttct 1800
   gaatggggag cctcgagaat ttgcccatga ggccgaatgc ttctcctgcc acccggaatg 1860
   ccaacccatg gagggcactg ccacatgcaa tggctcgggc tctgatactt gtgctcaatg 1920
   tgcccatttt cgagatgggc cccactgtgt gagcagctgc ccccatggag tcctaggtgc 1980
   caagggccca atctacaagt acccagatgt tcagaatgaa tgtcggccct gccatgagaa 2040
   ctgcacccag gggtgtaaag gaccagagct tcaagactgt ttaggacaaa cactggtgct 2100
   gatcggcaaa acccatctga caatggcttt gacagtgata gcaggattgg tagtgatttt 2160
   catgatgctg ggcggcactt ttctctactg gcgtgggcgc cggattcaga ataaaagggc 2220
   tatgaggcga tacttggaac ggggtgagag catagagcct ctggacccca gtgagaaggc 2280
   taacaaagtc ttggccagaa tcttcaaaga gacagagcta aggaagctta aagtgcttgg 2340
   ctcgggtgtc tttggaactg tgcacaaagg agtgtggatc cctgagggtg aatcaatcaa 2400
   gattccagtc tgcattaaag tcattgagga caagagtgga cggcagagtt ttcaagctgt 2460
   gacagatcat atgctggcca ttggcagcct ggaccatgcc cacattgtaa ggctgctggg 2520
   actatgccca gggtcatctc tgcagcttgt cactcaatat ttgcctctgg gttctctgct 2580
   ggatcatgtg agacaacacc ggggggcact ggggccacag ctgctgctca actggggagt 2640
   acaaattgcc aagggaatgt actaccttga ggaacatggt atggtgcata gaaacctggc 2700
   tgcccgaaac gtgctactca agtcacccag tcaggttcag gtggcagatt ttggtgtggc 2760
   tgacctgctg cctcctgatg ataagcagct gctatacagt gaggccaaga ctccaattaa 2820
   gtggatggcc cttgagagta tccactttgg gaaatacaca caccagagtg atgtctggag 2880
   ctatggtgtg acagtttggg agttgatgac cttcggggca gagccctatg cagggctacg 2940
   attggctgaa gtaccagacc tgctagagaa gggggagcgg ttggcacagc cccagatctg 3000
   cacaattgat gtctacatgg tgatggtcaa gtgttggatg attgatgaga acattcgccc 3060
   aacctttaaa gaactagcca atgagttcac caggatggcc cgagacccac cacggtatct 3120
   ggtcataaag agagagagtg ggcctggaat agcccctggg ccagagcccc atggtctgac 3180
   aaacaagaag ctagaggaag tagagctgga gccagaacta gacctagacc tagacttgga 3240
   agcagaggag gacaacctgg caaccaccac actgggctcc gccctcagcc taccagttgg 3300
   aacacttaat cggccacgtg ggagccagag ccttttaagt ccatcatctg gatacatgcc 3360
   catgaaccag ggtaatcttg gggagtcttg ccaggagtct gcagtttctg ggagcagtga 3420
   acggtgcccc cgtccagtct ctctacaccc aatgccacgg ggatgcctgg catcagagtc 3480
   atcagagggg catgtaacag gctctgaggc tgagctccag gagaaagtgt caatgtgtag 3540
   gagccggagc aggagccgga gcccacggcc acgcggagat agcgcctacc attcccagcg 3600
   ccacagtctg ctgactcctg ttaccccact ctccccaccc gggttagagg aagaggatgt 3660
   caacggttat gtcatgccag atacacacct caaaggtact ccctcctccc gggaaggcac 3720
   cctttcttca gtgggtctca gttctgtcct gggtactgaa gaagaagatg aagatgagga 3780
   gtatgaatac atgaaccgga ggagaaggca cagtccacct catcccccta ggccaagttc 3840
   ccttgaggag ctgggttatg agtacatgga tgtggggtca gacctcagtg cctctctggg 3900
   cagcacacag agttgcccac tccaccctgt acccatcatg cccactgcag gcacaactcc 3960
   agatgaagac tatgaatata tgaatcggca acgagatgga ggtggtcctg ggggtgatta 4020
   tgcagccatg ggggcctgcc cagcatctga gcaagggtat gaagagatga gagcttttca 4080
   ggggcctgga catcaggccc cccatgtcca ttatgcccgc ctaaaaactc tacgtagctt 4140
   agaggctaca gactctgcct ttgataaccc tgattactgg catagcaggc ttttccccaa 4200
   ggctaatgcc cagagaacgt aactcctgct ccctgtggca ctcagggagc atttaatggc 4260
   agctagtgcc tttagagggt accgtcttct ccctattccc tctctctccc aggtcccagc 4320
   cccttttccc cagtcccaga caattccatt caatctttgg aggcttttaa acattttgac 4380
   acaaaattct tatggtatgt agccagctgt gcactttctt ctctttccca accccaggaa 4440
   aggttttcct tattttgtgt gctttcccag tcccattcct cagcttcttc acaggcactc 4500
   ctggagatat gaaggattac tctccatatc ccttcctctc aggctcttga ctacttggaa 4560
   ctaggctctt atgtgtgcct ttgtttccca tcagactgtc aagaagagga aagggaggaa 4620
   acctagcaga ggaaagtgta attttggttt atgactctta accccctaga aagacagaag 4680
   cttaaaatct gtgaagaaag aggttaggag tagatattga ttactatcat aattcagcac 4740
   ttaactatga gccaggcatc atactaaact tcacctacat tatctcactt agtcctttat 4800
   catccttaaa acaattctgt gacatacata ttatctcatt ttacacaaag ggaagtcggg 4860
   catggtggct catgcctgta atctcagcac tttgggaggc tgaggcagaa ggattacctg 4920
   aggcaaggag tttgagacca gcttagccaa catagtaaga cccccatctc tttaaaaaaa 4980
   aaaaaaaaaa aaaaaaaaaa actttagaac tgggtgcagt ggctcatgcc tgtaatccca 5040
   gccagcactt tgggaggctg agatgggaag atcacttgag cccagaatta gagataagcc 5100
   tatggaaaca tagcaagaca ctgtctctac aggggaaaaa aaaaaaagaa actgagcctt 5160
   aaagagatga aataaattaa gcagtagatc caggatgcaa aatcctccca attcctgtgc 5220
   atgtgctctt attgtaaggt gccaagaaaa actgatttaa gttacagccc ttgtttaagg 5280
   ggcactgttt cttgtttttg cactgaatca agtctaaccc caacagccac atcctcctat 5340
   acctagacat ctcatctcag gaagtggtgg tgggggtagt cagaaggaaa aataactgga 5400
   catctttgtg taaaccataa tccacatgtg ccgtaaatga tcttcactcc ttatccgagg 5460
   gcaaattcac aaggatcccc aagatccact tttagaagcc attctcatcc a 5511

<210> 12
<211> 1654
<212> DNA
<213> Homo sapiens

<220>
<221> misc_feature
<223> Gene ID: KCTD14

<400> 12
ggctgcgcag tggagcggcc agtgggcagg atgacgagcc agacccctct gccccagtcc 60

ccccggccca ggcggccgac gatgtctact gttgtggagc tgaacgtcgg gggtgagttc 120
cacaccacca ccctgggtac cctgaggaag tttccgggct caaagctggc agagatgttc 180
tctagcttag ccaaggcctc cacggacgcg gagggccgct tcttcatcga ccgccccagc 240
acctatttca gacccatcct ggactacctg cgcactgggc aagtgcccac acagcacatc 300
cctgaagtgt accgtgaggc tcagttctac gaaatcaagc ctttggtcaa gctgctggag 360
gacatgccac agatctttgg tgagcaggtg tctcggaagc agtttttgct gcaagtgccg 420
ggctacagcg agaacctgga gctcatggtg cgcctggcac gtgcagaagc cataacagca 480
cggaagtcca gcgtgcttgt gtgcctggtg gaaactgagg agcaggatgc atattattca 540
gaggtcctgt gttttctgca ggataagaag atgttcaagt ctgttgtcaa gtttgggccc 600
tggaaggcgg tcctagacaa cagcgacctc atgcactgcc tggagatgga cattaaggcc 660
caggggtaca aggtattctc caagttctac ctgacgtacc ccaccaaaag aaacgaattc 720
cattttaaca tttattcatt caccttcacc tggtggtgat cctcaggagc agagactgtt 780
atgaattctg gcgtggctta tgaaattaaa agttgccatc aaagccattt tcttttaatt 840
tcacaaacat caggcaattt ccagggttgg tctagagtct tgccactaaa tattgatcac 900
tcgtttaagg actttccact ccattgcaac tgatgccact atatttgcct agcaacttgc 960
agctacttcc ttttcaaagc ctcatgtatc tcccagaccc ttctcttgaa gtccaataac 1020
aagaccaagt aagaatgttt caacaatgcg ttggcaagag atgtgagatg acaacaggaa 1080
catacaagat actgtgaatc tagatgttct gacccaaaga tgtagtctac atagccccag 1140
cttggggtcc aatccatctg tccctggcat gtgccttcat gtagtaggtg ctttcctgat 1200
cccctttgcg agatgctgtg ggtgctaaca cctcagagct gtcctcttct ctagagtgaa 1260
ggttttcaga gtgcatcatc agcattacct gtgaacttgc tggaaataca aatcctcagg 1320
ccccacctca gacctactga atcagaatct ctgggggttg gcacagcatt ctgatttacc 1380
aaaccctcca agtgattttg atgtattcta attttgagac catctctaga aaagaattgc 1440
tacctcttgt atggaggtac aaaagactga cctcttacat caaggaactt cctttcccag 1500
agctcctcat ggaatcaagc tgaagtcagt cttcttctga gagcacattc ttactcagtt 1560
tttttcctct gtcctacgct gcttccctca ctccccttct cctaagagca ctccatcaat 1620
aaaccacttg cacgagaaaa aaaaaaaaaa aaaa 1654

<210> 13
<211> 3350
<212> DNA
<213> Homo sapiens

<220>
<221> misc_feature
<223> Gene ID: KLF5

<400> 13
tagtcgcggg gcaggtacgt gcgctcgcgg ttctctcgcg gaggtcggcg gtggcgggag 60
cgggctccgg agagcctgag agcacggtgg ggcggggcgg gagaaagtgg ccgcccggag 120
gacgttggcg tttacgtgtg gaagagcgga agagttttgc ttttcgtgcg cgccttcgaa 180
aactgcctgc cgctgtctga ggagtccacc cgaaacctcc cctcctccgc cggcagcccc 240
gcgctgagct cgccgaccca agccagcgtg ggcgaggtgg gaagtgcgcc cgacccgcgc 300
ctggagctgc gcccccgagt gcccatggct acaagggtgc tgagcatgag cgcccgcctg 360
ggacccgtgc cccagccgcc ggcgccgcag gacgagccgg tgttcgcgca gctcaagccg 420
gtgctgggcg ccgcgaatcc ggcccgcgac gcggcgctct tccccggcga ggagctgaag 480
cacgcgcacc accgcccgca ggcgcagccc gcgcccgcgc aggccccgca gccggcccag 540
ccgcccgcca ccggcccgcg gctgcctcca gaggacctgg tccagacaag atgtgaaatg 600
gagaagtatc tgacacctca gcttcctcca gttcctataa ttccagagca taaaaagtat 660
agacgagaca gtgcctcagt cgtagaccag ttcttcactg acactgaagg gttaccttac 720
agtatcaaca tgaacgtctt cctccctgac atcactcacc tgagaactgg cctctacaaa 780
tcccagagac cgtgcgtaac acacatcaag acagaacctg ttgccatttt cagccaccag 840
agtgaaacga ctgcccctcc tccggccccg acccaggccc tccctgagtt caccagtata 900
ttcagctcac accagaccgc agctccagag gtgaacaata ttttcatcaa acaagaactt 960
cctacaccag atcttcatct ttctgtccct acccagcagg gccacctgta ccagctactg 1020

aatacaccgg atctagatat gcccagttct acaaatcaga cagcagcaat ggacactctt 1080
aatgtttcta tgtcagctgc catggcaggc cttaacacac acacctctgc tgttccgcag 1140
actgcagtga aacaattcca gggcatgccc ccttgcacat acacaatgcc aagtcagttt 1200
cttccacaac aggccactta ctttcccccg tcaccaccaa gctcagagcc tggaagtcca 1260
gatagacaag cagagatgct ccagaattta accccacctc catcctatgc tgctacaatt 1320
gcttctaaac tggcaattca caatccaaat ttacccacca ccctgccagt taactcacaa 1380
aacatccaac ctgtcagata caatagaagg agtaaccccg atttggagaa acgacgcatc 1440
cactactgcg attaccctgg ttgcacaaaa gtttatacca agtcttctca tttaaaagct 1500
cacctgagga ctcacactgg tgaaaagcca tacaagtgta cctgggaagg ctgcgactgg 1560
aggttcgcgc gatcggatga gctgacccgc cactaccgga agcacacagg cgccaagccc 1620
ttccagtgcg gggtgtgcaa ccgcagcttc tcgcgctctg accacctggc cctgcatatg 1680
aagaggcacc agaactgagc actgcccgtg tgacccgttc caggtcccct gggctccctc 1740
aaatgacaga cctaactatt cctgtgtaaa aacaacaaaa acaaacaaaa gcaagaaaac 1800
cacaactaaa actggaaatg tatattttgt atatttgaga aaacagggaa tacattgtat 1860
taataccaaa gtgtttggtc attttaagaa tctggaatgc ttgctgtaat gtatatggct 1920
ttactcaagc agatctcatc tcatgacagg cagccacgtc tcaacatggg taaggggtgg 1980
gggtggaggg gagtgtgtgc agcgttttta cctaggcacc atcatttaat gtgacagtgt 2040
tcagtaaaca aatcagttgg caggcaccag aagaagaatg gattgtatgt caagatttta 2100
cttggcattg agtagttttt ttcaatagta ggtaattcct tagagataca gtatacctgg 2160
caattcacaa atagccattg aacaaatgtg tgggttttta aaaattatat acatatatga 2220
gttgcctata tttgctattc aaaattttgt aaatatgcaa atcagcttta taggtttatt 2280
acaagttttt taggattctt ttggggaaga gtcataattc ttttgaaaat aaccatgaat 2340
acacttacag ttaggatttg tggtaaggta cctctcaaca ttaccaaaat catttcttta 2400
gagggaagga ataatcattc aaatgaactt taaaaaagca aatttcatgc actgattaaa 2460
ataggattat tttaaataca aaaggcattt tatatgaatt ataaactgaa gagcttaaag 2520
atagttacaa aatacaaaag ttcaacctct tacaataagc taaacgcaat gtcattttta 2580
aaaagaagga cttagggtgt cgttttcaca tatgacaatg ttgcatttat gatgcagttt 2640
caagtaccaa aacgttgaat tgatgatgca gttttcatat atcgagatgt tcgctcgtgc 2700
agtactgttg gttaaatgac aatttatgtg gattttgcat gtaatacaca gtgagacaca 2760
gtaattttat ctaaattaca gtgcagttta gttaatctat taatactgac tcagtgtctg 2820
cctttaaata taaatgatat gttgaaaact taaggaagca aatgctacat atatgcaata 2880
taaaatagta atgtgatgct gatgctgtta accaaagggc agaataaata agcaaaatgc 2940
caaaaggggt cttaattgaa atgaaaattt aattttgttt ttaaaatatt gtttatcttt 3000
atttattttg tggtaatata gtaagttttt ttagaagaca attttcataa cttgataaat 3060
tatagttttg tttgttagaa aagttgctct taaaagatgt aaatagatga caaacgatgt 3120
aaataatttt gtaagaggct tcaaaatgtt tatacgtgga aacacaccta catgaaaagc 3180
agaaatcggt tgctgttttg cttctttttc cctcttattt ttgtattgtg gtcatttcct 3240
atgcaaataa tggagcaaac agctgtatag ttgtagaatt ttttgagaga atgagatgtt 3300
tatatattaa cgacaatttt ttttttggaa aataaaaagt gcctaaaaga 3350

<210> 14
<211> 1788
<212> DNA
<213> Homo sapiens

<220>
<221> misc_feature
<223> Gene ID: KRT8

<400> 14
attcctgaga gctctcctca ccaagaagca gcttctccgc tccttctagg atctccgcct 60
ggttcggccc gcctgcctcc actcctgcct ctaccatgtc catcagggtg acccagaagt 120
cctacaaggt gtccacctct ggcccccggg ccttcagcag ccgctcctac acgagtgggc 180
ccggttcccg catcagctcc tcgagcttct cccgagtggg cagcagcaac tttcgcggtg 240
gcctgggcgg cggctatggt ggggccagcg gcatgggagg catcaccgca gttacggtca 300

accagagcct gctgagcccc cttgtcctgg aggtggaccc caacatccag gccgtgcgca 360
cccaggagaa ggagcagatc aagaccctca acaacaagtt tgcctccttc atagacaagg 420
tacggttcct ggagcagcag aacaagatgc tggagaccaa gtggagcctc ctgcagcagc 480
agaagacggc tcgaagcaac atggacaaca tgttcgagag ctacatcaac aaccttaggc 540
ggcagctgga gactctgggc caggagaagc tgaagctgga ggcggagctt ggcaacatgc 600
aggggctggt ggaggacttc aagaacaagt atgaggatga gatcaataag cgtacagaga 660
tggagaacga atttgtcctc atcaagaagg atgtggatga agcttacatg aacaaggtag 720
agctggagtc tcgcctggaa gggctgaccg acgagatcaa cttcctcagg cagctatatg 780
aagaggagat ccgggagctg cagtcccaga tctcggacac atctgtggtg ctgtccatgg 840
acaacagccg ctccctggac atggacagca tcattgctga ggtcaaggca cagtacgagg 900
atattgccaa ccgcagccgg gctgaggctg agagcatgta ccagatcaag tatgaggagc 960
tgcagagcct ggctgggaag cacggggatg acctgcggcg cacaaagact gagatctctg 1020
agatgaaccg gaacatcagc cggctccagg ctgagattga gggcctcaaa ggccagaggg 1080
cttccctgga ggccgccatt gcagatgccg agcagcgtgg agagctggcc attaaggatg 1140
ccaacgccaa gttgtccgag ctggaggccg ccctgcagcg ggccaagcag gacatggcgc 1200
ggcagctgcg tgagtaccag gagctgatga acgtcaagct ggccctggac atcgagatcg 1260
ccacctacag gaagctgctg gagggcgagg agagccggct ggagtctggg atgcagaaca 1320
tgagtattca tacgaagacc accagcggct atgcaggtgg tctgagctcg gcctatgggg 1380
gcctcacaag ccccggcctc agctacagcc tgggctccag ctttggctct ggcgcgggct 1440
ccagctcctt cagccgcacc agctcctcca gggccgtggt tgtgaagaag atcgagacac 1500
gtgatgggaa gctggtgtct gagtcctctg acgtcctgcc caagtgaaca gctgcggcag 1560
cccctcccag cctacccctc ctgcgctgcc ccagagcctg ggaaggaggc cgctatgcag 1620
ggtagcactg ggaacaggag acccacctga ggctcagccc tagccctcag cccacctggg 1680
gagtttacta cctggggacc ccccttgccc atgcctccag ctacaaaaca attcaattgc 1740
tttttttttt tggtccaaaa taaaacctca gctagctctg ccaatgtc 1788

<210> 15
<211> 4018
<212> DNA
<213> Homo sapiens

<220>
<221> misc_feature
<223> Gene ID: MAP7

<400> 15
gttccgcccg ccgctcttcc agctgagact cggcagcggc ggtcggagcg gctgccacgc 60
cgcacctgcc cgcgaggggg cgcgccaggc ccaggccgca gccgactgcc gcgctctctg 120
tccccgccct ccgccatccc cggactgcgc tcacctgtct gggccgctgg cctgggaggc 180
gggggccggc gggagccaag ccgaggaaag ggcggagcgg ctctccgggc gcgtcatcgg 240
agcaccatgg cggagctagg agctggcggc gacggccaca ggggcggcga cggcgcagtg 300
cgaagcgaaa cagcacccga cagctacaaa gtgcaagata agaaaaatgc ctccagccgc 360
cctgcctctg caatttcagg acaaaataac aaccactcag gaaataaacc agaccctccg 420
cctgtgttac gtgttgatga ccggcagcgg ctggcccggg agcgacgtga ggaacgggag 480
aaacagctag ctgcaagaga aatagtgtgg ttagaaagag aagagcgagc caggcagcac 540
tacgagaagc acctggaaga gcggaagaag aggttggagg agcagaggca gaaggaggag 600
cggaggaggg ctgctgtgga ggagaagcgg aggcagagac ttgaggagga caaagaacgc 660
cacgaagctg ttgtacggcg cacaatggaa aggagccaga agccaaaaca gaagcataac 720
cgttggtcgt ggggaggctc tctccatggg agccctagca tccacagtgc agatccagac 780
aggcggtcag tttccaccat gaatctttcg aaatatgttg atcccgtcat tagcaagcgg 840
ctctcctctt catctgcaac tttactaaat tctccagata gagctcgccg cctgcagctc 900
agcccatggg agagcagcgt tgttaacaga ctcctgacgc ccacacattc gttcctggcc 960
agaagtaaaa gcacagctgc cttgtctgga gaagcagcat cttgcagccc catcatcatg 1020
ccctacaaag ctgcacactc tagaaattcg atggatcgac caaaactctt tgtaacacca 1080
cctgagggct cttctcgcag gaggatcatt catggcacag cgagctataa aaaagaaaga 1140

gagagagaaa atgtactctt cctcacatct ggcacccgaa gggctgtatc tccatctaat 1200
cccaaagcaa gacaaccagc tcgctcccga ctttggcttc cgtccaagtc tcttcctcat 1260
ttgcctggca cacccagacc gacatcctcc ttgccacccg gctcagtcaa agctgctcct 1320
gctcaggtcc ggcccccatc ccccggcaac atccgccctg tcaagaggga agtcaaagtg 1380
gagcctgaga agaaagatcc tgagaaggaa cctcagaaag ttgccaatga gccctcacta 1440
aagggcagag cacctttagt gaaggtagaa gaagccacag ttgaagagcg gacacctgct 1500
gaaccagaag ttggccctgc tgctccagcc atggccccag ctccagcctc ggccccagct 1560
ccagcctcgg ccccagctcc agccccggtc cccaccccag ccatggtctc agccccgtca 1620
tccactgtga atgccagtgc ttctgttaag acttctgcag gcaccaccga cccagaggag 1680
gccacaaggc ttctagctga gaagaggcgg ctggcccgag agcagagaga aaaggaagaa 1740
agggagagga gggagcagga agagcttgaa agacaaaaga gagaggaatt ggctcaacgt 1800
gtggctgaag agaggacgac tcgccgtgag gaggagtcgc gcaggctgga agccgagcag 1860
gcccgggaga aggaggagca gctgcagcgg caggcggagg agcgggcgct gcgcgagcgg 1920
gaggaggcag agcgcgccca gaggcagaaa gaagaagaag ctcgcgttcg tgaagaagca 1980
gagagggtcc ggcaggaacg agagaagcat ttccagagag aagagcaaga gcgcctggag 2040
agaaagaagc gacttgagga gattatgaaa agaaccagga gaacagaagc tacagataag 2100
aaaaccagtg atcagagaaa cggtgatata gccaagggag ctctcactgg aggaacagag 2160
gtgtctgcac ttccatgtac aacaaacgct ccgggaaatg gaaagccagt tggcagccca 2220
catgtggtta cctcacacca gtcaaaagtg acagtggaga gcactcccga tttggaaaaa 2280
caaccaaatg aaaatggtgt atctgttcag aatgaaaatt ttgaagaaat tataaactta 2340
cccattggat ctaaaccatc cagattagat gtcaccaaca gtgagagccc agaaattcct 2400
ttgaatccaa ttttggcctt tgatgatgaa gggacacttg ggcccctgcc tcaggtagat 2460
ggtgttcaga cacagcagac tgcagaagtt atatgagtgt ttcttctgaa gaaccaaagc 2520
tgaaatttaa tgagaatttc tacaattaat ggaattcctt tcctgctata aaggagcatc 2580
ccctccaccc gttttctaga gttcttgacc atcattttga aaagatttat taaaactagc 2640
taaagacaac agactggata gcttttctaa taattttcat caataggaaa aaagaaatac 2700
gtctcattct tcaatacttt aaaatggctt tttccagtgt gctccttctt agcaatcaat 2760
atttttctgc attctttaaa agacaagaga atttggttat aaaagaaatg ggctgactag 2820
gcatgatttt tttggtctta aaagcttaac atgtaaaatt ggcaaaaaaa attttttacc 2880
ttttataata cttgaaaaat aagtacctct ttgttctaca agtagaatga ataggagaag 2940
agtttaagcc tgttttttta aaatattatt gcaaagagct ctatttgtag aagcaaatta 3000
taggcagatt accaggttct tataaataca gcttgtacat ggacattctg caaacccagc 3060
tgtcacattt ttcttgcaac tccttttgca aaagcagact aaaatgtttt aaaatgtgaa 3120
aaaacattat tttttcaaag caagaaaata atttactgcc ctcttacata atgtatttat 3180
aaagtttttc cagataaact aatcaaataa attagaataa tgtgacaaca ttacaaattt 3240
aatttgttag ctgcattcct tctgatgtta ccacgataga atgttactga tgattcaggg 3300
ctatttctga agtctgtatg ttgctgctgt ccccagtgat ggtggactta tctttgcctt 3360
acctgatcac aaattatgtt ggggaaaata aagatttaat atttctttaa atagaaaaag 3420
aatttggttt tgctcgttta agagcaatga gaaaatgatg gaatgttgac tgtgtttggc 3480
acacaggaca cggaccttca tggaagtcct tgctctgcgt ggcatctgtc agcttttcac 3540
ctttcattct tattcttcac ttttgctgct gagcctagct gtacaaactt gcactttcat 3600
ttgctaatat aaattcagtt ttattttacc attttagaga ctactaatga ttaaatgtag 3660
aaggagaggg tgcacatgtt tttatgtgga gtgtttaaaa gataaattta taccactgta 3720
atgtgcagct tttattaaaa gagaaattgg ttgaactgct aggttgaatg agagacttca 3780
tctattggac tatttttttt aatccaggca tatggtcttt agtaatggct tgtaatttgt 3840
gaaaacatta atttgggggt tttccctgtt ttcagttgtc catgtacaca tagtcattat 3900
attagaaaag aaagctgttc aacaaacttg tttaatttgt ttaaatcaac atagcatgaa 3960
acaccaaata aaatgtttga catagtttta aaaaaaaaaa aaaaaaaaaa aaaaaaaa 4018

<210> 16
<211> 2601
<212> DNA
<213> Homo sapiens

<220>

<221>  misc_feature
<223>  Gene ID: NQO1

<400>  16
ccgcccttgt aggctgtcca cctcaaacgg gccggacagg atatataaga gagaatgcac      60
cgtgcactac acacgcgact cccacaaggt tgcagccgga gccgcccagc tcaccgagag     120
cctagttccg gccagggtcg ccccggcaac cacgagccca gccaatcagc gccccggact     180
gcaccagagc catggtcggc agaagagcac tgatcgtact ggctcactca gagaggacgt     240
ccttcaacta tgccatgaag gaggctgctg cagcggcttt gaagaagaaa ggatgggagg     300
tggtggagtc ggacctctat gccatgaact tcaatcccat catttccaga aaggacatca     360
caggtaaact gaaggaccct gcgaactttc agtatcctgc cgagtctgtt ctggcttata     420
aagaaggcca tctgagccca gatattgtgg ctgaacaaaa gaagctggaa gccgcagacc     480
ttgtgatatt ccagttcccc ctgcagtggt ttggagtccc tgccattctg aaaggctggt     540
ttgagcgagt gttcatagga gagtttgctt acacttacgc tgccatgtat gacaaaggac     600
ccttccggag taagaaggca gtgctttcca tcaccactgg tggcagtggc tccatgtact     660
ctctgcaagg gatccacggg gacatgaatg tcattctctg gccaattcag agtggcattc     720
tgcatttctg tggcttccaa gtcttagaac ctcaactgac atatagcatt gggcacactc     780
cagcagacgc ccgaattcaa atcctggaag gatggaagaa acgcctggag aatatttggg     840
atgagacacc actgtatttt gctccaagca gcctctttga cctaaacttc caggcaggat     900
tcttaatgaa aaaagaggta caggatgagg agaaaaacaa gaaatttggc ctttctgtgg     960
gccatcactt gggcaagtcc atcccaactg acaaccagat caaagctaga aaatgagatt    1020
ccttagcctg gatttccttc taacatgtta tcaaatctgg gtatctttcc aggcttccct    1080
gacttgcttt agtttttaag atttgtgttt ttcttttttc acaaggaata aatgagaggg    1140
aatcgactgt attcgtgcat ttttggatca tttttaactg attcttatga ttactatcat    1200
ggcatataac caaaatccga ctgggctcaa gaggccactt agggaaagat gtagaaagat    1260
gctagaaaaa tgttctttaa aggcatctac acaatttaat tcctcttttt agggctaaag    1320
ttttagggta cagtttggct aggtatcatt caactctcca atgttctatt aatcacctct    1380
ctgtagttta tggcagaagg gaattgctca gagaaggaaa agactgaatc tacctgccct    1440
aagggactta acttgtttgg tagttagcca tctaatgctt gtttatgata tttcttgctt    1500
tcaattacaa agcagttact aatatgccta gcacaagtac cactcttggt cagcttttgt    1560
tgtttatata cagtacacag ataccttgaa aggaagagct aataaatctc ttctttgctg    1620
cagtcatcta cttttttttt aattaaaaaa aatttttttt tgaagcagtc ttgctctgtt    1680
acccaggctg gagtgcagtg gtgtgatctc ggctcactgc aacctctgcc tcccaggttc    1740
cagcaattct cctgcctcag cctccctagt agctgggatg acaggcgcct gccatcatgc    1800
ctgactaatt tttgtatttt tagtagagac ggcgtttcac catgttggcc aggctggtct    1860
caaactcctg acctcaggtg atccgcctac ctcagcctcc caaagtgctg ggattacagg    1920
cgtgatccac cacacctggc ccttgcaatc ttctacttta aggtttgcag agataaacca    1980
ataaatccac accgtacatc tgcaatatga attcaagaaa ggaaatagta ccttcaatac    2040
ttaaaaatag tcttccacaa aaaatacttt atttctgatc tatacaaatt ttcagaaggt    2100
tattttcttt atcattgcta aactgatgac ttactatggg atggggtcca gtcccatgac    2160
cttggggtac aattgtaaac ctagagtttt atcaactttg gtgaacagtt ttggcataat    2220
agtcaatttc tacttctgga agtcatctca ttccactgtt ggtattatat aattcaagga    2280
gaatatgata aaacactgcc ctcttgtggt gcattgaaag aagagatgag aaatgatgaa    2340
aaggttgcct gaaaaatggg agacagcctc ttacttgcca agaaaatgaa gggattggac    2400
cgagctggaa aacctccttt accagatgct gactggcact ggtggttttt gctctcgaca    2460
gtatccacaa tagctgacgg ctgggtgttt cagtttgaaa atattttgtt gccttcatct    2520
tcactgcaat tttgtgtaaa tttctcaaag atctgaatta aataaataaa attcatttct    2580
acagacccac aaaaaaaaaa a                                              2601

<210>  17
<211>  2499
<212>  DNA
<213>  Homo sapiens

<220>

<221>  misc_feature
<223>  Gene ID: NQO1

<400>  17
ccgcccttgt aggctgtcca cctcaaacgg gccggacagg atatataaga gagaatgcac      60
cgtgcactac acacgcgact cccacaaggt tgcagccgga gccgcccagc tcaccgagag     120
cctagttccg gccagggtcg ccccggcaac cacgagccca gccaatcagc gccccggact     180
gcaccagagc catggtcggc agaagagcac tgatcgtact ggctcactca gagaggacgt     240
ccttcaacta tgccatgaag gaggctgctg cagcggcttt gaagaagaaa ggatgggagg     300
tggtggagtc ggacctctat gccatgaact tcaatcccat catttccaga aaggacatca     360
caggtaaact gaaggaccct gcgaactttc agtatcctgc cgagtctgtt ctggcttata     420
aagaaggcca tctgagccca gatattgtgg ctgaacaaaa gaagctggaa gccgcagacc     480
ttgtgatatt ccagttcccc ctgcagtggt ttggagtccc tgccattctg aaaggctggt     540
ttgagcgagt gttcatagga gagtttgctt acacttacgc tgccatgtat gacaaaggac     600
ccttccggag tggcattctg catttctgtg gcttccaagt cttagaacct caactgacat     660
atagcattgg gcacactcca gcagacgccc gaattcaaat cctggaagga tggaagaaac     720
gcctggagaa tatttgggat gagacaccac tgtattttgc tccaagcagc ctctttgacc     780
taaacttcca ggcaggattc ttaatgaaaa aagaggtaca ggatgaggag aaaaacaaga     840
aatttggcct ttctgtgggc catcacttgg gcaagtccat cccaactgac aaccagatca     900
aagctagaaa atgagattcc ttagcctgga tttccttcta acatgttatc aaatctgggt     960
atctttccag gcttccctga cttgctttag tttttaagat ttgtgttttt ctttttccac    1020
aaggaataaa tgagagggaa tcgactgtat tcgtgcattt ttggatcatt tttaactgat    1080
tcttatgatt actatcatgg catataacca aaatccgact gggctcaaga ggccacttag    1140
ggaaagatgt agaaagatgc tagaaaaatg ttctttaaag gcatctacac aatttaattc    1200
ctctttttag ggctaaagtt ttagggtaca gtttggctag gtatcattca actctccaat    1260
gttctattaa tcacctctct gtagtttatg gcagaaggga attgctcaga gaaggaaaag    1320
actgaatcta cctgccctaa gggacttaac ttgtttggta gttagccatc taatgcttgt    1380
ttatgatatt tcttgctttc aattacaaag cagttactaa tatgcctagc acaagtacca    1440
ctcttggtca gcttttgttg tttatataca gtacacagat accttgaaag gaagagctaa    1500
taaatctctt ctttgctgca gtcatctact ttttttttaa ttaaaaaaaa tttttttttg    1560
aagcagtctt gctctgttac ccaggctgga gtgcagtggt gtgatctcgg ctcactgcaa    1620
cctctgcctc ccaggttcca gcaattctcc tgcctcagcc tccctagtag ctgggatgac    1680
aggcgcctgc catcatgcct gactaatttt tgtattttta gtagagacgg cgtttcacca    1740
tgttggccag gctggtctca aactcctgac ctcaggtgat ccgcctacct cagcctccca    1800
aagtgctggg attacaggcg tgatccacca cacctggccc ttgcaatctt ctactttaag    1860
gtttgcagag ataaaccaat aaatccacac cgtacatctg caatatgaat tcaagaaagg    1920
aaatagtacc ttcaatactt aaaaatagtc ttccacaaaa aatactttat ttctgatcta    1980
tacaaatttt cagaaggtta ttttctttat cattgctaaa ctgatgactt actatgggat    2040
ggggtccagt cccatgacct tggggtacaa ttgtaaacct agagttttat caactttggt    2100
gaacagtttt ggcataatag tcaatttcta cttctggaag tcatctcatt ccactgttgg    2160
tattatataa ttcaaggaga atatgataaa acactgccct cttgtggtgc attgaaagaa    2220
gagatgagaa atgatgaaaa ggttgcctga aaaatgggag acagcctctt acttgccaag    2280
aaaatgaagg gattggaccg agctggaaaa cctcctttac cagatgctga ctggcactgg    2340
tggtttttgc tctcgacagt atccacaata gctgacggct gggtgtttca gtttgaaaat    2400
attttgttgc cttcatcttc actgcaattt tgtgtaaatt tctcaaagat ctgaattaaa    2460
taaataaaat tcatttctac agacccacaa aaaaaaaaa                            2499

<210>  18
<211>  2487
<212>  DNA
<213>  Homo sapiens

<220>
<221>  misc_feature
<223>  Gene ID: NQO1

<400>  18
ccgcccttgt aggctgtcca cctcaaacgg gccggacagg atatataaga gagaatgcac      60
cgtgcactac acacgcgact cccacaaggt tgcagccgga gccgcccagc tcaccgagag     120
cctagttccg gccagggtcg ccccggcaac cacgagccca gccaatcagc gccccggact     180
gcaccagagc catggtcggc agaagagcac tgatcgtact ggctcactca gagaggacgt     240
ccttcaacta tgccatgaag gaggctgctg cagcggcttt gaagaagaaa ggatgggagg     300
tggtggagtc ggacctctat gccatgaact tcaatcccat catttccaga aaggacatca     360
caggtaaact gaaggaccct gcgaactttc agtatcctgc cgagtctgtt ctggcttata     420
aagaaggcca tctgagccca gatattgtgg ctgaacaaaa gaagctggaa gccgcagacc     480
ttgtgatatt ccagagtaag aaggcagtgc tttccatcac cactggtggc agtggctcca     540
tgtactctct gcaagggatc cacggggaca tgaatgtcat tctctggcca attcagagtg     600
gcattctgca tttctgtggc ttccaagtct tagaacctca actgacatat agcattgggc     660
acactccagc agacgcccga attcaaatcc tggaaggatg gaagaaacgc ctggagaata     720
tttgggatga gacaccactg tattttgctc caagcagcct ctttgaccta aacttccagg     780
caggattctt aatgaaaaaa gaggtacagg atgaggagaa aaacaagaaa tttggccttt     840
ctgtgggcca tcacttgggc aagtccatcc caactgacaa ccagatcaaa gctagaaaat     900
gagattcctt agcctggatt tccttctaac atgttatcaa atctgggtat ctttccaggc     960
ttccctgact tgctttagtt tttaagattt gtgtttttct ttttccacaa ggaataaatg    1020
agagggaatc gactgtattc gtgcattttt ggatcatttt taactgattc ttatgattac    1080
tatcatggca tataaccaaa atccgactgg gctcaagagg ccacttaggg aaagatgtag    1140
aaagatgcta gaaaaatgtt ctttaaaggc atctacacaa tttaattcct ctttttaggg    1200
ctaaagtttt agggtacagt ttggctaggt atcattcaac tctccaatgt tctattaatc    1260
acctctctgt agtttatggc agaagggaat tgctcagaga aggaaaagac tgaatctacc    1320
tgccctaagg gacttaactt gtttggtagt tagccatcta atgcttgttt atgatatttc    1380
ttgctttcaa ttacaaagca gttactaata tgcctagcac aagtaccact cttggtcagc    1440
ttttgttgtt tatatacagt acacagatac cttgaaagga agagctaata aatctcttct    1500
ttgctgcagt catctacttt ttttttaatt aaaaaaaatt tttttttgaa gcagtcttgc    1560
tctgttaccc aggctggagt gcagtggtgt gatctcggct cactgcaacc tctgcctccc    1620
aggttccagc aattctcctg cctcagcctc cctagtagct gggatgacag gcgcctgcca    1680
tcatgcctga ctaatttttg tatttttagt agagacggcg tttcaccatg ttggccaggc    1740
tggtctcaaa ctcctgacct caggtgatcc gcctacctca gcctcccaaa gtgctgggat    1800
tacaggcgtg atccaccaca cctggccctt gcaatcttct actttaaggt ttgcagagat    1860
aaaccaataa atccacaccg tacatctgca atatgaattc aagaaaggaa atagtacctt    1920
caatacttaa aaatagtctt ccacaaaaaa tactttattt ctgatctata caaattttca    1980
gaaggttatt ttctttatca ttgctaaact gatgacttac tatgggatgg ggtccagtcc    2040
catgaccttg ggtacaattt gtaaacctag agttttatca actttggtga acagttttgg    2100
cataatagtc aatttctact tctggaagtc atctcattcc actgttggta ttatataatt    2160
caaggagaat atgataaaac actgccctct tgtggtgcat tgaaagaaga gatgagaaat    2220
gatgaaaagg ttgcctgaaa aatgggagac agcctcttac ttgccaagaa aatgaaggga    2280
ttggaccgag ctggaaaacc tcctttacca gatgctgact ggcactggtg gtttttgctc    2340
tcgacagtat ccacaatagc tgacggctgg gtgtttcagt ttgaaaatat tttgttgcct    2400
tcatcttcac tgcaattttg tgtaaatttc tcaaagatct gaattaaata aataaaattc    2460
atttctacag acccacaaaa aaaaaaa                                       2487

<210>  19
<211>  4021
<212>  DNA
<213>  Homo sapiens

<220>
<221>  misc_feature
<223>  Gene ID:  RBM35B

<400>  19

ggtagccgcc ccgccccgcg gggcgccacg ggcgggtctt ggcagcgccc actgagccag 60
ccgggccgca ggtgccgccc ccgatacacg gtgtcccgcc caagctgatc cgcgtctgcg 120
gtcggtcggt gcgtgcgtgc gcctcgtcgg tccgcgtgtc tggccgagag cccccttcct 180
ctgcggccat gactccgccg ccgccgccgc cccctccccc gggccctgac cccgcggccg 240
accccgccgc ggacccctgc ccctggcccg gatcactggt cgtcctcttc ggggctacgg 300
cgggtgcgct gggacgggac ctgggctcgg acgagaccga cttaatcctc ctagtttggc 360
aagtggttga gccgcggagc cgccaggtgg ggacgctgca caaatcgctg gttcgtgccg 420
aggcggccgc actgagtacg cagtgccgcg aggcgagcgg cctgagcgcc gacagcctgg 480
cgcgggcaga gccgctggac aaggtgctgc agcagttctc acagctggtg aacggggatg 540
tggctttgct gggcgggggc ccctacatgc tctgcactga tgggcagcag ctattgcgac 600
aggtcctgca ccccgaggcc tccaggaaga acctggtgct ccccgacatg ttcttctcct 660
tctatgacct ccgaagagaa ttccatatgc agcatccaag cacctgccct gccagggacc 720
tcactgtggc caccatggca cagggtttag gactggagac agatgccaca gaggatgact 780
ttggggtctg ggaagtcaag acaatggtag ctgttatcct ccatctactc aaagagccca 840
gcagtcaatt gttttcgaag cccgaggtga taaagcagaa atacgagacg gggccttgca 900
gcaaggctga tgtggtggac agtgagactg tggtacgggc tcgtgggttg ccgtggcagt 960
catcagacca ggacgtggct cgcttcttca aagggctcaa cgtggccagg ggtggtgtag 1020
cactctgcct caacgcccag ggccgcagaa atggcgaggc cctcatccgc tttgtggaca 1080
gcgagcagcg ggacctagcg ctgcagagac acaagcacca catgggcgtc cgctatattg 1140
aggtgtataa agcgacaggg gaggagtttg taaagattgc agggggcaca tcactagagg 1200
tggctcgttt cttgtcacgg gaagaccaag tgatcctgcg gctgcgggga ctgcccttct 1260
cggctgggcc aacggacgtg cttggcttcc tggggccaga gtgcccagtg actgggggta 1320
ccgaggggct gctctttgtg cgccatcctg atggccggcc gactggtgat gccttcgccc 1380
tctttgcttg tgaggagctg gcacaggctg cactgcgcag gcacaagggc atgctgggta 1440
agcgatacat tgaactcttc cggagcactg cagccgaagt gcagcaggtc ttgaaccgct 1500
atgcatccgg cccactcctt cctacactga ctgccccact gctgcccatc cccttcccac 1560
tggcacctgg gactgggagg gactgtgtac gcctccgagg cctgccctac acggccacca 1620
ttgaagacat cctgagcttt ctgggggagg cagcagctga cattcggccc cacggtgtac 1680
acatggtgct caaccagcag ggccggccat cgggcgatgc cttcattcag atgacatcag 1740
cagagcgagc cctagctgct gctcagcgtt gccataagaa ggtgatgaag gagcgctacg 1800
tggaggtggt cccctgttcc acagaggaga tgagccgagt gctgatgggg ggcaccttgg 1860
gccgcagtgg catgtcccct ccaccctgca agctgccctg cctctcacca cctacctaca 1920
ccaccttcca agccacccca acgctcattc ccacggagac ggcagctcta tacccctctt 1980
cagcactgct cccagctgcc agggtgcctg ctgcccccac ccctgttgcc tactatccag 2040
ggccagccac tcaactctac ctgaactaca cagcctacta cccaagcccc ccagtctccc 2100
ccaccactgt gggctacctc actacaccca ctgctgccct ggcctctgct cccacctcag 2160
tgttgtccca gtcaggagcc ttggtccgca tgcagggtgt cccatacacg gctggtatga 2220
aggatctgct cagcgtcttc caggcctacc agctacccgc tgatgactac accagtctga 2280
tgcctgttgg tgacccacct cgcactgtgt tacaagcccc caaggaatgg gtgtgtttgt 2340
aggagagaaa gccaggaggt aagagccagc tgatatcctc ggcgaacatg tctctcctga 2400
gtccagaaga ccagcaccct caacctggta gcttctttct ggcttgtcaa agctctcaga 2460
aggtacctag aggagcccaa gccccagctc catcctccac ttattctgcc tgtttccccc 2520
aaagacaatg gctggaccct gcatgcaggg ctgggggtgg aatggggcta accagctcct 2580
gatggcctga gccaggcatc ttgactggca cctggagagc ccttaagtct gtcctggctg 2640
tggcccatgc cgacagatat cgtggggctg acaggtccac ggcaggcttg ctttctttta 2700
taaaatggaa gctctggtac cttcaatgta tgactcctgg gagaatcaag ggtccatctg 2760
agcctctgag taaagatccc aatgttctac ctctccctgt ccctcttgta ggggataggg 2820
aggcagagag agccagcccc taccctcaga gtatctggac ctcagagacc atgttgtgcc 2880
aggggtggtc ccacctaaag atgctagccc ctctccaggt gggcataagg agtaacagat 2940
ggcaaaacca caaactattt tgatggactg tgctgcagta tcaccagaag acattagggg 3000
gcagtaggcc cccacacaaa accttcaggc ttgaatttta aaggggagga ctttctgcca 3060
acttttcttg tatgccttgg gaaagccagt tgccctgaac ccagcagaca ccatggaatg 3120
tcctttgcac gcattaaatg gtacagaact gaagcctcgg aagcaatttg gaactcgatc 3180
ttctcttcct taaatgaaaa gttattgacc aaatggactt tttaaaagac acaggaccct 3240
taactttgcc ccaaagtgag gggctccaca ccaaccccag gcggaggaac actcagacag 3300

attaaggata ctgttgacct gtcactgttt attatttcag cactaaaact gaggagcctc 3360
aactgctggc tcttcttccc tttgtatttg tgtaaggagc actgcactcc cataaaaggt 3420
tttaaaatac aaaatgtaca agaacacaca attccaagtg ctgtaaacat aactgagaac 3480
cagttccttt actaaacatc cattttataa aacacaaggt ttcaatttga gcccatctga 3540
gccttaaaga tccattctga ataccaaaaa cagggcttca cagccaggcc cagaagaggt 3600
ctggtgataa tggctggccc tgggtgggga tagtttacac ccgggcagca gcaccacaca 3660
tgaacccaaa gacatgttct ttttaaagct gttttcagcc atgtttctct gtgcatctcc 3720
agtaagcaga aggctaccca ttccattcct caacccaaga gctagcacag ttagagtagg 3780
agggggtgcg tactagcacg tgcccagttg ctcagtgctg ctagtagaaa ttgatttgca 3840
tagtccaatg gatgtgtgct ttaacaccac tatgttgcac aaaaatttaa gtctttatct 3900
acaaagccaa aaaatattga ctcttaacac caaagctttt acaaagctga tataaaactg 3960
cttacatagt atacaaagct ctattttaaa atttaatgtt tattttaaat aggaaagcat 4020
t 4021

<210> 20
<211> 3226
<212> DNA
<213> Homo sapiens

<220>
<221> misc_feature
<223> Gene ID: TMPRSS

<400> 20
cgcgagctaa gcaggaggcg gaggcggagg cggagggcga ggggcgggga gcgccgcctg 60
gagcgcggca ggtcatattg aacattccag atacctatca ttactcgatg ctgttgataa 120
cagcaagatg gctttgaact cagggtcacc accagctatt ggaccttact atgaaaacca 180
tggataccaa ccggaaaacc cctatcccgc acagcccact gtggtcccca ctgtctacga 240
ggtgcatccg gctcagtact acccgtcccc cgtgccccag tacgccccga gggtcctgac 300
gcaggcttcc aaccccgtcg tctgcacgca gcccaaatcc ccatccggga cagtgtgcac 360
ctcaaagact aagaaagcac tgtgcatcac cttgaccctg gggaccttcc tcgtgggagc 420
tgcgctggcc gctggcctac tctggaagtt catgggcagc aagtgctcca actctgggat 480
agagtgcgac tcctcaggta cctgcatcaa cccctctaac tggtgtgatg gcgtgtcaca 540
ctgccccggc ggggaggacg agaatcggtg tgttcgcctc tacggaccaa acttcatcct 600
tcagatgtac tcatctcaga ggaagtcctg gcaccctgtg tgccaagacg actggaacga 660
gaactacggg cgggcggcct gcagggacat gggctataag aataattttt actctagcca 720
aggaatagtg gatgacagcg gatccaccag ctttatgaaa ctgaacacaa gtgccggcaa 780
tgtcgatatc tataaaaaac tgtaccacag tgatgcctgt tcttcaaaag cagtggtttc 840
tttacgctgt atagcctgcg gggtcaactt gaactcaagc cgccagagca ggatcgtggg 900
cggtgagagc gcgctcccgg gggcctggcc ctggcaggtc agcctgcacg tccagaacgt 960
ccacgtgtgc ggaggctcca tcatcacccc cgagtggatc gtgacagccg cccactgcgt 1020
ggaaaaacct cttaacaatc catggcattg gacggcattt gcggggattt tgagacaatc 1080
tttcatgttc tatggagccg gataccaagt agaaaaagtg atttctcatc caaattatga 1140
ctccaagacc aagaacaatg acattgcgct gatgaagctg cagaagcctc tgactttcaa 1200
cgacctagtg aaaccagtgt gtctgcccaa cccaggcatg atgctgcagc cagaacagct 1260
ctgctggatt tccgggtggg gggccaccga ggagaaaggg aagacctcag aagtgctgaa 1320
cgctgccaag gtgcttctca ttgagacaca gagatgcaac agcagatatg tctatgacaa 1380
cctgatcaca ccagccatga tctgtgccgg cttcctgcag gggaacgtcg attcttgcca 1440
gggtgacagt ggagggcctc tggtcacttc gaagaacaat atctggtggc tgatagggga 1500
tacaagctgg ggttctggct gtgccaaagc ttacagacca ggagtgtacg ggaatgtgat 1560
ggtattcacg gactggattt atcgacaaat gagggcagac ggctaatcca catggtcttc 1620
gtccttgacg tcgttttaca agaaaacaat ggggctggtt ttgcttcccc gtgcatgatt 1680
tactcttaga gatgattcag aggtcacttc atttttatta aacagtgaac ttgtctggct 1740
ttggcactct ctgccattct gtgcaggctg cagtggctcc cctgcccagc ctgctctccc 1800
taaccccttg tccgcaaggg gtgatggccg gctggttgtg ggcactggcg gtcaagtgtg 1860

gaggagaggg gtggaggctg ccccattgag atcttcctgc tgagtccttt ccaggggcca 1920
attttggatg agcatggagc tgtcacctct cagctgctgg atgacttgag atgaaaaagg 1980
agagacatgg aaagggagac agccaggtgg cacctgcagc ggctgccctc tggggccact 2040
tggtagtgtc cccagcctac ctctccacaa ggggattttg ctgatgggtt cttagagcct 2100
tagcagccct ggatggtggc cagaaataaa gggaccagcc cttcatgggt ggtgacgtgg 2160
tagtcacttg taaggggaac agaaacattt ttgttcttat ggggtgagaa tatagacagt 2220
gcccttggtg cgagggaagc aattgaaaag gaacttgccc tgagcactcc tggtgcaggt 2280
ctccacctgc acattgggtg gggctcctgg gagggagact cagccttcct cctcatcctc 2340
cctgaccctg ctcctagcac cctggagagt gcacatgccc cttggtcctg gcagggcgcc 2400
aagtctggca ccatgttggc ctcttcaggc ctgctagtca ctggaaattg aggtccatgg 2460
gggaaatcaa ggatgctcag tttaaggtac actgtttcca tgttatgttt ctacacattg 2520
ctacctcagt gctcctggaa acttagcttt tgatgtctcc aagtagtcca ccttcattta 2580
actctttgaa actgtatcac ctttgccaag taagagtggt ggcctatttc agctgctttg 2640
acaaaatgac tggctcctga cttaacgttc tataaatgaa tgtgctgaag caaagtgccc 2700
atggtggcgg cgaagaagag aaagatgtgt tttgttttgg actctctgtg gtcccttcca 2760
atgctgtggg tttccaacca ggggaagggt cccttttgca ttgccaagtg ccataaccat 2820
gagcactact ctaccatggt tctgcctcct ggccaagcag gctggtttgc aagaatgaaa 2880
tgaatgattc tacagctagg acttaacctt gaaatggaaa gtcttgcaat cccatttgca 2940
ggatccgtct gtgcacatgc ctctgtagag agcagcattc ccagggacct tggaaacagt 3000
tggcactgta aggtgcttgc tccccaagac acatcctaaa aggtgttgta atggtgaaaa 3060
cgtcttcctt ctttattgcc ccttcttatt tatgtgaaca actgtttgtc tttttttgta 3120
tcttttttaa actgtaaagt tcaattgtga aaatgaatat catgcaaata aattatgcga 3180
tttttttttc aaagcaaaaa aaaaaaaaaa aaaaaaaaaa aaaaaa 3226

<210> 21
<211> 25
<212> DNA
<213> Homo sapiens

<220>
<221> misc_feature
<223> Gene ID: ANXA3 ; Taken from Table 4 of the Specification

<400> 21
tcagaggaat tggaactgat gagaa 25

<210> 22
<211> 25
<212> DNA
<213> Homo sapiens

<220>
<221> misc_feature
<223> Gene ID: AP1M2; Taken from Table 4 of the Specification

<400> 22
agggcaagcc attgatcagc cgcaa 25

<210> 23
<211> 25
<212> DNA
<213> Homo sapiens

<220>
<221> misc_feature

<223>  Gene ID: ATP1B1; Taken from Table 4 of the Specification

<400>  23
cagtgcactg gcaagcgaga tgaag                                          25

<210>  24
<211>  25
<212>  DNA
<213>  Homo sapiens

<220>
<221>  misc_feature
<223>  Gene ID: C11orf9; Sequence taken from Table 4 of the
       Specification

<400>  24
gtcatggcct tcagcgtggt gtcca                                          25

<210>  25
<211>  25
<212>  DNA
<213>  Homo sapiens

<220>
<221>  misc_feature
<223>  Gene ID: C1orf106; Taken from Table 4 of the Specification

<400>  25
tgggacagct ggggaagctc caggc                                          25

<210>  26
<211>  25
<212>  DNA
<213>  Homo sapiens

<220>
<221>  misc_feature
<223>  Gene ID: CDH1; Taken from Table 4 of the Specification

<400>  26
cccgccccat caggcctccg tttct                                          25

<210>  27
<211>  25
<212>  DNA
<213>  Homo sapiens

<220>
<221>  misc_feature
<223>  Gene ID: COBL; Taken from Table 4 of the Specification

<400>  27
ctgagaaagg tgcccttgct cgtgt                                          25

<210>  28

<211>  25
<212>  DNA
<213>  Homo sapiens

<220>
<221>  misc_feature
<223>  Gene ID: CYP3A7; Taken from Table 4 of the Specification

<400>  28
ttattttgat ggctctaaca gtgac                                          25

<210>  29
<211>  25
<212>  DNA
<213>  Homo sapiens

<220>
<221>  misc_feature
<223>  Gene ID: DSG2; Taken from Table 4 of the Specification

<400>  29
cagagaggaa cacagcagct acact                                          25

<210>  30
<211>  25
<212>  DNA
<213>  Homo sapiens

<220>
<221>  misc_feature
<223>  Gene ID: ELF3; Taken from Table 4 of the Specification

<400>  30
caagcacgcg cccagaggca cccac                                          25

<210>  31
<211>  25
<212>  DNA
<213>  Homo sapiens

<220>
<221>  misc_feature
<223>  Gene ID: ERBB3; Taken from Table 4 of the Specification

<400>  31
gcaactctca ggcagtgtgt cctgg                                          25

<210>  32
<211>  25
<212>  DNA
<213>  Homo sapiens

<220>
<221>  misc_feature
<223>  Gene ID: KLF5; Taken from Table 4 of the Specification

<400>  32
actgcgatta ccctggttgc acaaa                                          25

<210>  33
<211>  25
<212>  DNA
<213>  Homo sapiens

<220>
<221>  misc_feature
<223>  Gene ID: MAP7; Taken from Table 4 of the Specification

<400>  33
gcgaagcgaa acagcacccg acagc                                          25

<210>  34
<211>  25
<212>  DNA
<213>  Homo sapiens

<220>
<221>  misc_feature
<223>  Gene ID: NQO1; Taken from Table 4 of the Specification

<400>  34
gagccatggt cggcagaaga gcact                                          25

<210>  35
<211>  25
<212>  DNA
<213>  Homo sapiens

<220>
<221>  misc_feature
<223>  Gene ID: RBM35B; Taken from Table 4 of the Specification

<400>  35
gtgctgcagc agttctcaca gctgg                                          25

<210>  36
<211>  25
<212>  DNA
<213>  Homo sapiens

<220>
<221>  misc_feature
<223>  Gene ID: TMPRSS2; Taken from Table 4 of the Specification

<400>  36
tcggtgtgtt cgcctctacg gacca                                          25

<210>  37
<211>  1331
<212>  DNA

<213>  Homo sapiens

<220>
<221>  misc_feature
<223>  Gene ID: HPRT1*; Taken from Table 4 of the Specification

<400>  37
   tcttgctgcg cctccgcctc ctcctctgct ccgccaccgg cttcctcctc ctgagcagtc    60
   agcccgcgcg ccggccggct ccgttatggc gacccgcagc cctggcgtcg tgattagtga   120
   tgatgaacca ggttatgacc ttgatttatt ttgcatacct aatcattatg ctgaggattt   180
   ggaaagggtg tttattcctc atggactaat tatggacagg actgaacgtc ttgctcgaga   240
   tgtgatgaag gagatgggag gccatcacat tgtagccctc tgtgtgctca aggggggcta   300
   taaattcttt gctgacctgc tggattacat caaagcactg aatagaaata gtgatagatc   360
   cattcctatg actgtagatt ttatcagact gaagagctat tgtaatgacc agtcaacagg   420
   ggacataaaa gtaattggtg gagatgatct ctcaacttta actggaaaga atgtcttgat   480
   tgtggaagat ataattgaca ctggcaaaac aatgcagact ttgctttcct tggtcaggca   540
   gtataatcca aagatggtca aggtcgcaag cttgctggtg aaaaggaccc cacgaagtgt   600
   tggatataag ccagactttg ttggatttga aattccagac aagtttgttg taggatatgc   660
   ccttgactat aatgaatact tcaggatttt gaatcatgtt tgtgtcatta gtgaaactgg   720
   aaaagcaaaa tacaaagcct aagatgagag ttcaagttga gtttggaaac atctggagtc   780
   ctattgacat cgccagtaaa attatcaatg ttctagttct gtggccatct gcttagtaga   840
   gctttttgca tgtatcttct aagaatttta tctgttttgt actttagaaa tgtcagttgc   900
   tgcattccta aactgtttat ttgcactatg agcctataga ctatcagttc cctttgggcg   960
   gattgttgtt taacttgtaa atgaaaaaat tctcttaaac cacagcacta ttgagtgaaa  1020
   cattgaactc atatctgtaa gaaataaaga gaagatatat tagtttttta attggtattt  1080
   taatttttat atatgcagga aagaatagaa gtgattgaat attgttaatt ataccaccgt  1140
   gtgttagaaa agtaagaagc agtcaatttt cacatcaaag acagcatcta agaagttttg  1200
   ttctgtcctg gaattatttt agtagtgttt cagtaatgtt gactgtattt tccaacttgt  1260
   tcaaattatt accagtgaat ctttgtcagc agttcccttt taaatgcaaa tcaataaatt  1320
   cccaaaaatt t                                                        1331

<210>  38
<211>  2212
<212>  DNA
<213>  Homo sapiens

<220>
<221>  misc_feature
<223>  Gene ID:  ATP1B1 ... NM_0010001787;  Taken from Table 4 of the
       Specification

<220>
<221>  misc_feature
<223>  Gene ID:  ATP1B1 ... NM_0010001787

<400>  38
cagcggcgcg tcctgcctgc agagagccag gccggagaag ccgagcggcg cagaggacgc    60
cagggcgcgc gccgcagcca cccaccctcc ggaccgcggc agctgctgac ccgccatcgc   120
catggcccgc gggaaagcca aggaggaggg cagctggaag aaattcatct ggaactcaga   180
gaagaaggag tttctgggca ggaccggtgg cagttggttt aagatccttc tattctacgt   240
aatattttat ggctgcctgg ctggcatctt catcggaacc atccaagtga tgctgctcac   300
catcagtgaa tttaagccca catatcagga ccgagtggcc ccgccaggat taacacagat   360
tcctcagatc cagaagactg aaatttcctt tcgtcctaat gatcccaaga gctatgaggc   420
atatgtactg aacatagtta ggttcctgga aaagtacaaa gattcagccc agaggatgat   480
catgattttt gaagattgtg gcgatgtgcc cagtgaaccg aaagaacgag gagactttaa   540

tcatgaacga ggagagcgaa aggtctgcag attcaagctt gaatggctgg gaaattgctc 600
tggattaaat gatgaaactt atggctacaa agagggcaaa ccgtgcatta ttataaagct 660
caaccgagtt ctaggcttca aacctaagcc tcccaagaat gagtccttgg agacttaccc 720
agtgatgaag tataacccaa atgtccttcc cgttcagtgc actggcaagc gagatgaaga 780
taaggataaa gttggaaatg tggagtattt tggactgggc aactcccctg gttttcctct 840
gcagtattat ccgtactatg gcaaactcct gcagcccaaa tacctgcagc ccctgctggc 900
cgtacagttc accaatctta ccatggacac tgaaattcgc atagagtgta aggcgtacgg 960
tgagaacatt gggtacagtg agaaagaccg ttttcaggga cgttttgatg taaaaattga 1020
agttaagagc tgatcacaag cacaaatctt tcccactagc catttaataa gttaaaaaaa 1080
gatacaaaaa caaaaaccta ctagtcttga acaaactgtc atacgtatgg gacctacact 1140
taatctatat gctttacact agctttctgc atttaatagg ttagaatgta aattaaagtg 1200
tagcaatagc aacaaaatat ttattctact gtaaatgaca aaagaaaaag aaaaattgag 1260
ccttgggacg tgcccatttt tactgtaaat tatgattccg taactgactt gtagtaagca 1320
gtgtttctgg cccctaagta ttgctgcctt gtgtatttta tttagtgtac agtactacag 1380
gtgcatactc tggtcatttt tcaagccatg ttttattgta tctgttttct actttatgtg 1440
agcaaggttt gctgtccaag gtgtaaatat tcaacgggaa taaaactggc atggtaattt 1500
tttttttttt tttttttttg tttttggctc ctttcaaagg taatggccca tcgatgagca 1560
tttttaacat actccatagt cttttcctgt ggtgttaggt ctttattttt atttttttcc 1620
tgggggctgg ggtgggggtt tgtcatgggg gaactgccct ttaaatttta agtgacacta 1680
cagaaaaaca caaaaaggtg atgggttgtg ttatgcttgt attgaatgct gtcttgacat 1740
ctcttgcctt gtcctccggt atgttctaaa gctgtgtctg agatctggat ctgcccatca 1800
ctttggctag tgacagggct aattaatttg ctttatacat tttcttttac tttccttttt 1860
tcctttctgg aggcatcaca tgctggtgct gtgtctttat gaatgtttta accattttca 1920
tggtggaaga attttatatt tatgcagttg tacaatttta tttttttctg caagaaaaag 1980
tgtaatgtat gaaataaacc aaagtcactt gtttgaaaat aaatctttat tttgaacttt 2040
ataaaaagca atgcagtacc ccatagactg gtgttaaatg ttgtctacag tgcaaaatcc 2100
atgttctaac atatgtaata attgccagga gtacagtgct cttgttgatc ttgtattcag 2160
tcaggttaaa acaacggaca ataaaagaat gaacacattc aaaaaaaaaa aa 2212

<210> 39
<211> 1050
<212> DNA
<213> Homo sapiens

<220>
<221> misc_feature
<223> Gene ID: ERBB3 ... NM_001005915

<400> 39
acacacacac acccctcccc tgccatccct ccccggactc cggctccggc tccgattgca 60
atttgcaacc tccgctgccg tcgccgcagc agccaccaat tcgccagcgg ttcaggtggc 120
tcttgcctcg atgtcctagc ctaggggccc ccgggccgga cttggctggg ctcccttcac 180
cctctgcgga gtcatgaggg cgaacgacgc tctgcaggtg ctgggcttgc ttttcagcct 240
ggcccggggc tccgaggtgg gcaactctca ggcagtgtgt cctgggactc tgaatggcct 300
gagtgtgacc ggcgatgctg agaaccaata ccagacactg tacaagctct acgagaggtg 360
tgaggtggtg atggggaacc ttgagattgt gctcacggga cacaatgccg acctctcctt 420
cctgcagtgg attcgagaag tgacaggcta tgtcctcgtg gccatgaatg aattctctac 480
tctaccattg cccaacctcc gcgtggtgcg agggacccag gtctacgatg ggaagtttgc 540
catcttcgtc atgttgaact ataacaccaa ctccagccac gctctgcgcc agctccgctt 600
gactcagctc accggtcagt tcccgatggt tccttctggc ctcacccctc agccagccca 660
agactggtac ctccttgatg atgacccaag actgctcact ctaagtgcct cttccaaggt 720
gcctgtcacc ttggccgctg tctaaaggtc cattgctccc taagcaatag agggccccca 780
gtagggggag ctaggggcat ctgctccagg gaaaggaacc ctgtgtcctt gtggggctgg 840
agtcagagct ggatctgtta accgtttttc taatttcaaa gtacagtgta ccggaggcca 900
ggcctgatgg cttacacctg taatcccagc attttgggag gccaaggagg gcagatcact 960

tgagatcagg agtttgagac cagcctggcc aacatggcga aaccctgtct ctactaaaaa 1020
tacaaaaaaa taaaataaaa taaaaaatta 1050

SEQUENCE LISTING

<110> Ming, Xu

<120> DETECTION OF LYMPH NODE METASTASIS FROM GASTRIC CARCINOMA

<130> P046248EP

<160> 39

<170> PatentIn version 3.3

<210> 1
<211> 1339
<212> DNA
<213> Homo sapiens

<220>
<221> misc_feature
<223> Gene ID: ANXA3

<400> 1
gaattccgat tagtgtgatc tcagctcaag gcaaaggtgg gatatcatgg catctatctg 60
ggttggacac cgaggaacag taagagatta tccagacttt agcccatcag tggatgctga 120
agctattcag aaagcaatca gaggaattgg aactgatgag aaaatgctca tcagcattct 180
gactgagagg tcaaatgcac agcggcagct gattgttaag gaatatcaag cagcatatgg 240
aaaggagctg aaagatgact tgaagggtga tctctctggc cactttgagc atctcatggt 300
ggccctagtg actccaccag cagtctttga tgcaaagcag ctaaagaaat ccatgaaggg 360
cgcgggaaca aacgaagatg ccttgattga aatcttaact accaggacaa gcaggcaaat 420
gaaggatatc tctcaagcct attatacagt atacaagaag agtcttggag atgacattag 480
ttccgaaaca tctggtgact tccggaaagc tctgttgact ttggcagatg gcagaagaga 540
tgaaagtctg aaagtggatg agcatctggc caaacaagat gcccagattc tctataaagc 600
tggtgagaac agatggggca cggatgaaga caaattcact gagatcctgt gtttaaggag 660
ctttcctcaa ttaaaactaa catttgatga atacagaaat atcagccaaa aggacattgt 720
ggacagcata aaaggagaat tatctgggca ttttgaagac ttactgttgg ccatagttaa 780
ttgtgtgagg aacacgccgg cctttttagc cgaaagactg catcgagcct tgaagggtat 840
tggaactgat gagtttactc tgaaccgaat aatggtgtcc agatcagaaa ttgacctttt 900
ggacattcga acagagttca agaagcatta tggctattcc ctatattcag caattaaatc 960
ggatacttct ggagactatg aaatcacact cttaaaaatc tgtggtggag atgactgaac 1020
caagaagata atctccaaag gtccacgatg ggctttccca acagctccac cttacttctt 1080
ctcatactat ttaagagaac aagcaaatat aaacagcaac ttgtgttcct aacaggaatt 1140

ttcattgttc tataacaaca acaacaaaag cgattattat tttagagcat ctcatttata 1200
atgtagcagc tcataaatga aattgaaaat ggtattaaag atctgcaact actatccaac 1260
ttatatttct gctttcaaag ttaagaatct ttatagttct actccattaa atataaagca 1320
agataataaa acggaattc 1339

<210> 2
<211> 1749
<212> DNA
<213> Homo sapiens

<220>
<221> misc_feature
<223> Gene ID: AP1M2

<400> 2
ggcgcttccg caggaagaag gaagcggcgc cgccatcgcc tcccggcgct ccctccccga 60
ctcctaagtc cttcggccgc caccatgtcc gcctcggctg tcttcattct ggacgttaag 120
ggcaagccat tgatcagccg caactacaag ggcgatgtgg ccatgagcaa gattgagcac 180
ttcatgcctt tgctggtaca gcgggaggag gaaggcgccc tggccccgct gctgagccac 240
ggccaggtcc acttcctatg gatcaaacac agcaacctct acttggtggc caccacatcg 300
aagaatgcca atgcctccct ggtgtactcc ttcctgtata agacaataga ggtattctgc 360
gaatacttca aggagctgga ggaggagagc atccgggaca actttgtcat cgtctacgag 420
ttgctggacg agctcatgga ctttggcttc ccgcagacca ccgacagcaa gatcctgcag 480
gagtacatca ctcagcagag caacaagctg gagacgggca agtcacgggt gccacccact 540
gtcaccaacg ctgtgtcctg gcgctccgag ggtatcaagt ataagaagaa cgaggtcttc 600
attgatgtca tagagtctgt caacctgctg gtcaatgcca acggcagcgt ccttctgagc 660
gaaatcgtcg gtaccatcaa gctcaaggtg tttctgtcag gaatgccaga gctgcggctg 720
ggcctcaatg accgcgtgct cttcgagctc actggccgca gcaagaacaa atcagtagag 780
ctggaggatg taaaattcca ccagtgcgtg cggctctctc gctttgacaa cgaccgcacc 840
atctccttca tcccgcctga tggtgacttt gagctcatgt cataccgcct cagcacccag 900
gtcaagccac tgatctggat tgagtctgtc attgagaagt tctcccacag ccgcgtggag 960
atcatggtca aggccaaggg gcagtttaag aaacagtcag tggccaacgg tgtggagata 1020
tctgtgcctg tacccagcga tgccgactcc cccagattca agaccagtgt gggcagcgcc 1080
aagtatgtgc cggagagaaa cgtcgtgatt tggagtatta agtctttccc ggggggcaag 1140
gagtacttga tgcgagccca ctttggcctc cccagtgtgg aaaaggaaga ggtggagggc 1200
cggcccccca tcggggtcaa gtttgagatc ccctacttca ccgtctctgg gatccaggtc 1260
cgatacatga agatcattga gaaaagtggt taccaggccc tgccctgggt tcgctacatc 1320
acccagagtg gcgattacca acttcgtacc agctagaagg gagaagagat gggggcttga 1380
acacggggct tccttacagc cccggatgca gattttagag ggagggcagg tgcgggctgt 1440
gtgtgtctgt gtgagggcag gtcctggact tggcagtttc ttgctcccag cacccgcccc 1500
ttcctcacct cttccttatt ccataggctg ggagagaaac tctctgcttc cctcgcccct 1560

ggagctttcc ccatccccct gattttatat gaagaaatag aagaggggct tgaagtcccc 1620
ctcgcgagtg ccttcttgca attacctgcc ttagcgggtg ttgcgggtcc ctccttcaca 1680
gccgctgagc ccagaggtcc cgctggcccc tcctctgaat tttaggatgt cattaaaaag 1740
atgaatcta 1749

<210> 3
<211> 1568
<212> DNA
<213> Homo sapiens

<220>
<221> misc_feature
<223> Gene ID: ATP1B1

<400> 3
cagcggcgcg tcctgcctgc agagagccag gccggagaag ccgagcggcg cagaggacgc 60
cagggcgcgc gccgcagcca cccaccctcc ggaccgcggc agctgctgac ccgccatcgc 120
catggcccgc gggaaagcca aggaggaggg cagctggaag aaattcatct ggaactcaga 180
gaagaaggag tttctgggca ggaccggtgg cagttggttt aagatccttc tattctacgt 240
aatattttat ggctgcctgg ctggcatctt catcggaacc atccaagtga tgctgctcac 300
catcagtgaa tttaagccca catatcagga ccgagtggcc ccgccaggat taacacagat 360
tcctcagatc cagaagactg aaatttcctt tcgtcctaat gatcccaaga gctatgaggc 420
atatgtactg aacatagtta ggttcctgga aaagtacaaa gattcagccc agagggatga 480
catgattttt gaagattgtg gcgatgtgcc cagtgaaccg aaagaacgag gagactttaa 540
tcatgaacga ggagagcgaa aggtctgcag attcaagctt gaatggctgg gaaattgctc 600
tggattaaat gatgaaactt atggctacaa agagggcaaa ccgtgcatta ttataaagct 660
caaccgagtt ctaggcttca aacctaagcc tcccaagaat gagtccttgg agacttaccc 720
agtgatgaag tataacccaa atgtccttcc cgttcagtgc actggcaagc gagatgaaga 780
taaggataaa gttggaaatg tggagtattt tggactgggc aactcccctg gttttcctct 840
gcagtattat ccgtactatg gcaaactcct gcagcccaaa tacctgcagc ccctgctggc 900
cgtacagttc accaatctta ccatggacac tgaaattcgc atagagtgta aggcgtacgg 960
tgagaacatt gggtacagtg agaaagaccg ttttcaggga cgttttgatg taaaaattaa 1020
attttaagtg acactacaga aaaacacaaa aaggtgatgg gttgtgttat gcttgtattg 1080
aatgctgtct tgacatctct tgccttgtcc tccggtatgt tctaaagctg tgtctgagat 1140
ctggatctgc ccatcacttt ggctagtgac agggctaatt aatttgcttt atacattttc 1200
ttttactttc cttttttcct ttctggaggc atcacatgct ggtgctgtgt ctttatgaat 1260
gttttaacca ttttcatggt ggaagaattt tatatttatg cagttgtaca attttatttt 1320
tttctgcaag aaaaagtgta atgtatgaaa taaaccaaag tcacttgttt gaaaataaat 1380
ctttattttg aactttataa aaagcaatgc agtaccccat agactggtgt taaatgttgt 1440
ctacagtgca aaatccatgt tctaacatat gtaataattg ccaggagtac agtgctcttg 1500
ttgatcttgt attcagtcag gttaaaacaa cggacaataa aagaatgaac acattcaaaa 1560

aaaaaaaa                                                          1568

<210>  4
<211>  5511
<212>  DNA
<213>  Homo sapiens

<220>
<221>  misc_feature
<223>  Gene ID: C11orf9

<400>  4
acacacacac acccctcccc tgccatccct ccccggactc cggctccggc tccgattgca      60
atttgcaacc tccgctgccg tcgccgcagc agccaccaat tcgccagcgg ttcaggtggc     120
tcttgcctcg atgtcctagc ctaggggccc ccgggccgga cttggctggg ctcccttcac     180
cctctgcgga gtcatgaggg cgaacgacgc tctgcaggtg ctgggcttgc ttttcagcct     240
ggcccggggc tccgaggtgg gcaactctca ggcagtgtgt cctgggactc tgaatggcct     300
gagtgtgacc ggcgatgctg agaaccaata ccagacactg tacaagctct acgagaggtg     360
tgaggtggtg atggggaacc ttgagattgt gctcacggga cacaatgccg acctctcctt     420
cctgcagtgg attcgagaag tgacaggcta tgtcctcgtg gccatgaatg aattctctac     480
tctaccattg cccaacctcc gcgtggtgcg agggacccag gtctacgatg ggaagtttgc     540
catcttcgtc atgttgaact ataacaccaa ctccagccac gctctgcgcc agctccgctt     600
gactcagctc accgagattc tgtcaggggg tgtttatatt gagaagaacg ataagctttg     660
tcacatggac acaattgact ggagggacat cgtgagggac cgagatgctg agatagtggt     720
gaaggacaat ggcagaagct gtcccccctg tcatgaggtt tgcaaggggc gatgctgggg     780
tcctggatca gaagactgcc agacattgac caagaccatc tgtgctcctc agtgtaatgg     840
tcactgcttt gggcccaacc ccaaccagtg ctgccatgat gagtgtgccg ggggctgctc     900
aggccctcag gacacagact gctttgcctg ccggcacttc aatgacagtg gagcctgtgt     960
acctcgctgt ccacagcctc ttgtctacaa caagctaact ttccagctgg aacccaatcc    1020
ccacaccaag tatcagtatg gaggagtttg tgtagccagc tgtccccata actttgtggt    1080
ggatcaaaca tcctgtgtca gggcctgtcc tcctgacaag atggaagtag ataaaaatgg    1140
gctcaagatg tgtgagcctt gtgggggact atgtcccaaa gcctgtgagg gaacaggctc    1200
tgggagccgc ttccagactg tggactcgag caacattgat ggatttgtga actgcaccaa    1260
gatcctgggc aacctggact ttctgatcac cggcctcaat ggagacccct ggcacaagat    1320
ccctgccctg gacccagaga agctcaatgt cttccggaca gtacgggaga tcacaggtta    1380
cctgaacatc cagtcctggc cgccccacat gcacaacttc agtgtttttt ccaatttgac    1440
aaccattgga ggcagaagcc tctacaaccg gggcttctca ttgttgatca tgaagaactt    1500
gaatgtcaca tctctgggct tccgatccct gaaggaaatt agtgctgggc gtatctatat    1560
aagtgccaat aggcagctct gctaccacca ctctttgaac tggaccaagg tgcttcgggg    1620
gcctacggaa gagcgactag acatcaagca taatcggccg cgcagagact gcgtggcaga    1680
gggcaaagtg tgtgacccac tgtgctcctc tgggggatgc tggggcccag gccctggtca    1740

gtgcttgtcc tgtcgaaatt atagccgagg aggtgtctgt gtgacccact gcaactttct 1800
gaatggggag cctcgagaat ttgcccatga ggccgaatgc ttctcctgcc acccggaatg 1860
ccaacccatg gagggcactg ccacatgcaa tggctcgggc tctgatactt gtgctcaatg 1920
tgcccatttt cgagatgggc cccactgtgt gagcagctgc ccccatggag tcctaggtgc 1980
caagggccca atctacaagt acccagatgt tcagaatgaa tgtcggccct gccatgagaa 2040
ctgcacccag gggtgtaaag gaccagagct tcaagactgt ttaggacaaa cactggtgct 2100
gatcggcaaa acccatctga caatggcttt gacagtgata gcaggattgg tagtgatttt 2160
catgatgctg ggcggcactt ttctctactg gcgtgggcgc cggattcaga ataaaagggc 2220
tatgaggcga tacttggaac ggggtgagag catagagcct ctggacccca gtgagaaggc 2280
taacaaagtc ttggccagaa tcttcaaaga gacagagcta aggaagctta aagtgcttgg 2340
ctcgggtgtc tttggaactg tgcacaaagg agtgtggatc cctgagggtg aatcaatcaa 2400
gattccagtc tgcattaaag tcattgagga caagagtgga cggcagagtt ttcaagctgt 2460
gacagatcat atgctggcca ttggcagcct ggaccatgcc cacattgtaa ggctgctggg 2520
actatgccca gggtcatctc tgcagcttgt cactcaatat ttgcctctgg gttctctgct 2580
ggatcatgtg agacaacacc ggggggcact ggggccacag ctgctgctca actggggagt 2640
acaaattgcc aagggaatgt actaccttga ggaacatggt atggtgcata gaaacctggc 2700
tgcccgaaac gtgctactca agtcacccag tcaggttcag gtggcagatt ttggtgtggc 2760
tgacctgctg cctcctgatg ataagcagct gctatacagt gaggccaaga ctccaattaa 2820
gtggatggcc cttgagagta tccactttgg gaaatacaca caccagagtg atgtctggag 2880
ctatggtgtg acagtttggg agttgatgac cttcggggca gagccctatg cagggctacg 2940
attggctgaa gtaccagacc tgctagagaa gggggagcgg ttggcacagc cccagatctg 3000
cacaattgat gtctacatgg tgatggtcaa gtgttggatg attgatgaga acattcgccc 3060
aacctttaaa gaactagcca atgagttcac caggatggcc cgagacccac cacggtatct 3120
ggtcataaag agagagagtg ggcctggaat agcccctggg ccagagcccc atggtctgac 3180
aaacaagaag ctagaggaag tagagctgga gccagaacta gacctagacc tagacttgga 3240
agcagaggag gacaacctgg caaccaccac actgggctcc gccctcagcc taccagttgg 3300
aacacttaat cggccacgtg ggagccagag ccttttaagt ccatcatctg gatacatgcc 3360
catgaaccag ggtaatcttg gggagtcttg ccaggagtct gcagtttctg ggagcagtga 3420
acggtgcccc cgtccagtct ctctacaccc aatgccacgg ggatgcctgg catcagagtc 3480
atcagagggg catgtaacag gctctgaggc tgagctccag gagaaagtgt caatgtgtag 3540
gagccggagc aggagccgga gcccacggcc acgcggagat agcgcctacc attcccagcg 3600
ccacagtctg ctgactcctg ttaccccact ctccccaccc gggttagagg aagaggatgt 3660
caacggttat gtcatgccag atacacacct caaaggtact ccctcctccc gggaaggcac 3720
cctttcttca gtgggtctca gttctgtcct gggtactgaa gaagaagatg aagatgagga 3780
gtatgaatac atgaaccgga ggagaaggca cagtccacct catcccccta ggccaagttc 3840
ccttgaggag ctgggttatg agtacatgga tgtggggtca gacctcagtg cctctctggg 3900
cagcacacag agttgcccac tccaccctgt acccatcatg cccactgcag gcacaactcc 3960
agatgaagac tatgaatata tgaatcggca acgagatgga ggtggtcctg ggggtgatta 4020
tgcagccatg ggggcctgcc cagcatctga gcaagggtat gaagagatga gagcttttca 4080
ggggcctgga catcaggccc cccatgtcca ttatgcccgc ctaaaaactc tacgtagctt 4140
agaggctaca gactctgcct ttgataaccc tgattactgg catagcaggc ttttccccaa 4200

ggctaatgcc cagagaacgt aactcctgct ccctgtggca ctcagggagc atttaatggc 4260
agctagtgcc tttagagggt accgtcttct ccctattccc tctctctccc aggtcccagc 4320
cccttttccc cagtcccaga caattccatt caatctttgg aggcttttaa acattttgac 4380
acaaaattct tatggtatgt agccagctgt gcactttctt ctctttccca accccaggaa 4440
aggttttcct tattttgtgt gctttcccag tcccattcct cagcttcttc acaggcactc 4500
ctggagatat gaaggattac tctccatatc ccttcctctc aggctcttga ctacttggaa 4560
ctaggctctt atgtgtgcct ttgtttccca tcagactgtc aagaagagga aagggaggaa 4620
acctagcaga ggaaagtgta attttggttt atgactctta accccctaga aagacagaag 4680
cttaaaatct gtgaagaaag aggttaggag tagatattga ttactatcat aattcagcac 4740
ttaactatga gccaggcatc atactaaact tcacctacat tatctcactt agtcctttat 4800
catccttaaa acaattctgt gacatacata ttatctcatt ttacacaaag ggaagtcggg 4860
catggtggct catgcctgta atctcagcac tttgggaggc tgaggcagaa ggattacctg 4920
aggcaaggag tttgagacca gcttagccaa catagtaaga cccccatctc tttaaaaaaa 4980
aaaaaaaaaa aaaaaaaaaa actttagaac tgggtgcagt ggctcatgcc tgtaatccca 5040
gccagcactt tgggaggctg agatgggaag atcacttgag cccagaatta gagataagcc 5100
tatggaaaca tagcaagaca ctgtctctac aggggaaaaa aaaaaaagaa actgagcctt 5160
aaagagatga aataaattaa gcagtagatc caggatgcaa aatcctccca attcctgtgc 5220
atgtgctctt attgtaaggt gccaagaaaa actgatttaa gttacagccc ttgtttaagg 5280
ggcactgttt cttgtttttg cactgaatca agtctaaccc caacagccac atcctcctat 5340
acctagacat ctcatctcag gaagtggtgg tgggggtagt cagaaggaaa aataactgga 5400
catctttgtg taaaccataa tccacatgtg ccgtaaatga tcttcactcc ttatccgagg 5460
gcaaattcac aaggatcccc aagatccact tttagaagcc attctcatcc a 5511

<210> 5
<211> 4098
<212> DNA
<213> Homo sapiens

<220>
<221> misc_feature
<223> Gene ID: C1orf106

<400> 5
   atgctgcaaa tgccgaagtt aaatgaaata cctccgggga gggcaggccg cagggaggct 60
   cggggggagg gaagatggcc tggacaaaca ggtcctgaag ctgcgaggct ggagtggagg 120
   gcgcaggggc aggcgggcgg cgccagagct ccatgggaca gctggggaag ctccaggcta 180
   cctacacaac ctggcccagg ctggtcacgg tgtcccccct ccctgctctg tgccctctcc 240
   ttccagaaat ccaccatgga gagtaaggat gaggtcagcg acaccgacag tggcatcatc 300
   ctgcagtctg gccccgacag cccggtctcc ccaatgaagg agctgaccca tgcagtgcac 360
   aagcagcaga gggccctgga agcgaggctg gaggcctgcc tggaggagct gaggagactc 420
   tgccttcggg aagcggagct gacgggcacc ttgccagcgg agtatcccct caaaccaggg 480
   gaaaaggccc ccaaggttcg ccgcaggatc ggagcggctt acaaactgga tgactgggcc 540
   ttgcacagag aggaccccct aagcagcctg gagcgccagc tggccctgca gctgcagatc 600
   acagaggcag cccgtcggct gtgcctggag gagaacctca gcaggcaggc tcggcggcag 660
   cggaagcact ccatgctgca ggaggagaag aagctgcagg agctccagcg ctgcctggtc 720
   gagcggcggc gcaatagcga gccacctccg gctgctgctc tccccctggg ccgagagctc 780
   agtgcctctg atgacagctc cctgtcagat gggctcctcc tggaggaaga ggaatcccaa 840
   gtgccaaaac ctcctccaga gtctccagcc ccaccttctc ggcctctccc accccaaacc 900
   cttgagggtc tgcagccaac aggacctgag gctgggagcc cagaacgggc tccagtccag 960
   aacagcccct ggaaggaaac cagcctggac cacccctatg agaagcccag gaagtcttct 1020
   gagccctgga gcgagtccag cagcccagcc accacaccac aggatgggcc cagtgcctcc 1080
   agcctgtggc ttctggagcc tgcctcctac cacgtggttc ccatccgtgg tgttcctggc 1140
   cagtggcagg gccgcaccag tgccccagcc acccctgaga tacaggggag gaggggccag 1200
   tcgcagtctc tgagggtgga ttccttccgg gcgggtcctg agggccgagg tcgcagcgcc 1260
   tttccccgcc gccgccccac tcactacacg gtgacagtgc cagattcctg ctttcccgcg 1320
   accaagcccc cgctgcccca cgccgcctgc cactcctgct cagaagacag tggctctgac 1380
   gtctccagca tctcccaccc cacttcgccg ggcagcagca gccccgacat ctcctttctg 1440
   cagcctctct cccctcccaa gacccatcgt caccgcgggg cctgggtccc agccggcagc 1500
   agagagctgg tcgcccacca ccccaagcta ctgctgccgc ctggctattt cccggcgggg 1560
   cggtacgtgg tggtggctga gagccccctg ccgcctggcg agtgggagct gtgccgcgca 1620
   gccccgggcc ctgcttacga ggaggagggc actcccctgc gctaccagcg tctggtgccc 1680
   tcccgcagcc gcatcgtgcg gacgccctcc ctgaaggaca gcccggcagg ccgggggctc 1740
   agcaaggccg ccgtgtccga ggagctcaag tggtggcacg agcgtgcacg cctccggagc 1800
   acccgacccc actcactgga ccgccaagga gctttccggg tcaggagcct gccccttggg 1860
   agagagggct tcggacgagc cctgggaccc cgggcacagg tgcccacagt ttgtgtgctg 1920
   tggagatcgc ctgatggggc ccctgtgcaa gtctttgtac ctgaaaaagg agagatcatc 1980
   agccaggtgt aactctgcgc cccacgctgg aaaaaactgt ttcatagagg ggctgggctg 2040
   agaccccccc acccctgagt gcctctttca gctcccccat ccccatcgca ggccgatgac 2100
   ctggagctaa gaccttttat tatttttttt ttacacgact tttttcagaa gccctgacct 2160
   aaggatttat atatgtggat tgtcctcaat acccctgtga tatgattatg ttttatcccc 2220
   cagagtttgg cctactggac ttaaggcctt gcctgtctga ctgacagcct ctatctcctt 2280
   atataagaca agtggcaggg gacgagtgaa gcagagtgag ccaccttggg agttctccaa 2340
   cgctctgtgc tctggttcta agaaattccc tggggaactg cccctggccc tcctgtccca 2400
   ctattgctgg aggctggaca tggtacatac tcatgcacat gactctcccc catttcccag 2460
   gtctctgggt accccagcct gggctggggg agaatctctt cccccttttc taatgtgctc 2520
   tgtgatgcac acaccaagtg gtaggtcaaa ggtcagtata tcccggtggt gtattgtctt 2580
   gctagaccct gctattttcc tgacccccta aatcctcttt agggacccag tcactatacc 2640
   ctgtctatgc cctgtgggct cccagacccc tgagctttga gtcagtggca tcacagtttg 2700
   tagcctcagg gggtctggct gggggctggt ccatgcttgt ggttagtgga cagcagccac 2760
   cctttgacag ctacctctgg acatctcaag ggcttgcagc cccactgctc cttctaacat 2820
   tttgtttgtt tttgagatgg agtctcgctc tgtcgcccag gctggaatgc tgtagcaaga 2880
   tttcggctca ctgcaacccc cgtctcccgg gttcaagcga atctcctgcc tcagccttct 2940
   gagtagctgg gattacaggc aagcaccacc atgcctgcta attttttatt tttagtagag 3000
   atggggtttc atcatgttgg ccaggctggt ctcgaactcc tgacctcaag tgatccacct 3060
   gccttggcct ttcaaagtgc tgggattaca ggcatgagcc accgcacctg gcccttctaa 3120
   cgttttttca tcatagtccc aaaaaccaat actttacaag tggtttttga aaggcaccac 3180
   ttttgtggca tgttctggtt gggagaggga gtcacagttc ctactccccc caccagctat 3240
   gcttctgctc tgagaaggtg gttatttata caaacatgga catactcact cccaagggct 3300
   gatgagatgc tgaattttct ttgggggcat tcattaattg tcccagctgc agcgactgga 3360
   gcaagtctgg aagctgcctg tgctaagacc acccagctgt ccctgggttc tcatcctagg 3420
   gccttctttg cttccaggtc aggggacctg cttcaatgag aaagcaactg aattgaggct 3480
   aggagaggta gggagagctg agttctgact tcacctgtgc agaactctct gcccccatgt 3540
   tacctggact ggaacagact gtgaatatag cagaaggttc caagaactct ggtgtctgac 3600
   ctagaagagg cacagttctc tctactggaa agaaaacgat gtagccgatt gcacaagggt 3660
   gccaagggaa gacccaggat ggcccatcaa aggaacctgg gggaggatgc aggaggctga 3720
   agggatgcac ctggcatttc tctcactgtg ctcttaccgc atcagcaacc cccaactttt 3780
   gggcctactc tgccccccat gcgtgaatac cctgcttgga tgctgtgctt ttccggtttg 3840
   tctctaagcc cctttctcca gggcatgttg gtttccctgg cctctcagtg tcctaactgg 3900
   agcccagagt gccttgttct gagccaggag acggctgagc actggccctc cacacctaag 3960
   cgtcctttac attaacttat tggtcttgta taacacctgg tgccattgcc aagtggctgt 4020
   gtcctcagct acagagctgg aattgtgtgg ggtttagtgc taaatacttc aataaagtct 4080
   gttttttgtg attggctg 4098

<210> 6
<211> 4828
<212> DNA
<213> Homo sapiens

<220>
<221> misc_feature
<223> Gene ID: CDH1

<400> 6
agtggcgtcg gaactgcaaa gcacctgtga gcttgcggaa gtcagttcag actccagccc 60
gctccagccc ggcccgaccc gaccgcaccc ggcgcctgcc ctcgctcggc gtccccggcc 120
agccatgggc ccttggagcc gcagcctctc ggcgctgctg ctgctgctgc aggtctcctc 180
ttggctctgc caggagccgg agccctgcca ccctggcttt gacgccgaga gctacacgtt 240
cacggtgccc cggcgccacc tggagagagg ccgcgtcctg ggcagagtga attttgaaga 300
ttgcaccggt cgacaaagga cagcctattt ttccctcgac acccgattca aagtgggcac 360
agatggtgtg attacagtca aaaggcctct acggtttcat aacccacaga tccatttctt 420
ggtctacgcc tgggactcca cctacagaaa gttttccacc aaagtcacgc tgaatacagt 480
ggggcaccac caccgccccc cgccccatca ggcctccgtt tctggaatcc aagcagaatt 540
gctcacattt cccaactcct ctcctggcct cagaagacag aagagagact gggttattcc 600

tcccatcagc tgcccagaaa atgaaaaagg cccatttcct aaaaacctgg ttcagatcaa 660
atccaacaaa gacaaagaag gcaaggtttt ctacagcatc actggccaag gagctgacac 720
accccctgtt ggtgtcttta ttattgaaag agaaacagga tggctgaagg tgacagagcc 780
tctggataga gaacgcattg ccacatacac tctcttctct cacgctgtgt catccaacgg 840
gaatgcagtt gaggatccaa tggagatttt gatcacggta accgatcaga atgacaacaa 900
gcccgaattc acccaggagg tctttaaggg gtctgtcatg gaaggtgctc ttccaggaac 960
ctctgtgatg gaggtcacag ccacagacgc ggacgatgat gtgaacacct acaatgccgc 1020
catcgcttac accatcctca gccaagatcc tgagctccct gacaaaaata tgttcaccat 1080
taacaggaac acaggagtca tcagtgtggt caccactggg ctggaccgag agagtttccc 1140
tacgtatacc ctggtggttc aagctgctga ccttcaaggt gaggggttaa gcacaacagc 1200
aacagctgtg atcacagtca ctgacaccaa cgataatcct ccgatcttca atcccaccac 1260
gtacaagggt caggtgcctg agaacgaggc taacgtcgta atcaccacac tgaaagtgac 1320
tgatgctgat gcccccaata ccccagcgtg ggaggctgta tacaccatat tgaatgatga 1380
tggtggacaa tttgtcgtca ccacaaatcc agtgaacaac gatggcattt tgaaaacagc 1440
aaagggcttg gattttgagg ccaagcagca gtacattcta cacgtagcag tgacgaatgt 1500
ggtacctttt gaggtctctc tcaccacctc cacagccacc gtcaccgtgg atgtgctgga 1560
tgtgaatgaa gcccccatct ttgtgcctcc tgaaaagaga gtggaagtgt ccgaggactt 1620
tggcgtgggc caggaaatca catcctacac tgcccaggag ccagacacat ttatggaaca 1680
gaaaataaca tatcggattt ggagagacac tgccaactgg ctggagatta atccggacac 1740
tggtgccatt tccactcggg ctgagctgga cagggaggat tttgagcacg tgaagaacag 1800
cacgtacaca gccctaatca tagctacaga caatggttct ccagttgcta ctggaacagg 1860
gacacttctg ctgatcctgt ctgatgtgaa tgacaacgcc cccataccag aacctcgaac 1920
tatattcttc tgtgagagga atccaaagcc tcaggtcata aacatcattg atgcagacct 1980
tcctcccaat acatctccct tcacagcaga actaacacac ggggcgagtg ccaactggac 2040
cattcagtac aacgacccaa cccaagaatc tatcattttg aagccaaaga tggccttaga 2100
ggtgggtgac tacaaaatca atctcaagct catggataac cagaataaag accaagtgac 2160
caccttagag gtcagcgtgt gtgactgtga aggggccgcc ggcgtctgta ggaaggcaca 2220
gcctgtcgaa gcaggattgc aaattcctgc cattctgggg attcttggag gaattcttgc 2280
tttgctaatt ctgattctgc tgctcttgct gtttcttcgg aggagagcgg tggtcaaaga 2340
gcccttactg cccccagagg atgacacccg ggacaacgtt tattactatg atgaagaagg 2400
aggcggagaa gaggaccagg actttgactt gagccagctg cacaggggcc tggacgctcg 2460
gcctgaagtg actcgtaacg acgttgcacc aaccctcatg agtgtccccc ggtatcttcc 2520
ccgccctgcc aatcccgatg aaattggaaa ttttattgat gaaaatctga aagcggctga 2580
tactgacccc acagccccgc cttatgattc tctgctcgtg tttgactatg aaggaagcgg 2640
ttccgaagct gctagtctga gctccctgaa ctcctcagag tcagacaaag accaggacta 2700
tgactacttg aacgaatggg gcaatcgctt caagaagctg gctgacatgt acggaggcgg 2760
cgaggacgac taggggactc gagagaggcg ggccccagac ccatgtgctg ggaaatgcag 2820
aaatcacgtt gctggtggtt tttcagctcc cttcccttga gatgagtttc tggggaaaaa 2880
aaagagactg gttagtgatg cagttagtat agctttatac tctctccact ttatagctct 2940
aataagtttg tgttagaaaa gtttcgactt atttcttaaa gctttttttt ttttcccatc 3000
actctttaca tggtggtgat gtccaaaaga tacccaaatt ttaatattcc agaagaacaa 3060

ctttagcatc agaaggttca cccagcacct tgcagatttt cttaaggaat tttgtctcac 3120
ttttaaaaag aaggggagaa gtcagctact ctagttctgt tgttttgtgt atataatttt 3180
ttaaaaaaaa tttgtgtgct tctgctcatt actacactgg tgtgtccctc tgcctttttt 3240
ttttttttta agacagggtc tcattctatc ggccaggctg gagtgcagtg gtgcaatcac 3300
agctcactgc agccttgtcc tcccaggctc aagctatcct tgcacctcag cctcccaagt 3360
agctgggacc acaggcatgc accactacgc atgactaatt ttttaaatat ttgagacggg 3420
gtctccctgt gttacccagg ctggtctcaa actcctgggc tcaagtgatc ctcccatctt 3480
ggcctcccag agtattggga ttacagacat gagccactgc acctgcccag ctccccaact 3540
ccctgccatt ttttaagaga cagtttcgct ccatcgccca ggcctgggat gcagtgatgt 3600
gatcatagct cactgtaacc tcaaactctg gggctcaagc agttctccca ccagcctcct 3660
ttttattttt ttgtacagat ggggtcttgc tatgttgccc aagctggtct taaactcctg 3720
gcctcaagca atccttctgc cttggccccc caaagtgctg ggattgtggg catgagctgc 3780
tgtgcccagc ctccatgttt taatatcaac tctcactcct gaattcagtt gctttgccca 3840
agataggagt tctctgatgc agaaattatt gggctctttt agggtaagaa gtttgtgtct 3900
ttgtctggcc acatcttgac taggtattgt ctactctgaa gacctttaat ggcttccctc 3960
tttcatctcc tgagtatgta acttgcaatg ggcagctatc cagtgacttg ttctgagtaa 4020
gtgtgttcat taatgtttat ttagctctga agcaagagtg atatactcca ggacttagaa 4080
tagtgcctaa agtgctgcag ccaaagacag agcggaacta tgaaaagtgg gcttggagat 4140
ggcaggagag cttgtcattg agcctggcaa tttagcaaac tgatgctgag gatgattgag 4200
gtgggtctac ctcatctctg aaaattctgg aaggaatgga ggagtctcaa catgtgtttc 4260
tgacacaaga tccgtggttt gtactcaaag cccagaatcc ccaagtgcct gcttttgatg 4320
atgtctacag aaaatgctgg ctgagctgaa cacatttgcc caattccagg tgtgcacaga 4380
aaaccgagaa tattcaaaat tccaaatttt ttcttaggag caagaagaaa atgtggccct 4440
aaagggggtt agttgagggg tagggggtag tgaggatctt gatttggatc tctttttatt 4500
taaatgtgaa tttcaacttt tgacaatcaa agaaaagact tttgttgaaa tagctttact 4560
gtttctcaag tgttttggag aaaaaaatca accctgcaat cactttttgg aattgtcttg 4620
atttttcggc agttcaagct atatcgaata tagttctgtg tagagaatgt cactgtagtt 4680
ttgagtgtat acatgtgtgg gtgctgataa ttgtgtattt tctttggggg tggaaaagga 4740
aaacaattca agctgagaaa agtattctca aagatgcatt tttataaatt ttattaaaca 4800
attttgttaa accataaaaa aaaaaaaa 4828

<210> 7
<211> 5289
<212> DNA
<213> Homo sapiens

<220>
<221> misc_feature
<223> Gene ID: COBL

<400> 7

ccagcccctt ccgtccgccg cgctccgccg ctgtcctccg ccggcccgcg tgggaggacg 60
atgggtcagc gcggaaaaag tgaatgaggg cggcagcggt ggcggcggta gccagcgcct 120
gactcggcgg gcccggagca ccgcccgcgt cccggccccc ggcaccatgg acgcgccgcg 180
cgcctcggcg gccaagcccc cgaccgggag gaagatgaag gctcgtgctc ccccacctcc 240
tggaaaggct gccactctgc atgtgcacag tgaccagaag cccccccacg atggggccct 300
cgggtcgcag cagaacttgg ttcgcatgaa ggaggcgctg agggccagca ccatggacgt 360
caccgtggtc ctgcctagcg ggctggagaa gaggagcgtg ctcaatggga gccatgcgat 420
gatggaccta ctggttgaac tttgccttca gaaccacctg aatccatccc accatgccct 480
tgaaattcgg tcttcagaaa cccaacaacc tttgagtttt aagccaaata ctttgattgg 540
gaccctgaat gtgcatactg tgtttctgaa agaaaaagtt cctgaagaga aggttaagcc 600
tggtccccct aaggtgcctg agaaatctgt gcgtttggtc gtgaattacc tgcggacaca 660
aaaagctgtt gtgcgtgtga gccctgaggt tcctctccag aatattctcc cagtcatttg 720
tgcaaagtgt gaggtcagcc cagagcacgt ggttctcctc agggacaaca ttgccggaga 780
ggagctggag ctgtccaagt ccctgaacga gctcgggata aaggagctct acgcgtggga 840
caacagaaga gaaaccttta ggaaatcatc acttggcaat gatgagacag ataaagagaa 900
gaaaaaattt ctgggatttt tcaaagttaa taaaagaagc aatagtaagg gctgtttaac 960
gacccccaac tccccatcca tgcactcacg ttctcttacg ctgggtccat ccctctcgct 1020
gggcagcatc tcaggggtgt ccgtgaagtc ggagatgaag aagcgccgag cccctcctcc 1080
tccaggttca gggccacctg tgcaagacaa ggcatcggaa aaggtatctc ttgggtcaca 1140
gattgattta cagaagaaga agcggcgagc gccagctccc cctccaccac agccaccacc 1200
accgagtccc ctgatcccca accgcactga ggataaggag gagaacagga agagcacgat 1260
ggtaagcctg cccctggggt ctggcagcca ctgcagcccg gatggagccc cgcaggtgct 1320
gtcagaggcg gaggagaccg tgtcagttgg cagctgtttt gcgtcggagg acacgaccga 1380
ggactcagga gtgatgagtt ccccctcaga catcgtctct ctggactcgc agcaggacag 1440
catgaaatac aaagacaagt gggccacaga ccaggaagac tgcagtgacc aggacctcgc 1500
tggaacccca gacctgggtc cccagaagag ccccttgtgg gagaagaatg gctctgagaa 1560
ctcacatctg agaactgaaa aagctgtcac agcctcaaat gatgaagaag acctattaat 1620
tgctggagag ttccgtaaaa cccttgcaga acttgatgaa gacctggaag aaatggaaga 1680
cagctatgaa acagacacca gctccctcac cagctccatc catggtgcat ccaaccactg 1740
cccacaggat gccatgatcc ctcacggcga cacagatgca atcccagtaa cattcatagg 1800
ggaagtttca gatgatcctg tggattcggg gttgttttcc aatagaaaca acaatgctgg 1860
gtctttcgac tcggagggtg ttgccagcag gagagactcc ctggcgccac ttcaagctga 1920
gcacagccag ccccatgaaa aggcaaggga ggaagtacct gccctgcacc ccgcttccca 1980
tgacgtcgga aaaggaatcc gtgtggcctt atctaacatc tctaaagatg ggaatctaat 2040
ggaaacggcg cccagggtga cttcttttgc ttcgaatctt cacacagaca acctaaatgc 2100
aaaagtgaaa gacaaagtgt atggctgtgc tgacggggag aggactcaag ccacagagag 2160
agtgaattcc caaccggtga atgaaaagga cagcaacgat aaaaacgctg ccttggcacc 2220
aacatcatgg caccaacgag gccaaaaccc agggaaaagc tacagactta agcacggcct 2280
cacaacgtat aaaatcattc ctccaaagtc agagatgcga tgttacgaca gagatgtgtc 2340
cctctccacc ggagccatta agattgacga gctggggaac ttggtgagtc ctcacgccac 2400
cggcatcagg atcatttccc tgtcttcgtc tgtgcctgaa gcagaatctc agcccattgg 2460

gaaagtcaga gagttctgga ggtgcaactc tgtggaaaaa cacctgggcc gaccctcaga 2520
gagctctgcc aggggacccc cctccacccc tgtgcccacg cagacgcaga atccagagag 2580
cagactccaa gcagacccca agccaatatc gccccagcag aagtctgccc accatgaggg 2640
ccggaacccc ctaggggagg ggagaaacca gccccccacc atgggcatgg gtcacgtgag 2700
ggtgccagca gctcacacca cagaagtcac atttctcaag cctcagagaa gaacgtccag 2760
ccagtatgtg gcctctgcca ttgccaagcg cataggagcc ccaaaagtcc atgctgatgt 2820
ggtgaggcca cacggttatg ccgagaaggg ttatgcaggc aaggcgccag tgctggctgc 2880
accacctgtc actgtgaaag atgacaggac atccagccca cactcagaga cacaaggatg 2940
gaaggatggt gcccagtggc cctgtgtcac tcctcccaac aaccacgggg aagatttggc 3000
agtgggagcc cctcctaggg gggaggtcat tggcccacac aggaagttgt ctactcagga 3060
cagacctgct gctatccaca gaagctcctg tttctcactg gttcagtctt cccagaggga 3120
tcgtgtttct gtgggacaga gctgtggttt cagtggaaag caaagcacaa gtagccagga 3180
ggccagctca gcatctgagc ccagacgcgc acctgatggt acagacccac cccctccaca 3240
cacatctgac actcaggcct gcagcaggga gctggtcaat ggctctgtgc gcgccccagg 3300
ccacggcgag ccttcccacc ctccaggagg gtctggcaca gaaagccaca ttctcctaga 3360
aagagaggaa aagcccagtg tgttctctac agatggaaat gaaacagaca gtatttggcc 3420
acccagcatt tttgggccga agaaaaaatt caaacctgtt gtccagagac cagtcccaaa 3480
agacacatcc ctgcactctg ccctgatgga agccatccac tctgcgggag ggaaggacag 3540
actacgcaag acggcagaac acaccgggga gggcaggcca gcgaaactgt cctacacgga 3600
ggcagagggc gaacgatctg cactgctggc agctatccgc gggcacagcg gcacctgcag 3660
cctgaggaag gtggcatcct ctgcttctga ggagctccag agcttccgag atgccgcact 3720
ctctgctcag ggctcggaaa gtcctctgct agaagacctt ggtcttctgt ccccaccagc 3780
cattccccca ccaccccctc caccctccca ggctctctct gcaccaagga cggcctccag 3840
gttcagcacg ggcaccctca gcaacaccgc agacgcaagg caagccttga tggacgccat 3900
ccgctccggc acaggggctg cgagactgag aaaggtgccc ttgctcgtgt gaatgaacag 3960
aggccagaga attgcagaaa cccacctgta atatgcccac aggcgtcact caaggagtct 4020
gcatagccag gaacatcaag ttttggtgtt actttcttgt ctacaggcca aatgcgcaca 4080
gttgattttc ggtgtgttcc tgtataacgg cttgaaagga aaggagaaga tgctgtgtcg 4140
ctgcgcagct cgtgtgccaa ggaaatgtgt tttgcctctg aatatcttaa gttgctaata 4200
aacgattctt gttggcaggt taatgggact ataaatgctg gagcccagca ctcacactta 4260
attatattta tgggcaaagt aaaaagaatc atgccttaga ctgattttgg acctttgaga 4320
cttttaccac atacagatgg atgaaactac ttactgattt aagatctttt gaattttaga 4380
aaaaacatat gaatcagatg gaacaatctg aatgtgatat gctccatgat gctgtgtgtt 4440
ttggtagtaa gaaaaaaaat tgggaggctt ttcaaaggtg gctttgttag gtagtaaaac 4500
tgctggattc ttcttcctca aatatttggg gctaacactt cacagagggt cacagtccat 4560
gatctgacca actaagcttt taaaaatcaa ctattaccac ccagaaaaca gtcctacgtg 4620
atcatgtggc cagggcatgc ccgagtcctg caagggtggc gggtggctga ggtcacacca 4680
ccactggagt ggatgggcag tgccccacat ggacagccat tccccaggct atgaagtgac 4740
ataggcctcc cccacggtgc ctgtgtacgg agcctgatga cttcactggg agccttctgg 4800
aatcctgcag agggtcaagg agcagggatg ttggatgccc acctgtcaag agttcagatc 4860
aaagttgcgc tgagagcatc acaattttgg ttcagccttg acgcgtttgt ccaacagctc 4920

attggctccc tttgtatgat atcgtggtct tctcacatgg tgcccagtca ccaatattta 4980
taatgaggtc taactacagc agtagttttt catatatatc tctaaaacat tttgttatat 5040
tgaaaaaagt aatagaaatc aagatgtgtt gatgaaataa aatgtgtatc tgagtgagaa 5100
aacaagtatg gtgaggtcac tttaatgttt cacagcgatc tcagatctag gcctcaggta 5160
gaatggaagc tgttctgcat tcactgatta acgttgctaa actcttggtg aggcacgagc 5220
taccagccaa ttgctcttca tcacagctat ctgtctttta gtgccacaaa taaaagaaaa 5280
tcaaatctt 5289

<210> 8
<211> 2080
<212> DNA
<213> Homo sapiens

<220>
<221> misc_feature
<223> Gene ID: CYP3A7

<400> 8
tcactgctgt gcagggcagg aaagctccac acacacagcc cagcaaacag cagcacgctg 60
ctgaaaaaaa gactcagagg agagagataa ggaaggaaag tagtgatgga tctcatccca 120
aacttggccg tggaaacctg gcttctcctg gctgtcagcc tgatactcct ctatctatat 180
ggaacccgta cacatggact ttttaagaag cttggaattc cagggcccac acctctgcct 240
tttttgggaa atgctttgtc cttccgtaag ggctattgga cgtttgacat ggaatgttat 300
aaaaagtata gaaaagtctg gggtatttat gactgtcaac agcctatgct ggctatcaca 360
gatcccgaca tgatcaaaac agtgctagtg aaagaatgtt attctgtctt cacaaaccgg 420
aggcctttcg ggccagtggg atttatgaaa aatgccatct ctatagctga ggatgaagaa 480
tggaagagaa tacgatcatt gctgtctcca acattcacca gcggaaaact caaggagatg 540
gtccctatca ttgcccagta tggagatgtg ttggtgagaa atctgaggcg ggaagcagag 600
acaggcaagc ctgtcacctt gaaacacgtc tttggggcct acagcatgga tgtgatcact 660
agcacatcat ttggagtgag catcgactct ctcaacaatc cacaagaccc ctttgtggaa 720
aacaccaaga agcttttaag atttaatcca ttagatccat tcgttctctc aataaaagtc 780
tttccattcc ttaccccaat tcttgaagca ttaaatatca ctgtgtttcc aagaaaagtt 840
ataagttttc taacaaaatc tgtaaaacag ataaaagaag gtcgcctcaa agagacacaa 900
aagcaccgag tggatttcct tcagctgatg attgactctc agaattcaaa agactctgag 960
acccacaaag ctctgtctga tctggagctc atggcccaat caattatctt tatttttgct 1020
ggctatgaaa ccacgagcag tgttctctcc ttcattatat atgaactggc cactcaccct 1080
gatgtccagc agaaagtgca gaaggaaatt gatacagttt tacccaataa ggcaccaccc 1140
acctatgata ctgtgctaca gttggagtat cttgacatgg tggtgaatga aacactcaga 1200
ttattcccag ttgctatgag acttgagagg gtctgcaaaa aagatgttga aatcaatggg 1260
atgtttattc ccaaaggggt ggtggtgatg attccaagct atgttcttca tcatgaccca 1320
aagtactgga cagagcctga gaagttcctc cctgaaaggt tcagtaaaaa gaacaaggac 1380

aacatagatc cttacatata cacacccttt ggaagtggac ccagaaactg cattggcatg 1440
aggtttgctc tcgtgaacat gaaacttgct ctagtcagag tccttcagaa cttctccttc 1500
aaaccttgta aagaaacaca gatccccctg aaattacgct ttggaggact tcttctaaca 1560
gaaaaaccca ttgttctaaa ggctgagtca agggatgaga ccgtaagtgg agcctgattt 1620
ccctaaggac ttctggtttg ctctttaaga aagctgtgcc ccagaacacc agagacctca 1680
aattacttta caaatagaac cctgaaatga agacgggctt catccaatgt gctgcataaa 1740
taatcaggga ttctgtacgt gcattgtgct ctctcatggt ctgtatagag tgttatactt 1800
ggtaatatag aggagatgac caaatcagtg ctggggaagt agatttggct tctctgcttc 1860
tcataggact atctccacca cccccagtta gcaccattaa ctcctcctga gctctgataa 1920
cataattaac atttctcaat aatttcaacc acaatcatta ataaaaatag gaattatttt 1980
gatggctcta acagtgacat ttatatcatg tgttatatct gtagtattct atagtaagct 2040
ttatattaag caaatcaata aaaacctctt tacaaaagta 2080

<210> 9
<211> 3516
<212> DNA
<213> Homo sapiens

<220>
<221> misc_feature
<223> Gene ID: DSG2

<400> 9
tcgagggtgc gatggcgcgg acgcgggacc gcgtacgcct gctgcttctc ctgatctgct 60
ttaacgttgg aagtggactt cacttacagg tcttaagcac aagaaatgaa aataagctgc 120
ttcctaaaca tcctcattta gtgcggcaaa agcgcgcctg gatcaccgcc cccgtggctc 180
ttcgggaggg agaggatctg tccaagaaga atccaattgc caagatacat tctgatcttg 240
cagaagaaag aggactcaaa attacttaca aatacactgg aaaagggatt acagagccac 300
cttttggtat atttgtcttt aacaaagata ctggagaact gaatgttacc agcattcttg 360
atcgagaaga aacaccattt tttctgctaa caggttacgc tttggatgca agaggaaaca 420
atgtagagaa acccttagag ctacgcatta aggttcttga tatcaatgac aacgaaccag 480
tgttcacaca ggatgtcttt gttgggtctg ttgaagagtt gagtgcagca catactcttg 540
tgatgaaaat caatgcaaca gatgcagatg agcccaatac cctgaattcg aaaatttcct 600
atagaatcgt atctctggag cctgcttatc ctccagtgtt ctacctaaat aaagatacag 660
gagagattta tacaaccagt gttaccttgg acagagagga acacagcagc tacactttga 720
cagtagaagc aagagatggc aatggagaag ttacagacaa acctgtaaaa caagctcaag 780
ttcagattcg tattttggat gtcaatgaca atatacctgt agtagaaaat aaagtgcttg 840
aagggatggt tgaagaaaat caagtcaatg tagaagttac gcgcataaaa gtgttcgatg 900
cagatgaaat aggttctgat aattggctgg caaattttac atttgcatca ggaaatgaag 960
gaggttattt ccacatagaa acagatgctc aaactaacga aggaattgtg acccttatta 1020
aggaagtaga ttatgaagaa atgaagaatc ttgacttcag tgttattgtc gctaataaag 1080

cagcttttca caagtcgatt aggagtaaat acaagcctac acccattccc atcaaggtca 1140
aagtgaaaaa tgtgaaagaa ggcattcatt ttaaaagcag cgtcatctca atttatgtta 1200
gcgagagcat ggatagatca agcaaaggcc aaataattgg aaattttcaa gcttttgatg 1260
aggacactgg actaccagcc catgcaagat atgtaaaatt agaagataga gataattgga 1320
tctctgtgga ttctgtcaca tctgaaatta aacttgcaaa acttcctgat tttgaatcta 1380
gatatgttca aaatggcaca tacactgtaa agattgtggc catatcagaa gattatccta 1440
gaaaaaccat cactggcaca gtccttatca atgttgaaga catcaacgac aactgtccca 1500
cactgataga gcctgtgcag acaatctgtc acgatgcaga gtatgtgaat gttactgcag 1560
aggacctgga tggacaccca aacagtggcc ctttcagttt ctccgtcatt gacaaaccac 1620
ctggcatggc agaaaaatgg aaaatagcac gccaagaaag taccagtgtg ctgctgcaac 1680
aaagtgagaa aaagcttggg agaagtgaaa ttcagttcct gatttcagac aatcagggtt 1740
ttagttgtcc tgaaaagcag gtccttacac tcacagtttg tgaggttctg catggcagcg 1800
gctgcaggga agcacagcat gactcctatg tgggcctggg acccgcagca attgcgctca 1860
tgattttggc ctttctgctc ctgctattgg taccactttt actgctgatg tgccattgcg 1920
gaaagggcgc caaagcgttt acccccatac ctggcaccat agagatgctg catccttgga 1980
ataatgaagg agcaccacct gaagacaagg tggtgccatc atttctgcca gtggatcaag 2040
ggggcagtct agtaggaaga aatggagtag gaggtatggc caaggaagcc acgatgaaag 2100
gaagtagctc tgcttccatt gtcaaagggc aacatgagat gtccgagatg gatggaaggt 2160
gggaagaaca cagaagcctg ctttctggta gagctaccca gtttacaggg gccacaggcg 2220
ctatcatgac cactgaaacc acgaagaccg caagggccac aggggcttcc agagacatgg 2280
ccggagctca ggcagctgct gttgcactga acgaagaatt cttaagaaat tatttcactg 2340
ataaagcggc ctcttacact gaggaagatg aaaatcacac agccaaagat tgccttctgg 2400
tttattctca ggaagaaact gaatcgctga atgcttctat tggttgttgc agttttattg 2460
aaggagagct agatgaccgc ttcttagatg atttgggact taaattcaag acgctagctg 2520
aagtttgcct gggtcaaaaa atagatataa ataaggaaat tgagcagaga caaaaacctg 2580
ccacagaaac aagtatgaac acagcttcac attcactctg tgagcaaact atggttaatt 2640
cagagaatac ctactcctct ggcagtagct tcccagttcc aaaatctttg caagaagcca 2700
atgcagagaa agtaactcag gaaatagtca ctgaaagatc tgtgtcttct aggcaggcgc 2760
aaaaggtagc tacacctctt cctgacccaa tggcttctag aaatgtgata gcaacagaaa 2820
cttcctatgt cacagggtcc actatgccac caaccactgt gatcctgggt cctagccagc 2880
cacagagcct tattgtgaca gagagggtgt atgctccagc ttctaccttg gtagatcagc 2940
cttatgctaa tgaaggtaca gttgtggtca ctgaaagagt aatacagcct catgggggtg 3000
gatcgaatcc tctggaaggc actcagcatc ttcaagatgt accttacgtc atggtgaggg 3060
aaagagagag cttccttgcc cccagctcag gtgtgcagcc tactctggcc atgcctaata 3120
tagcagtagg acagaatgtg acagtgacag aaagagttct agcacctgct tccactctgc 3180
aatccagtta ccagattccc actgaaaatt ctatgacggc taggaacacc acggtgtctg 3240
gagctggagt ccctggccct ctgccagatt ttggtttaga ggaatctggt cattctaatt 3300
ctaccataac cacatcttcc accagagtca ccaagcatag cactgtacag cattcttact 3360
cctaaacagc agtcagccac aaactgaccc agagtttaat tagcagtgac taatttcatg 3420
tttccaatgt acctgatttt tcatgagcct tacagacaca cagagacaca tacacattga 3480
tcttaaaatt tttctcagtc actgatatgc aaagga 3516

<210> 10
<211> 5036
<212> DNA
<213> Homo sapiens

<220>
<221> misc_feature
<223> Gene ID: ELF3

<400> 10
   gtgctgccgt gtgggcaagc tctgtgggga gaggggaggc cagtgggttg tttttgccat 60
   cacagaatac tgggaagccc ctggcatcct gctccatagc tctcttcacc actatcctgg 120
   aaccttctcc ccacccccat ccccatgcct ccaaggcact gacctcaaat ccaagtcttt 180
   cccacttatc tcaagctgcc agcctgtagg gattccttat ctcagctcca tgtcagcggt 240
   gaggaagccc caagaaggca agggagctga cagccttctc atttttctcg tacatcctcc 300
   tgttcacccc gccatcccgg gagccccagc cagatgctct tcagggcagg gagcacgtga 360
   gcagccctgg ggctagaagc cggttctccc acattcctgg gtgagggact gggtggaggg 420
   tgtgcctgcc tcaggctcct tgggggaggc ccctgaaggg ctggggaaaa tcctactgag 480
   ccccaggctc tcctgcctgc actggcccag tgcgggggcg ggggggaggg gggatcctgg 540
   acattaactc tctcctaggc acctgggagc ttcagagagg aagaaggtaa ggtaagaagc 600
   agattctctg cgcaggactt tcttagtcct caggctatag agttaaagaa agaagtatct 660
   gtagaaggtg gagtcacctt ttgtttgagt aaccgtttca cccaccctgg ctgagggtgg 720
   ccaggaactc cccaagttgt gcattaatgg aggtgggtca cacagatcct acattttttt 780
   gtttttgttt tttttttttg agacgcagtg attacaggtg tgagccaccg gatcctacat 840
   ttcaaatgca taaaaatcta gatatgggct gggcgcagta gctcatgcct gtaatcccag 900
   cacattggga ggctgaggca ggcagatcat gaggtcagga gatcgagtcc atcctggcta 960
   acatggtgaa accctgtctc tactaaaaat acaaaaagcc gggcatggca gcgggcgcct 1020
   gtaatcccag ctactcggaa agctgaggca ggagaatcgc ttgaacccag gagtcagagg 1080
   ttgcagtgag cagagatcac gccactgcac tccaacctgg gcgacagagc gagactccac 1140
   ctcaaaacaa aataaacaaa atactagatc tggaagagat cttagggatt attaaattca 1200
   gacaacctca ttttttatag atggggaaac aagcacagac tccaagggcc tcatccaaga 1260
   tcacacagtt gcagatgctg gctacaagtc tcctgcctca accacctgta ttaccccatt 1320
   cagggtctca agaagggtct ataagacact atccattgtg cttcgggctg agtccataga 1380
   gacaaccaca gacatggggg actctgccca cagggaaggc aagggctctg gccatggagc 1440
   tggatgggaa gactctgaag cccgaagaca ttgaatcctg tgcagggaaa gagcgagggt 1500
   tttgtgtaca acacacctgc atacctggat gtgaatctca gctccacccc ttcaccaact 1560
   ctgtgtggcc tgggcaagcc attctaaggg aaccctccac actgcaactt tcatgtctat 1620
   aaaatgggaa taaccatgca ttccttacag gacttttttg gtgtgaggat taaatgagag 1680
   aatatgttga aaagtgcttg gtaaatatat taatactatg cattccctct tctttgaatg 1740
   acgtgaccca ggtagtcagg cttctgacca ctagagggca gcagaaggta ctggaaaact 1800
   gggccgagtg aaccagagat tagatggggt ccagagagca gggatgaact tacccgtgtg 1860
   gattctggca actccggcag ggagggctcc agcaggcgct gagggaagaa ctttcaagca 1920
   gagccgggtc tcttcaggag cgactgcagc aaccctgatg cttggatgga gtccaggcag 1980
   gtgatggtag tgaagacctt gccaacagag tgggcgctgg agaaggagcc ctttagtggg 2040
   gaccctgggg ccacgactag gctggcaggc ccagccagca ccaattaatc catgagtatt 2100
   gcccagcatt gagcctggag caccttccag cccctggcca gagtcctggg tgttctggga 2160
   aaaaccccta aacctagtaa ctcctctccc tactaggcct ctttgttgct gaatctctgg 2220
   aatttagggg ccagcagctt tctgactcag gtcagccagg ggttcatgtt ccctcacttg 2280
   ccctccccct gcctggccca tctctggcct ggcccctggg aggaatttcc tgggccagag 2340
   ggcagccgaa agcacagatg cccaccccag caacgttccc gccacctgcc caggccagtg 2400
   ccccgtgccc aaccccagag ggtgcgggat gacagactct gacaatcatt aaaccagccg 2460
   ggcctgattt cccagcactg cctgctaaga tccgggccaa gtggcactga atatgcaaat 2520
   cacctggggc caggagccca gtctaaaggc caggaaatcc cctccatcca atgagacacc 2580
   agctcaggtt actgcagggg acacactata aagccctgag ctcagggagg agctccctcc 2640
   aggctctatt tagagccggg taggggagcg cagcggccag atacctcagc gctacctggc 2700
   ggaactggat ttctctcccg cctgccggcc tgcctgccac agccggactc cgccactccg 2760
   gtaggattcc ccgcctgtca ttccctagcc cagctcttgg gaaactgcag aggggtccag 2820
   aggatttgca gttctgaacc tgcacactcc agtctaggat ctccgagcaa gagcgtaggt 2880
   gtcctgaggg tcaaagaaca gagagagatt gtctctggga aggcagaatg gccatgacgc 2940
   cgctagcctg gctccagggc cccagagatc tgaggaggga agcccagctg gaggctcctg 3000
   tggtcctgcc ctggtctgag atcttggagc ccttcttgaa gagagggtgt ccgcagagtt 3060
   gctgatcttc ctgcccctgg ggctactctt tgcccagggt tgggcaaagc agagtagctg 3120
   ggagtgtaag gagaggaccc tcgtcccctc accaacctca tcctctctcc ccctacccac 3180
   gggtagcctc atggctgcaa cctgtgagat tagcaacatt tttagcaact acttcagtgc 3240
   gatgtacagc tcggaggact ccaccctggc ctctgttccc cctgctgcca cctttggggc 3300
   cgatgacttg gtactgaccc tgagcaaccc ccagatgtca ttggagggta cagagaaggc 3360
   cagctggttg ggggaacagc cccagttctg gtcgaagacg caggttctgg actggatcag 3420
   ctaccaagtg gagaagaaca agtacgacgc aagcgccatt gacttctcac gatgtgacat 3480
   ggatggcgcc accctctgca attgtgccct tgaggagctg cgtctggtct ttgggcctct 3540
   gggggaccaa ctccatgccc agctgcgaga cctcacttcc agctcttctg atgagctcag 3600
   ttggatcatt gagctgctgg agaaggatgg catggccttc caggaggccc tagacccagg 3660
   gccctttgac cagggcagcc cctttgccca ggagctgctg gacgacggtc agcaagccag 3720
   cccctaccac cccggcagct gtggcgcagg agccccctcc cccggcagct ctgacgtctc 3780
   caccgcaggg actggtgctt ctcggagctc ccactcctca gactccggtg gaagtgacgt 3840
   ggacctggat cccactgatg gcaagctctt ccccagcgat ggttttcgtg actgcaagaa 3900
   gggggatccc aagcacgggg agcggaaacg aggccggccc cgaaagctga gcaaagagta 3960
   ctgggactgt ctcgagggca agaagagcaa gcacgcgccc agaggcaccc acctgtggga 4020
   gttcatccgg gacatcctca tccacccgga gctcaacgag ggcctcatga agtgggagaa 4080
   tcggcatgaa ggcgtcttca agttcctgcg ctccgaggct gtggcccaac tatggggcca 4140
   aaagaaaaag aacagcaaca tgacctacga gaagctgagc cgggccatga ggtactacta 4200
   caaacgggag atcctggaac gggtggatgg ccggcgactc gtctacaagt ttggcaaaaa 4260
   ctcaagcggc tggaaggagg aagaggttct ccagagtcgg aactgagggt tggaactata 4320
   cccgggacca aactcacgga ccactcgagg cctgcaaacc ttcctgggag gacgggcagg 4380
   ccagatggcc cctccactgg ggaatgctcc cagctgtgct gtggagagaa gctgatgttt 4440
   tggtgtattg tcagccatcg tcctgggact cggagactat ggcctcgcct ccccaccctc 4500
   ctcttggaat tacaagccct ggggtttgaa gctgacttta tagctgcaag tgtatctcct 4560
   tttatctggt gcctcctcaa acccagtctc agacactaaa tgcagacaac accttcctcc 4620
   tgcagacacc tggactgagc caaggaggcc tggggaggcc ctaggggagc accgtgatgg 4680
   agaggacaga gcaggggctc cagcaccttc tttctggact ggcgttcacc tccctgctca 4740
   gtgcttgggc tccacgggca ggggtcagag cactccctaa tttatgtgct atataaatac 4800
   gtcagatgta catagagatc tattttttct aaaacattcc cctccccact cctctcccac 4860
   agagtgctgg actgttccag gccctccagt gggctgatgc tgggaccctt aggatggggc 4920
   tcccagctcc tttctcctgt gaatggaggc agagacctcc aataaagtgc cttctgggct 4980
   ttttctaaaa aaaaaaaaaa aaaaaaaaaa aaagaaaaaa aaaaaaaaaa aaaaca 5036

<210> 11
<211> 5511
<212> DNA
<213> Homo sapiens

<220>
<221> misc_feature
<223> Gene ID: ERBB3

<400> 11
acacacacac acccctcccc tgccatccct ccccggactc cggctccggc tccgattgca 60
atttgcaacc tccgctgccg tcgccgcagc agccaccaat tcgccagcgg ttcaggtggc 120
tcttgcctcg atgtcctagc ctaggggccc ccgggccgga cttggctggg ctcccttcac 180
cctctgcgga gtcatgaggg cgaacgacgc tctgcaggtg ctgggcttgc ttttcagcct 240
ggcccggggc tccgaggtgg gcaactctca ggcagtgtgt cctgggactc tgaatggcct 300
gagtgtgacc ggcgatgctg agaaccaata ccagacactg tacaagctct acgagaggtg 360
tgaggtggtg atggggaacc ttgagattgt gctcacggga cacaatgccg acctctcctt 420
cctgcagtgg attcgagaag tgacaggcta tgtcctcgtg gccatgaatg aattctctac 480
tctaccattg cccaacctcc gcgtggtgcg agggacccag gtctacgatg ggaagtttgc 540
catcttcgtc atgttgaact ataacaccaa ctccagccac gctctgcgcc agctccgctt 600
gactcagctc accgagattc tgtcaggggg tgtttatatt gagaagaacg ataagctttg 660
tcacatggac acaattgact ggagggacat cgtgagggac cgagatgctg agatagtggt 720
gaaggacaat ggcagaagct gtcccccctg tcatgaggtt tgcaaggggc gatgctgggg 780
tcctggatca gaagactgcc agacattgac caagaccatc tgtgctcctc agtgtaatgg 840
tcactgcttt gggcccaacc ccaaccagtg ctgccatgat gagtgtgccg ggggctgctc 900
aggccctcag gacacagact gctttgcctg ccggcacttc aatgacagtg gagcctgtgt 960
acctcgctgt ccacagcctc ttgtctacaa caagctaact ttccagctgg aacccaatcc 1020

ccacaccaag tatcagtatg gaggagtttg tgtagccagc tgtccccata actttgtggt 1080
ggatcaaaca tcctgtgtca gggcctgtcc tcctgacaag atggaagtag ataaaaatgg 1140
gctcaagatg tgtgagcctt gtgggggact atgtcccaaa gcctgtgagg gaacaggctc 1200
tgggagccgc ttccagactg tggactcgag caacattgat ggatttgtga actgcaccaa 1260
gatcctgggc aacctggact ttctgatcac cggcctcaat ggagacccct ggcacaagat 1320
ccctgccctg gacccagaga agctcaatgt cttccggaca gtacgggaga tcacaggtta 1380
cctgaacatc cagtcctggc cgccccacat gcacaacttc agtgtttttt ccaatttgac 1440
aaccattgga ggcagaagcc tctacaaccg gggcttctca ttgttgatca tgaagaactt 1500
gaatgtcaca tctctgggct tccgatccct gaaggaaatt agtgctgggc gtatctatat 1560
aagtgccaat aggcagctct gctaccacca ctctttgaac tggaccaagg tgcttcgggg 1620
gcctacggaa gagcgactag acatcaagca taatcggccg cgcagagact gcgtggcaga 1680
gggcaaagtg tgtgacccac tgtgctcctc tgggggatgc tggggcccag gccctggtca 1740
gtgcttgtcc tgtcgaaatt atagccgagg aggtgtctgt gtgacccact gcaactttct 1800
gaatggggag cctcgagaat ttgcccatga ggccgaatgc ttctcctgcc acccggaatg 1860
ccaacccatg gagggcactg ccacatgcaa tggctcgggc tctgatactt gtgctcaatg 1920
tgcccatttt cgagatgggc cccactgtgt gagcagctgc ccccatggag tcctaggtgc 1980
caagggccca atctacaagt acccagatgt tcagaatgaa tgtcggccct gccatgagaa 2040
ctgcacccag gggtgtaaag gaccagagct tcaagactgt ttaggacaaa cactggtgct 2100
gatcggcaaa acccatctga caatggcttt gacagtgata gcaggattgg tagtgatttt 2160
catgatgctg ggcggcactt ttctctactg gcgtgggcgc cggattcaga ataaaagggc 2220
tatgaggcga tacttggaac ggggtgagag catagagcct ctggacccca gtgagaaggc 2280
taacaaagtc ttggccagaa tcttcaaaga gacagagcta aggaagctta aagtgcttgg 2340
ctcgggtgtc tttggaactg tgcacaaagg agtgtggatc cctgagggtg aatcaatcaa 2400
gattccagtc tgcattaaag tcattgagga caagagtgga cggcagagtt ttcaagctgt 2460
gacagatcat atgctggcca ttggcagcct ggaccatgcc cacattgtaa ggctgctggg 2520
actatgccca gggtcatctc tgcagcttgt cactcaatat ttgcctctgg gttctctgct 2580
ggatcatgtg agacaacacc ggggggcact ggggccacag ctgctgctca actggggagt 2640
acaaattgcc aagggaatgt actaccttga ggaacatggt atggtgcata gaaacctggc 2700
tgcccgaaac gtgctactca agtcacccag tcaggttcag gtggcagatt ttggtgtggc 2760
tgacctgctg cctcctgatg ataagcagct gctatacagt gaggccaaga ctccaattaa 2820
gtggatggcc cttgagagta tccactttgg gaaatacaca caccagagtg atgtctggag 2880
ctatggtgtg acagtttggg agttgatgac cttcggggca gagccctatg cagggctacg 2940
attggctgaa gtaccagacc tgctagagaa gggggagcgg ttggcacagc cccagatctg 3000
cacaattgat gtctacatgg tgatggtcaa gtgttggatg attgatgaga acattcgccc 3060
aacctttaaa gaactagcca atgagttcac caggatggcc cgagacccac cacggtatct 3120
ggtcataaag agagagagtg ggcctggaat agcccctggg ccagagcccc atggtctgac 3180
aaacaagaag ctagaggaag tagagctgga gccagaacta gacctagacc tagacttgga 3240
agcagaggag gacaacctgg caaccaccac actgggctcc gccctcagcc taccagttgg 3300
aacacttaat cggccacgtg ggagccagag ccttttaagt ccatcatctg gatacatgcc 3360
catgaaccag ggtaatcttg gggagtcttg ccaggagtct gcagtttctg ggagcagtga 3420
acggtgcccc cgtccagtct ctctacaccc aatgccacgg ggatgcctgg catcagagtc 3480

atcagagggg catgtaacag gctctgaggc tgagctccag gagaaagtgt caatgtgtag 3540
gagccggagc aggagccgga gcccacggcc acgcggagat agcgcctacc attcccagcg 3600
ccacagtctg ctgactcctg ttaccccact ctccccaccc gggttagagg aagaggatgt 3660
caacggttat gtcatgccag atacacacct caaaggtact ccctcctccc gggaaggcac 3720
cctttcttca gtgggtctca gttctgtcct gggtactgaa gaagaagatg aagatgagga 3780
gtatgaatac atgaaccgga ggagaaggca cagtccacct catcccccta ggccaagttc 3840
ccttgaggag ctgggttatg agtacatgga tgtggggtca gacctcagtg cctctctggg 3900
cagcacacag agttgcccac tccaccctgt acccatcatg cccactgcag gcacaactcc 3960
agatgaagac tatgaatata tgaatcggca acgagatgga ggtggtcctg ggggtgatta 4020
tgcagccatg ggggcctgcc cagcatctga gcaagggtat gaagagatga gagcttttca 4080
ggggcctgga catcaggccc cccatgtcca ttatgcccgc ctaaaaactc tacgtagctt 4140
agaggctaca gactctgcct ttgataaccc tgattactgg catagcaggc ttttccccaa 4200
ggctaatgcc cagagaacgt aactcctgct ccctgtggca ctcagggagc atttaatggc 4260
agctagtgcc tttagagggt accgtcttct ccctattccc tctctctccc aggtcccagc 4320
cccttttccc cagtcccaga caattccatt caatctttgg aggcttttaa acattttgac 4380
acaaaattct tatggtatgt agccagctgt gcactttctt ctctttccca accccaggaa 4440
aggttttcct tattttgtgt gctttcccag tcccattcct cagcttcttc acaggcactc 4500
ctggagatat gaaggattac tctccatatc ccttcctctc aggctcttga ctacttggaa 4560
ctaggctctt atgtgtgcct ttgtttccca tcagactgtc aagaagagga aagggaggaa 4620
acctagcaga ggaaagtgta attttggttt atgactctta accccctaga aagacagaag 4680
cttaaaatct gtgaagaaag aggttaggag tagatattga ttactatcat aattcagcac 4740
ttaactatga gccaggcatc atactaaact tcacctacat tatctcactt agtcctttat 4800
catccttaaa acaattctgt gacatacata ttatctcatt ttacacaaag ggaagtcggg 4860
catggtggct catgcctgta atctcagcac tttgggaggc tgaggcagaa ggattacctg 4920
aggcaaggag tttgagacca gcttagccaa catagtaaga cccccatctc tttaaaaaaa 4980
aaaaaaaaaa aaaaaaaaaa actttagaac tgggtgcagt ggctcatgcc tgtaatccca 5040
gccagcactt tgggaggctg agatgggaag atcacttgag cccagaatta gagataagcc 5100
tatggaaaca tagcaagaca ctgtctctac aggggaaaaa aaaaaaagaa actgagcctt 5160
aaagagatga aataaattaa gcagtagatc caggatgcaa aatcctccca attcctgtgc 5220
atgtgctctt attgtaaggt gccaagaaaa actgatttaa gttacagccc ttgtttaagg 5280
ggcactgttt cttgtttttg cactgaatca agtctaaccc caacagccac atcctcctat 5340
acctagacat ctcatctcag gaagtggtgg tgggggtagt cagaaggaaa aataactgga 5400
catctttgtg taaaccataa tccacatgtg ccgtaaatga tcttcactcc ttatccgagg 5460
gcaaattcac aaggatcccc aagatccact tttagaagcc attctcatcc a 5511

<210> 12
<211> 1654
<212> DNA
<213> Homo sapiens

<220>

<221>  misc_feature
<223>  Gene ID:  KCTD14

<400>  12
ggctgcgcag tggagcggcc agtgggcagg atgacgagcc agacccctct gccccagtcc      60
ccccggccca ggcggccgac gatgtctact gttgtggagc tgaacgtcgg gggtgagttc     120
cacaccacca ccctgggtac cctgaggaag tttccgggct caaagctggc agagatgttc     180
tctagcttag ccaaggcctc cacggacgcg gagggccgct tcttcatcga ccgccccagc     240
acctatttca gacccatcct ggactacctg cgcactgggc aagtgcccac acagcacatc     300
cctgaagtgt accgtgaggc tcagttctac gaaatcaagc ctttggtcaa gctgctggag     360
gacatgccac agatctttgg tgagcaggtg tctcggaagc agtttttgct gcaagtgccg     420
ggctacagcg agaacctgga gctcatggtg cgcctggcac gtgcagaagc cataacagca     480
cggaagtcca gcgtgcttgt gtgcctggtg gaaactgagg agcaggatgc atattattca     540
gaggtcctgt gttttctgca ggataagaag atgttcaagt ctgttgtcaa gtttgggccc     600
tggaaggcgg tcctagacaa cagcgacctc atgcactgcc tggagatgga cattaaggcc     660
caggggtaca aggtattctc caagttctac ctgacgtacc ccaccaaaag aaacgaattc     720
cattttaaca tttattcatt caccttcacc tggtggtgat cctcaggagc agagactgtt     780
atgaattctg gcgtggctta tgaaattaaa agttgccatc aaagccattt tcttttaatt     840
tcacaaacat caggcaattt ccagggttgg tctagagtct tgccactaaa tattgatcac     900
tcgtttaagg actttccact ccattgcaac tgatgccact atatttgcct agcaacttgc     960
agctacttcc ttttcaaagc ctcatgtatc tcccagaccc ttctcttgaa gtccaataac    1020
aagaccaagt aagaatgttt caacaatgcg ttggcaagag atgtgagatg acaacaggaa    1080
catacaagat actgtgaatc tagatgttct gacccaaaga tgtagtctac atagccccag    1140
cttggggtcc aatccatctg tccctggcat gtgccttcat gtagtaggtg ctttcctgat    1200
cccctttgcg agatgctgtg ggtgctaaca cctcagagct gtcctcttct ctagagtgaa    1260
ggttttcaga gtgcatcatc agcattacct gtgaacttgc tggaaataca aatcctcagg    1320
ccccacctca gacctactga atcagaatct ctgggggttg gcacagcatt ctgatttacc    1380
aaaccctcca agtgattttg atgtattcta attttgagac catctctaga aaagaattgc    1440
tacctcttgt atggaggtac aaaagactga cctcttacat caaggaactt cctttcccag    1500
agctcctcat ggaatcaagc tgaagtcagt cttcttctga gagcacattc ttactcagtt    1560
tttttcctct gtcctacgct gcttccctca ctccccttct cctaagagca ctccatcaat    1620
aaaccacttg cacgagaaaa aaaaaaaaaa aaaa                                1654

<210>  13
<211>  3350
<212>  DNA
<213>  Homo sapiens

<220>
<221>  misc_feature
<223>  Gene ID: KLF5

<400> 13
tagtcgcggg gcaggtacgt gcgctcgcgg ttctctcgcg gaggtcggcg gtggcgggag 60
cgggctccgg agagcctgag agcacggtgg ggcggggcgg gagaaagtgg ccgcccggag 120
gacgttggcg tttacgtgtg gaagagcgga agagttttgc ttttcgtgcg cgccttcgaa 180
aactgcctgc cgctgtctga ggagtccacc cgaaacctcc cctcctccgc cggcagcccc 240
gcgctgagct cgccgaccca agccagcgtg ggcgaggtgg gaagtgcgcc cgacccgcgc 300
ctggagctgc gcccccgagt gcccatggct acaagggtgc tgagcatgag cgcccgcctg 360
ggacccgtgc cccagccgcc ggcgccgcag gacgagccgg tgttcgcgca gctcaagccg 420
gtgctgggcg ccgcgaatcc ggcccgcgac gcggcgctct tccccggcga ggagctgaag 480
cacgcgcacc accgcccgca ggcgcagccc gcgcccgcgc aggccccgca gccggcccag 540
ccgcccgcca ccggcccgcg gctgcctcca gaggacctgg tccagacaag atgtgaaatg 600
gagaagtatc tgacacctca gcttcctcca gttcctataa ttccagagca taaaaagtat 660
agacgagaca gtgcctcagt cgtagaccag ttcttcactg acactgaagg gttaccttac 720
agtatcaaca tgaacgtctt cctccctgac atcactcacc tgagaactgg cctctacaaa 780
tcccagagac cgtgcgtaac acacatcaag acagaacctg ttgccatttt cagccaccag 840
agtgaaacga ctgcccctcc tccggccccg acccaggccc tccctgagtt caccagtata 900
ttcagctcac accagaccgc agctccagag gtgaacaata ttttcatcaa acaagaactt 960
cctacaccag atcttcatct ttctgtccct acccagcagg gccacctgta ccagctactg 1020
aatacaccgg atctagatat gcccagttct acaaatcaga cagcagcaat ggacactctt 1080
aatgtttcta tgtcagctgc catggcaggc cttaacacac acacctctgc tgttccgcag 1140
actgcagtga aacaattcca gggcatgccc ccttgcacat acacaatgcc aagtcagttt 1200
cttccacaac aggccactta ctttcccccg tcaccaccaa gctcagagcc tggaagtcca 1260
gatagacaag cagagatgct ccagaattta accccacctc catcctatgc tgctacaatt 1320
gcttctaaac tggcaattca caatccaaat ttacccacca ccctgccagt taactcacaa 1380
aacatccaac ctgtcagata caatagaagg agtaaccccg atttggagaa acgacgcatc 1440
cactactgcg attaccctgg ttgcacaaaa gtttatacca agtcttctca tttaaaagct 1500
cacctgagga ctcacactgg tgaaaagcca tacaagtgta cctgggaagg ctgcgactgg 1560
aggttcgcgc gatcggatga gctgacccgc cactaccgga agcacacagg cgccaagccc 1620
ttccagtgcg gggtgtgcaa ccgcagcttc tcgcgctctg accacctggc cctgcatatg 1680
aagaggcacc agaactgagc actgcccgtg tgacccgttc caggtcccct gggctccctc 1740
aaatgacaga cctaactatt cctgtgtaaa aacaacaaaa acaaacaaaa gcaagaaaac 1800
cacaactaaa actggaaatg tatattttgt atatttgaga aaacagggaa tacattgtat 1860
taataccaaa gtgtttggtc attttaagaa tctggaatgc ttgctgtaat gtatatggct 1920
ttactcaagc agatctcatc tcatgacagg cagccacgtc tcaacatggg taaggggtgg 1980
gggtggaggg gagtgtgtgc agcgttttta cctaggcacc atcatttaat gtgacagtgt 2040
tcagtaaaca aatcagttgg caggcaccag aagaagaatg gattgtatgt caagatttta 2100
cttggcattg agtagttttt ttcaatagta ggtaattcct tagagataca gtatacctgg 2160
caattcacaa atagccattg aacaaatgtg tgggttttta aaaattatat acatatatga 2220
gttgcctata tttgctattc aaaattttgt aaatatgcaa atcagcttta taggtttatt 2280
acaagttttt taggattctt ttggggaaga gtcataattc ttttgaaaat aaccatgaat 2340

acacttacag ttaggatttg tggtaaggta cctctcaaca ttaccaaaat catttcttta 2400
gagggaagga ataatcattc aaatgaactt taaaaaagca aatttcatgc actgattaaa 2460
ataggattat tttaaataca aaaggcattt tatatgaatt ataaactgaa gagcttaaag 2520
atagttacaa aatacaaaag ttcaacctct tacaataagc taaacgcaat gtcattttta 2580
aaaagaagga cttagggtgt cgttttcaca tatgacaatg ttgcatttat gatgcagttt 2640
caagtaccaa aacgttgaat tgatgatgca gttttcatat atcgagatgt tcgctcgtgc 2700
agtactgttg gttaaatgac aatttatgtg gattttgcat gtaatacaca gtgagacaca 2760
gtaattttat ctaaattaca gtgcagttta gttaatctat taatactgac tcagtgtctg 2820
cctttaaata taaatgatat gttgaaaact taaggaagca aatgctacat atatgcaata 2880
taaaatagta atgtgatgct gatgctgtta accaaagggc agaataaata agcaaaatgc 2940
caaaaggggt cttaattgaa atgaaaattt aattttgttt ttaaaatatt gtttatcttt 3000
atttattttg tggtaatata gtaagttttt ttagaagaca attttcataa cttgataaat 3060
tatagttttg tttgttagaa aagttgctct taaaagatgt aaatagatga caaacgatgt 3120
aaataatttt gtaagaggct tcaaaatgtt tatacgtgga aacacaccta catgaaaagc 3180
agaaatcggt tgctgttttg cttctttttc cctcttattt ttgtattgtg gtcatttcct 3240
atgcaaataa tggagcaaac agctgtatag ttgtagaatt ttttgagaga atgagatgtt 3300
tatatattaa cgacaatttt ttttttggaa aataaaaagt gcctaaaaga 3350

<210> 14
<211> 1788
<212> DNA
<213> Homo sapiens

<220>
<221> misc_feature
<223> Gene ID: KRT8

<400> 14
attcctgaga gctctcctca ccaagaagca gcttctccgc tccttctagg atctccgcct 60
ggttcggccc gcctgcctcc actcctgcct ctaccatgtc catcagggtg acccagaagt 120
cctacaaggt gtccacctct ggcccccggg ccttcagcag ccgctcctac acgagtgggc 180
ccggttcccg catcagctcc tcgagcttct cccgagtggg cagcagcaac tttcgcggtg 240
gcctgggcgg cggctatggt ggggccagcg gcatgggagg catcaccgca gttacggtca 300
accagagcct gctgagcccc cttgtcctgg aggtggaccc caacatccag gccgtgcgca 360
cccaggagaa ggagcagatc aagaccctca acaacaagtt tgcctccttc atagacaagg 420
tacggttcct ggagcagcag aacaagatgc tggagaccaa gtggagcctc ctgcagcagc 480
agaagacggc tcgaagcaac atggacaaca tgttcgagag ctacatcaac aaccttaggc 540
ggcagctgga gactctgggc caggagaagc tgaagctgga ggcggagctt ggcaacatgc 600
aggggctggt ggaggacttc aagaacaagt atgaggatga gatcaataag cgtacagaga 660
tggagaacga atttgtcctc atcaagaagg atgtggatga agcttacatg aacaaggtag 720
agctggagtc tcgcctggaa gggctgaccg acgagatcaa cttcctcagg cagctatatg 780

aagaggagat ccgggagctg cagtcccaga tctcggacac atctgtggtg ctgtccatgg 840
acaacagccg ctccctggac atggacagca tcattgctga ggtcaaggca cagtacgagg 900
atattgccaa ccgcagccgg gctgaggctg agagcatgta ccagatcaag tatgaggagc 960
tgcagagcct ggctgggaag cacggggatg acctgcggcg cacaaagact gagatctctg 1020
agatgaaccg gaacatcagc cggctccagg ctgagattga gggcctcaaa ggccagaggg 1080
cttccctgga ggccgccatt gcagatgccg agcagcgtgg agagctggcc attaaggatg 1140
ccaacgccaa gttgtccgag ctggaggccg ccctgcagcg ggccaagcag gacatggcgc 1200
ggcagctgcg tgagtaccag gagctgatga acgtcaagct ggccctggac atcgagatcg 1260
ccacctacag gaagctgctg gagggcgagg agagccggct ggagtctggg atgcagaaca 1320
tgagtattca tacgaagacc accagcggct atgcaggtgg tctgagctcg gcctatgggg 1380
gcctcacaag ccccggcctc agctacagcc tgggctccag ctttggctct ggcgcgggct 1440
ccagctcctt cagccgcacc agctcctcca gggccgtggt tgtgaagaag atcgagacac 1500
gtgatgggaa gctggtgtct gagtcctctg acgtcctgcc caagtgaaca gctgcggcag 1560
cccctcccag cctacccctc ctgcgctgcc ccagagcctg ggaaggaggc cgctatgcag 1620
ggtagcactg ggaacaggag acccacctga ggctcagccc tagccctcag cccacctggg 1680
gagtttacta cctggggacc ccccttgccc atgcctccag ctacaaaaca attcaattgc 1740
tttttttttt tggtccaaaa taaaacctca gctagctctg ccaatgtc 1788

<210> 15
<211> 4018
<212> DNA
<213> Homo sapiens

<220>
<221> misc_feature
<223> Gene ID: MAP7

<400> 15
   gttccgcccg ccgctcttcc agctgagact cggcagcggc ggtcggagcg gctgccacgc 60
   cgcacctgcc cgcgaggggg cgcgccaggc ccaggccgca gccgactgcc gcgctctctg 120
   tccccgccct ccgccatccc cggactgcgc tcacctgtct gggccgctgg cctgggaggc 180
   gggggccggc gggagccaag ccgaggaaag ggcggagcgg ctctccgggc gcgtcatcgg 240
   agcaccatgg cggagctagg agctggcggc gacggccaca ggggcggcga cggcgcagtg 300
   cgaagcgaaa cagcacccga cagctacaaa gtgcaagata agaaaaatgc ctccagccgc 360
   cctgcctctg caatttcagg acaaaataac aaccactcag gaaataaacc agaccctccg 420
   cctgtgttac gtgttgatga ccggcagcgg ctggcccggg agcgacgtga ggaacgggag 480
   aaacagctag ctgcaagaga aatagtgtgg ttagaaagag aagagcgagc caggcagcac 540
   tacgagaagc acctggaaga gcggaagaag aggttggagg agcagaggca gaaggaggag 600
   cggaggaggg ctgctgtgga ggagaagcgg aggcagagac ttgaggagga caaagaacgc 660
   cacgaagctg ttgtacggcg cacaatggaa aggagccaga agccaaaaca gaagcataac 720
   cgttggtcgt ggggaggctc tctccatggg agccctagca tccacagtgc agatccagac 780
   aggcggtcag tttccaccat gaatctttcg aaatatgttg atcccgtcat tagcaagcgg 840
   ctctcctctt catctgcaac tttactaaat tctccagata gagctcgccg cctgcagctc 900
   agcccatggg agagcagcgt tgttaacaga ctcctgacgc ccacacattc gttcctggcc 960
   agaagtaaaa gcacagctgc cttgtctgga gaagcagcat cttgcagccc catcatcatg 1020
   ccctacaaag ctgcacactc tagaaattcg atggatcgac caaaactctt tgtaacacca 1080
   cctgagggct cttctcgcag gaggatcatt catggcacag cgagctataa aaaagaaaga 1140
   gagagagaaa atgtactctt cctcacatct ggcacccgaa gggctgtatc tccatctaat 1200
   cccaaagcaa gacaaccagc tcgctcccga ctttggcttc cgtccaagtc tcttcctcat 1260
   ttgcctggca cacccagacc gacatcctcc ttgccacccg gctcagtcaa agctgctcct 1320
   gctcaggtcc ggcccccatc ccccggcaac atccgccctg tcaagaggga agtcaaagtg 1380
   gagcctgaga agaaagatcc tgagaaggaa cctcagaaag ttgccaatga gccctcacta 1440
   aagggcagag cacctttagt gaaggtagaa gaagccacag ttgaagagcg gacacctgct 1500
   gaaccagaag ttggccctgc tgctccagcc atggccccag ctccagcctc ggccccagct 1560
   ccagcctcgg ccccagctcc agccccggtc cccaccccag ccatggtctc agccccgtca 1620
   tccactgtga atgccagtgc ttctgttaag acttctgcag gcaccaccga cccagaggag 1680
   gccacaaggc ttctagctga gaagaggcgg ctggcccgag agcagagaga aaaggaagaa 1740
   agggagagga gggagcagga agagcttgaa agacaaaaga gagaggaatt ggctcaacgt 1800
   gtggctgaag agaggacgac tcgccgtgag gaggagtcgc gcaggctgga agccgagcag 1860
   gcccgggaga aggaggagca gctgcagcgg caggcggagg agcgggcgct gcgcgagcgg 1920
   gaggaggcag agcgcgccca gaggcagaaa gaagaagaag ctcgcgttcg tgaagaagca 1980
   gagagggtcc ggcaggaacg agagaagcat ttccagagag aagagcaaga gcgcctggag 2040
   agaaagaagc gacttgagga gattatgaaa agaaccagga gaacagaagc tacagataag 2100
   aaaaccagtg atcagagaaa cggtgatata gccaagggag ctctcactgg aggaacagag 2160
   gtgtctgcac ttccatgtac aacaaacgct ccgggaaatg gaaagccagt tggcagccca 2220
   catgtggtta cctcacacca gtcaaaagtg acagtggaga gcactcccga tttggaaaaa 2280
   caaccaaatg aaaatggtgt atctgttcag aatgaaaatt ttgaagaaat tataaactta 2340
   cccattggat ctaaaccatc cagattagat gtcaccaaca gtgagagccc agaaattcct 2400
   ttgaatccaa ttttggcctt tgatgatgaa gggacacttg ggcccctgcc tcaggtagat 2460
   ggtgttcaga cacagcagac tgcagaagtt atatgagtgt ttcttctgaa gaaccaaagc 2520
   tgaaatttaa tgagaatttc tacaattaat ggaattcctt tcctgctata aaggagcatc 2580
   ccctccaccc gttttctaga gttcttgacc atcattttga aaagatttat taaaactagc 2640
   taaagacaac agactggata gcttttctaa taattttcat caataggaaa aaagaaatac 2700
   gtctcattct tcaatacttt aaaatggctt tttccagtgt gctccttctt agcaatcaat 2760
   atttttctgc attctttaaa agacaagaga atttggttat aaaagaaatg ggctgactag 2820
   gcatgatttt tttggtctta aaagcttaac atgtaaaatt ggcaaaaaaa attttttacc 2880
   ttttataata cttgaaaaat aagtacctct ttgttctaca agtagaatga ataggagaag 2940
   agtttaagcc tgttttttta aaatattatt gcaaagagct ctatttgtag aagcaaatta 3000
   taggcagatt accaggttct tataaataca gcttgtacat ggacattctg caaacccagc 3060
   tgtcacattt ttcttgcaac tccttttgca aaagcagact aaaatgtttt aaaatgtgaa 3120
   aaaacattat tttttcaaag caagaaaata atttactgcc ctcttacata atgtatttat 3180
   aaagtttttc cagataaact aatcaaataa attagaataa tgtgacaaca ttacaaattt 3240
   aatttgttag ctgcattcct tctgatgtta ccacgataga atgttactga tgattcaggg 3300
   ctatttctga agtctgtatg ttgctgctgt ccccagtgat ggtggactta tctttgcctt 3360
   acctgatcac aaattatgtt ggggaaaata aagatttaat atttctttaa atagaaaaag 3420
   aatttggttt tgctcgttta agagcaatga gaaaatgatg gaatgttgac tgtgtttggc 3480
   acacaggaca cggaccttca tggaagtcct tgctctgcgt ggcatctgtc agcttttcac 3540
   ctttcattct tattcttcac ttttgctgct gagcctagct gtacaaactt gcactttcat 3600
   ttgctaatat aaattcagtt ttattttacc attttagaga ctactaatga ttaaatgtag 3660
   aaggagaggg tgcacatgtt tttatgtgga gtgtttaaaa gataaattta taccactgta 3720
   atgtgcagct tttattaaaa gagaaattgg ttgaactgct aggttgaatg agagacttca 3780
   tctattggac tatttttttt aatccaggca tatggtcttt agtaatggct tgtaatttgt 3840
   gaaaacatta atttgggggt tttccctgtt ttcagttgtc catgtacaca tagtcattat 3900
   attagaaaag aaagctgttc aacaaacttg tttaatttgt ttaaatcaac atagcatgaa 3960
   acaccaaata aaatgtttga catagtttta aaaaaaaaaa aaaaaaaaaa aaaaaaaa 4018

<210> 16
<211> 2601
<212> DNA
<213> Homo sapiens

<220>
<221> misc_feature
<223> Gene ID: NQO1

<400> 16
ccgcccttgt aggctgtcca cctcaaacgg gccggacagg atatataaga gagaatgcac 60
cgtgcactac acacgcgact cccacaaggt tgcagccgga gccgcccagc tcaccgagag 120
cctagttccg gccagggtcg ccccggcaac cacgagccca gccaatcagc gccccggact 180
gcaccagagc catggtcggc agaagagcac tgatcgtact ggctcactca gagaggacgt 240
ccttcaacta tgccatgaag gaggctgctg cagcggcttt gaagaagaaa ggatgggagg 300
tggtggagtc ggacctctat gccatgaact tcaatcccat catttccaga aaggacatca 360
caggtaaact gaaggaccct gcgaactttc agtatcctgc cgagtctgtt ctggcttata 420
aagaaggcca tctgagccca gatattgtgg ctgaacaaaa gaagctggaa gccgcagacc 480
ttgtgatatt ccagttcccc ctgcagtggt ttggagtccc tgccattctg aaaggctggt 540
ttgagcgagt gttcatagga gagtttgctt acacttacgc tgccatgtat gacaaaggac 600
ccttccggag taagaaggca gtgctttcca tcaccactgg tggcagtggc tccatgtact 660
ctctgcaagg gatccacggg gacatgaatg tcattctctg gccaattcag agtggcattc 720
tgcatttctg tggcttccaa gtcttagaac ctcaactgac atatagcatt gggcacactc 780
cagcagacgc ccgaattcaa atcctggaag gatggaagaa acgcctggag aatatttggg 840
atgagacacc actgtatttt gctccaagca gcctctttga cctaaacttc caggcaggat 900
tcttaatgaa aaaagaggta caggatgagg agaaaaacaa gaaatttggc ctttctgtgg 960
gccatcactt gggcaagtcc atcccaactg acaaccagat caaagctaga aaatgagatt 1020

ccttagcctg gatttccttc taacatgtta tcaaatctgg gtatctttcc aggcttccct 1080
gacttgcttt agtttttaag atttgtgttt ttctttttcc acaaggaata aatgagaggg 1140
aatcgactgt attcgtgcat ttttggatca tttttaactg attcttatga ttactatcat 1200
ggcatataac caaaatccga ctgggctcaa gaggccactt agggaaagat gtagaaagat 1260
gctagaaaaa tgttctttaa aggcatctac acaatttaat tcctcttttt agggctaaag 1320
ttttagggta cagtttggct aggtatcatt caactctcca atgttctatt aatcacctct 1380
ctgtagttta tggcagaagg gaattgctca gagaaggaaa agactgaatc tacctgccct 1440
aagggactta acttgtttgg tagttagcca tctaatgctt gtttatgata tttcttgctt 1500
tcaattacaa agcagttact aatatgccta gcacaagtac cactcttggt cagcttttgt 1560
tgtttatata cagtacacag ataccttgaa aggaagagct aataaatctc ttctttgctg 1620
cagtcatcta cttttttttt aattaaaaaa aatttttttt tgaagcagtc ttgctctgtt 1680
acccaggctg gagtgcagtg gtgtgatctc ggctcactgc aacctctgcc tcccaggttc 1740
cagcaattct cctgcctcag cctccctagt agctgggatg acaggcgcct gccatcatgc 1800
ctgactaatt tttgtatttt tagtagagac ggcgtttcac catgttggcc aggctggtct 1860
caaactcctg acctcaggtg atccgcctac ctcagcctcc caaagtgctg ggattacagg 1920
cgtgatccac cacacctggc ccttgcaatc ttctacttta aggtttgcag agataaacca 1980
ataaatccac accgtacatc tgcaatatga attcaagaaa ggaaatagta ccttcaatac 2040
ttaaaaatag tcttccacaa aaaatacttt atttctgatc tatacaaatt ttcagaaggt 2100
tattttcttt atcattgcta aactgatgac ttactatggg atggggtcca gtcccatgac 2160
cttggggtac aattgtaaac ctagagtttt atcaactttg gtgaacagtt ttggcataat 2220
agtcaatttc tacttctgga agtcatctca ttccactgtt ggtattatat aattcaagga 2280
gaatatgata aaacactgcc ctcttgtggt gcattgaaag aagagatgag aaatgatgaa 2340
aaggttgcct gaaaaatggg agacagcctc ttacttgcca agaaaatgaa gggattggac 2400
cgagctggaa aacctccttt accagatgct gactggcact ggtggttttt gctctcgaca 2460
gtatccacaa tagctgacgg ctgggtgttt cagtttgaaa atattttgtt gccttcatct 2520
tcactgcaat tttgtgtaaa tttctcaaag atctgaatta aataaataaa attcatttct 2580
acagacccac aaaaaaaaaa a 2601

<210> 17
<211> 2499
<212> DNA
<213> Homo sapiens

<220>
<221> misc_feature
<223> Gene ID: NQO1

<400> 17
ccgcccttgt aggctgtcca cctcaaacgg gccggacagg atatataaga gagaatgcac 60
cgtgcactac acacgcgact cccacaaggt tgcagccgga gccgcccagc tcaccgagag 120
cctagttccg gccagggtcg ccccggcaac cacgagccca gccaatcagc gccccggact 180

gcaccagagc catggtcggc agaagagcac tgatcgtact ggctcactca gagaggacgt 240
ccttcaacta tgccatgaag gaggctgctg cagcggcttt gaagaagaaa ggatgggagg 300
tggtggagtc ggacctctat gccatgaact tcaatcccat catttccaga aaggacatca 360
caggtaaact gaaggaccct gcgaactttc agtatcctgc cgagtctgtt ctggcttata 420
aagaaggcca tctgagccca gatattgtgg ctgaacaaaa gaagctggaa gccgcagacc 480
ttgtgatatt ccagttcccc ctgcagtggt ttggagtccc tgccattctg aaaggctggt 540
ttgagcgagt gttcatagga gagtttgctt acacttacgc tgccatgtat gacaaaggac 600
ccttccggag tggcattctg catttctgtg gcttccaagt cttagaacct caactgacat 660
atagcattgg gcacactcca gcagacgccc gaattcaaat cctggaagga tggaagaaac 720
gcctggagaa tatttgggat gagacaccac tgtattttgc tccaagcagc ctctttgacc 780
taaacttcca ggcaggattc ttaatgaaaa aagaggtaca ggatgaggag aaaaacaaga 840
aatttggcct ttctgtgggc catcacttgg gcaagtccat cccaactgac aaccagatca 900
aagctagaaa atgagattcc ttagcctgga tttccttcta acatgttatc aaatctgggt 960
atctttccag gcttccctga cttgctttag tttttaagat ttgtgttttt ctttttccac 1020
aaggaataaa tgagagggaa tcgactgtat tcgtgcattt ttggatcatt tttaactgat 1080
tcttatgatt actatcatgg catataacca aaatccgact gggctcaaga ggccacttag 1140
ggaaagatgt agaaagatgc tagaaaaatg ttctttaaag gcatctacac aatttaattc 1200
ctctttttag ggctaaagtt ttagggtaca gtttggctag gtatcattca actctccaat 1260
gttctattaa tcacctctct gtagtttatg gcagaaggga attgctcaga gaaggaaaag 1320
actgaatcta cctgccctaa gggacttaac ttgtttggta gttagccatc taatgcttgt 1380
ttatgatatt tcttgctttc aattacaaag cagttactaa tatgcctagc acaagtacca 1440
ctcttggtca gcttttgttg tttatataca gtacacagat accttgaaag gaagagctaa 1500
taaatctctt ctttgctgca gtcatctact ttttttttaa ttaaaaaaaa tttttttttg 1560
aagcagtctt gctctgttac ccaggctgga gtgcagtggt gtgatctcgg ctcactgcaa 1620
cctctgcctc ccaggttcca gcaattctcc tgcctcagcc tccctagtag ctgggatgac 1680
aggcgcctgc catcatgcct gactaatttt tgtattttta gtagagacgg cgtttcacca 1740
tgttggccag gctggtctca aactcctgac ctcaggtgat ccgcctacct cagcctccca 1800
aagtgctggg attacaggcg tgatccacca cacctggccc ttgcaatctt ctactttaag 1860
gtttgcagag ataaaccaat aaatccacac cgtacatctg caatatgaat tcaagaaagg 1920
aaatagtacc ttcaatactt aaaaatagtc ttccacaaaa aatactttat ttctgatcta 1980
tacaaatttt cagaaggtta ttttctttat cattgctaaa ctgatgactt actatgggat 2040
ggggtccagt cccatgacct tggggtacaa ttgtaaacct agagttttat caactttggt 2100
gaacagtttt ggcataatag tcaatttcta cttctggaag tcatctcatt ccactgttgg 2160
tattatataa ttcaaggaga atatgataaa acactgccct cttgtggtgc attgaaagaa 2220
gagatgagaa atgatgaaaa ggttgcctga aaaatgggag acagcctctt acttgccaag 2280
aaaatgaagg gattggaccg agctggaaaa cctcctttac cagatgctga ctggcactgg 2340
tggtttttgc tctcgacagt atccacaata gctgacggct gggtgtttca gtttgaaaat 2400
attttgttgc cttcatcttc actgcaattt tgtgtaaatt tctcaaagat ctgaattaaa 2460
taaataaaat tcatttctac agacccacaa aaaaaaaaa 2499

<210> 18

<211>  2487
<212>  DNA
<213>  Homo sapiens

<220>
<221>  misc_feature
<223>  Gene ID: NQO1

<400>  18
ccgcccttgt aggctgtcca cctcaaacgg gccggacagg atatataaga gagaatgcac      60
cgtgcactac acacgcgact cccacaaggt tgcagccgga gccgcccagc tcaccgagag     120
cctagttccg gccagggtcg ccccggcaac cacgagccca gccaatcagc gccccggact     180
gcaccagagc catggtcggc agaagagcac tgatcgtact ggctcactca gagaggacgt     240
ccttcaacta tgccatgaag gaggctgctg cagcggcttt gaagaagaaa ggatgggagg     300
tggtggagtc ggacctctat gccatgaact tcaatcccat catttccaga aaggacatca     360
caggtaaact gaaggaccct gcgaactttc agtatcctgc cgagtctgtt ctggcttata     420
aagaaggcca tctgagccca gatattgtgg ctgaacaaaa gaagctggaa gccgcagacc     480
ttgtgatatt ccagagtaag aaggcagtgc tttccatcac cactggtggc agtggctcca     540
tgtactctct gcaagggatc cacggggaca tgaatgtcat tctctggcca attcagagtg     600
gcattctgca tttctgtggc ttccaagtct tagaacctca actgacatat agcattgggc     660
acactccagc agacgcccga attcaaatcc tggaaggatg gaagaaacgc ctggagaata     720
tttgggatga gacaccactg tattttgctc caagcagcct ctttgaccta aacttccagg     780
caggattctt aatgaaaaaa gaggtacagg atgaggagaa aaacaagaaa tttggccttt     840
ctgtgggcca tcacttgggc aagtccatcc caactgacaa ccagatcaaa gctagaaaat     900
gagattcctt agcctggatt tccttctaac atgttatcaa atctgggtat ctttccaggc     960
ttccctgact tgctttagtt tttaagattt gtgtttttct ttttccacaa ggaataaatg    1020
agagggaatc gactgtattc gtgcattttt ggatcatttt taactgattc ttatgattac    1080
tatcatggca tataaccaaa atccgactgg gctcaagagg ccacttaggg aaagatgtag    1140
aaagatgcta gaaaaatgtt ctttaaaggc atctacacaa tttaattcct ctttttaggg    1200
ctaaagtttt agggtacagt ttggctaggt atcattcaac tctccaatgt tctattaatc    1260
acctctctgt agtttatggc agaagggaat tgctcagaga aggaaaagac tgaatctacc    1320
tgccctaagg gacttaactt gtttggtagt tagccatcta atgcttgttt atgatatttc    1380
ttgctttcaa ttacaaagca gttactaata tgcctagcac aagtaccact cttggtcagc    1440
ttttgttgtt tatatacagt acacagatac cttgaaagga agagctaata aatctcttct    1500
ttgctgcagt catctacttt ttttttaatt aaaaaaaatt tttttttgaa gcagtcttgc    1560
tctgttaccc aggctggagt gcagtggtgt gatctcggct cactgcaacc tctgcctccc    1620
aggttccagc aattctcctg cctcagcctc cctagtagct gggatgacag gcgcctgcca    1680
tcatgcctga ctaatttttg tatttttagt agagacggcg tttcaccatg ttggccaggc    1740
tggtctcaaa ctcctgacct caggtgatcc gcctacctca gcctcccaaa gtgctgggat    1800
tacaggcgtg atccaccaca cctggccctt gcaatcttct actttaaggt ttgcagagat    1860
aaaccaataa atccacaccg tacatctgca atatgaattc aagaaaggaa atagtacctt    1920

caatacttaa aaatagtctt ccacaaaaaa tactttattt ctgatctata caaattttca 1980
gaaggttatt ttctttatca ttgctaaact gatgacttac tatgggatgg ggtccagtcc 2040
catgaccttg gggtacaatt gtaaacctag agttttatca actttggtga acagttttgg 2100
cataatagtc aatttctact tctggaagtc atctcattcc actgttggta ttatataatt 2160
caaggagaat atgataaaac actgccctct tgtggtgcat tgaaagaaga gatgagaaat 2220
gatgaaaagg ttgcctgaaa aatgggagac agcctcttac ttgccaagaa aatgaaggga 2280
ttggaccgag ctggaaaacc tcctttacca gatgctgact ggcactggtg gtttttgctc 2340
tcgacagtat ccacaatagc tgacggctgg gtgtttcagt ttgaaaatat tttgttgcct 2400
tcatcttcac tgcaattttg tgtaaatttc tcaaagatct gaattaaata aataaaattc 2460
atttctacag acccacaaaa aaaaaaa 2487

<210> 19
<211> 4021
<212> DNA
<213> Homo sapiens

<220>
<221> misc_feature
<223> Gene ID: RBM35B

<400> 19
ggtagccgcc ccgccccgcg gggcgccacg ggcgggtctt ggcagcgccc actgagccag 60
ccgggccgca ggtgccgccc ccgatacacg gtgtcccgcc caagctgatc cgcgtctgcg 120
gtcggtcggt gcgtgcgtgc gcctcgtcgg tccgcgtgtc tggccgagag cccccttcct 180
ctgcggccat gactccgccg ccgccgccgc cccctccccc gggccctgac cccgcggccg 240
accccgccgc ggacccctgc ccctggcccg gatcactggt cgtcctcttc ggggctacgg 300
cgggtgcgct gggacgggac ctgggctcgg acgagaccga cttaatcctc ctagtttggc 360
aagtggttga gccgcggagc cgccaggtgg ggacgctgca caaatcgctg gttcgtgccg 420
aggcggccgc actgagtacg cagtgccgcg aggcgagcgg cctgagcgcc gacagcctgg 480
cgcgggcaga gccgctggac aaggtgctgc agcagttctc acagctggtg aacggggatg 540
tggctttgct gggcgggggc ccctacatgc tctgcactga tgggcagcag ctattgcgac 600
aggtcctgca ccccgaggcc tccaggaaga acctggtgct ccccgacatg ttcttctcct 660
tctatgacct ccgaagagaa ttccatatgc agcatccaag cacctgccct gccagggacc 720
tcactgtggc caccatggca cagggtttag gactggagac agatgccaca gaggatgact 780
ttggggtctg ggaagtcaag acaatggtag ctgttatcct ccatctactc aaagagccca 840
gcagtcaatt gttttcgaag cccgaggtga taaagcagaa atacgagacg gggccttgca 900
gcaaggctga tgtggtggac agtgagactg tggtacgggc tcgtgggttg ccgtggcagt 960
catcagacca ggacgtggct cgcttcttca aagggctcaa cgtggccagg ggtggtgtag 1020
cactctgcct caacgcccag ggccgcagaa atggcgaggc cctcatccgc tttgtggaca 1080
gcgagcagcg ggacctagcg ctgcagagac acaagcacca catgggcgtc cgctatattg 1140
aggtgtataa agcgacaggg gaggagtttg taaagattgc agggggcaca tcactagagg 1200

tggctcgttt cttgtcacgg gaagaccaag tgatcctgcg gctgcgggga ctgcccttct 1260
cggctgggcc aacggacgtg cttggcttcc tggggccaga gtgcccagtg actgggggta 1320
ccgaggggct gctctttgtg cgccatcctg atggccggcc gactggtgat gccttcgccc 1380
tctttgcttg tgaggagctg gcacaggctg cactgcgcag gcacaagggc atgctgggta 1440
agcgatacat tgaactcttc cggagcactg cagccgaagt gcagcaggtc ttgaaccgct 1500
atgcatccgg cccactcctt cctacactga ctgccccact gctgcccatc cccttcccac 1560
tggcacctgg gactgggagg gactgtgtac gcctccgagg cctgccctac acggccacca 1620
ttgaagacat cctgagcttt ctgggggagg cagcagctga cattcggccc cacggtgtac 1680
acatggtgct caaccagcag ggccggccat cgggcgatgc cttcattcag atgacatcag 1740
cagagcgagc cctagctgct gctcagcgtt gccataagaa ggtgatgaag gagcgctacg 1800
tggaggtggt cccctgttcc acagaggaga tgagccgagt gctgatgggg ggcaccttgg 1860
gccgcagtgg catgtcccct ccaccctgca agctgccctg cctctcacca cctacctaca 1920
ccaccttcca agccacccca acgctcattc ccacggagac ggcagctcta tacccctctt 1980
cagcactgct cccagctgcc agggtgcctg ctgcccccac ccctgttgcc tactatccag 2040
ggccagccac tcaactctac ctgaactaca cagcctacta cccaagcccc ccagtctccc 2100
ccaccactgt gggctacctc actacaccca ctgctgccct ggcctctgct cccacctcag 2160
tgttgtccca gtcaggagcc ttggtccgca tgcagggtgt cccatacacg gctggtatga 2220
aggatctgct cagcgtcttc caggcctacc agctacccgc tgatgactac accagtctga 2280
tgcctgttgg tgacccacct cgcactgtgt tacaagcccc caaggaatgg gtgtgtttgt 2340
aggagagaaa gccaggaggt aagagccagc tgatatcctc ggcgaacatg tctctcctga 2400
gtccagaaga ccagcaccct caacctggta gcttctttct ggcttgtcaa agctctcaga 2460
aggtacctag aggagcccaa gccccagctc catcctccac ttattctgcc tgtttccccc 2520
aaagacaatg gctggaccct gcatgcaggg ctgggggtgg aatggggcta accagctcct 2580
gatggcctga gccaggcatc ttgactggca cctggagagc ccttaagtct gtcctggctg 2640
tggcccatgc cgacagatat cgtggggctg acaggtccac ggcaggcttg ctttctttta 2700
taaaatggaa gctctggtac cttcaatgta tgactcctgg gagaatcaag ggtccatctg 2760
agcctctgag taaagatccc aatgttctac ctctccctgt ccctcttgta ggggataggg 2820
aggcagagag agccagcccc taccctcaga gtatctggac ctcagagacc atgttgtgcc 2880
aggggtggtc ccacctaaag atgctagccc ctctccaggt gggcataagg agtaacagat 2940
ggcaaaacca caaactattt tgatggactg tgctgcagta tcaccagaag acattagggg 3000
gcagtaggcc cccacacaaa accttcaggc ttgaatttta aaggggagga ctttctgcca 3060
acttttcttg tatgccttgg gaaagccagt tgccctgaac ccagcagaca ccatggaatg 3120
tcctttgcac gcattaaatg gtacagaact gaagcctcgg aagcaatttg gaactcgatc 3180
ttctcttcct taaatgaaaa gttattgacc aaatggactt tttaaaagac acaggaccct 3240
taactttgcc ccaaagtgag gggctccaca ccaaccccag gcggaggaac actcagacag 3300
attaaggata ctgttgacct gtcactgttt attatttcag cactaaaact gaggagcctc 3360
aactgctggc tcttcttccc tttgtatttg tgtaaggagc actgcactcc cataaaaggt 3420
tttaaaatac aaaatgtaca agaacacaca attccaagtg ctgtaaacat aactgagaac 3480
cagttccttt actaaacatc cattttataa aacacaaggt ttcaatttga gcccatctga 3540
gccttaaaga tccattctga ataccaaaaa cagggcttca cagccaggcc cagaagaggt 3600
ctggtgataa tggctggccc tgggtgggga tagtttacac ccgggcagca gcaccacaca 3660

tgaacccaaa gacatgttct ttttaaagct gttttcagcc atgtttctct gtgcatctcc 3720
agtaagcaga aggctaccca ttccattcct caacccaaga gctagcacag ttagagtagg 3780
agggggtgcg tactagcacg tgcccagttg ctcagtgctg ctagtagaaa ttgatttgca 3840
tagtccaatg gatgtgtgct ttaacaccac tatgttgcac aaaaatttaa gtctttatct 3900
acaaagccaa aaaatattga ctcttaacac caaagctttt acaaagctga tataaaactg 3960
cttacatagt atacaaagct ctattttaaa atttaatgtt tattttaaat aggaaagcat 4020
t 4021

<210> 20
<211> 3226
<212> DNA
<213> Homo sapiens

<220>
<221> misc_feature
<223> Gene ID: TMPRSS

<400> 20
cgcgagctaa gcaggaggcg gaggcggagg cggagggcga ggggcgggga gcgccgcctg 60
gagcgcggca ggtcatattg aacattccag atacctatca ttactcgatg ctgttgataa 120
cagcaagatg gctttgaact cagggtcacc accagctatt ggaccttact atgaaaacca 180
tggataccaa ccggaaaacc cctatcccgc acagcccact gtggtcccca ctgtctacga 240
ggtgcatccg gctcagtact acccgtcccc cgtgccccag tacgccccga gggtcctgac 300
gcaggcttcc aaccccgtcg tctgcacgca gcccaaatcc ccatccggga cagtgtgcac 360
ctcaaagact aagaaagcac tgtgcatcac cttgaccctg gggaccttcc tcgtgggagc 420
tgcgctggcc gctggcctac tctggaagtt catgggcagc aagtgctcca actctgggat 480
agagtgcgac tcctcaggta cctgcatcaa cccctctaac tggtgtgatg gcgtgtcaca 540
ctgccccggc ggggaggacg agaatcggtg tgttcgcctc tacggaccaa acttcatcct 600
tcagatgtac tcatctcaga ggaagtcctg gcaccctgtg tgccaagacg actggaacga 660
gaactacggg cgggcggcct gcagggacat gggctataag aataattttt actctagcca 720
aggaatagtg gatgacagcg gatccaccag ctttatgaaa ctgaacacaa gtgccggcaa 780
tgtcgatatc tataaaaaac tgtaccacag tgatgcctgt tcttcaaaag cagtggtttc 840
tttacgctgt atagcctgcg gggtcaactt gaactcaagc cgccagagca ggatcgtggg 900
cggtgagagc gcgctcccgg gggcctggcc ctggcaggtc agcctgcacg tccagaacgt 960
ccacgtgtgc ggaggctcca tcatcacccc cgagtggatc gtgacagccg cccactgcgt 1020
ggaaaaacct cttaacaatc catggcattg gacggcattt gcggggattt tgagacaatc 1080
tttcatgttc tatggagccg gataccaagt agaaaaagtg atttctcatc caaattatga 1140
ctccaagacc aagaacaatg acattgcgct gatgaagctg cagaagcctc tgactttcaa 1200
cgacctagtg aaaccagtgt gtctgcccaa cccaggcatg atgctgcagc cagaacagct 1260
ctgctggatt tccgggtggg gggccaccga ggagaaaggg aagacctcag aagtgctgaa 1320
cgctgccaag gtgcttctca ttgagacaca gagatgcaac agcagatatg tctatgacaa 1380

cctgatcaca ccagccatga tctgtgccgg cttcctgcag gggaacgtcg attcttgcca 1440
gggtgacagt ggagggcctc tggtcacttc gaagaacaat atctggtggc tgatagggga 1500
tacaagctgg ggttctggct gtgccaaagc ttacagacca ggagtgtacg ggaatgtgat 1560
ggtattcacg gactggattt atcgacaaat gagggcagac ggctaatcca catggtcttc 1620
gtccttgacg tcgttttaca agaaaacaat ggggctggtt ttgcttcccc gtgcatgatt 1680
tactcttaga gatgattcag aggtcacttc atttttatta aacagtgaac ttgtctggct 1740
ttggcactct ctgccattct gtgcaggctg cagtggctcc cctgcccagc ctgctctccc 1800
taaccccttg tccgcaaggg gtgatggccg gctggttgtg ggcactggcg gtcaagtgtg 1860
gaggagaggg gtggaggctg ccccattgag atcttcctgc tgagtccttt ccaggggcca 1920
attttggatg agcatggagc tgtcacctct cagctgctgg atgacttgag atgaaaaagg 1980
agagacatgg aaagggagac agccaggtgg cacctgcagc ggctgccctc tggggccact 2040
tggtagtgtc cccagcctac ctctccacaa ggggattttg ctgatgggtt cttagagcct 2100
tagcagccct ggatggtggc cagaaataaa gggaccagcc cttcatgggt ggtgacgtgg 2160
tagtcacttg taaggggaac agaaacattt ttgttcttat ggggtgagaa tatagacagt 2220
gcccttggtg cgagggaagc aattgaaaag gaacttgccc tgagcactcc tggtgcaggt 2280
ctccacctgc acattgggtg gggctcctgg gagggagact cagccttcct cctcatcctc 2340
cctgaccctg ctcctagcac cctggagagt gcacatgccc cttggtcctg gcagggcgcc 2400
aagtctggca ccatgttggc ctcttcaggc ctgctagtca ctggaaattg aggtccatgg 2460
gggaaatcaa ggatgctcag tttaaggtac actgtttcca tgttatgttt ctacacattg 2520
ctacctcagt gctcctggaa acttagcttt tgatgtctcc aagtagtcca ccttcattta 2580
actctttgaa actgtatcac ctttgccaag taagagtggt ggcctatttc agctgctttg 2640
acaaaatgac tggctcctga cttaacgttc tataaatgaa tgtgctgaag caaagtgccc 2700
atggtggcgg cgaagaagag aaagatgtgt tttgttttgg actctctgtg gtcccttcca 2760
atgctgtggg tttccaacca ggggaagggt cccttttgca ttgccaagtg ccataaccat 2820
gagcactact ctaccatggt tctgcctcct ggccaagcag gctggtttgc aagaatgaaa 2880
tgaatgattc tacagctagg acttaacctt gaaatggaaa gtcttgcaat cccatttgca 2940
ggatccgtct gtgcacatgc ctctgtagag agcagcattc ccagggacct tggaaacagt 3000
tggcactgta aggtgcttgc tccccaagac acatcctaaa aggtgttgta atggtgaaaa 3060
cgtcttcctt ctttattgcc ccttcttatt tatgtgaaca actgtttgtc tttttttgta 3120
tcttttttaa actgtaaagt tcaattgtga aaatgaatat catgcaaata aattatgcga 3180
tttttttttc aaagcaaaaa aaaaaaaaaa aaaaaaaaaa aaaaaa 3226

<210> 21
<211> 25
<212> DNA
<213> Homo sapiens

<220>
<221> misc_feature
<223> Gene ID: ANXA3 ; Taken from Table 4 of the Specification

<400>  21
tcagaggaat tggaactgat gagaa                                            25

<210>  22
<211>  25
<212>  DNA
<213>  Homo sapiens

<220>
<221>  misc_feature
<223>  Gene ID: AP1M2; Taken from Table 4 of the Specification

<400>  22
agggcaagcc attgatcagc cgcaa                                            25

<210>  23
<211>  25
<212>  DNA
<213>  Homo sapiens

<220>
<221>  misc_feature
<223>  Gene ID: ATP1B1; Taken from Table 4 of the Specification

<400>  23
cagtgcactg gcaagcgaga tgaag                                            25

<210>  24
<211>  25
<212>  DNA
<213>  Homo sapiens

<220>
<221>  misc_feature
<223>  Gene ID: C11orf9; Sequence taken from Table 4 of the
       Specification

<400>  24
gtcatggcct tcagcgtggt gtcca                                            25

<210>  25

<211>  25
<212>  DNA
<213>  Homo sapiens

<220>
<221>  misc_feature
<223>  Gene ID: C1orf106; Taken from Table 4 of the Specification

<400>  25
tgggacagct ggggaagctc caggc                                          25

<210>  26
<211>  25
<212>  DNA
<213>  Homo sapiens

<220>
<221>  misc_feature
<223>  Gene ID: CDH1; Taken from Table 4 of the Specification

<400>  26
cccgccccat caggcctccg tttct                                          25

<210>  27
<211>  25
<212>  DNA
<213>  Homo sapiens

<220>
<221>  misc_feature
<223>  Gene ID: COBL; Taken from Table 4 of the Specification

<400>  27
ctgagaaagg tgcccttgct cgtgt                                          25

<210>  28
<211>  25
<212>  DNA
<213>  Homo sapiens

<220>

<221>  misc_feature
<223>  Gene ID: CYP3A7; Taken from Table 4 of the Specification

<400>  28
ttattttgat ggctctaaca gtgac                                          25

<210>  29
<211>  25
<212>  DNA
<213>  Homo sapiens

<220>
<221>  misc_feature
<223>  Gene ID: DSG2; Taken from Table 4 of the Specification

<400>  29
cagagaggaa cacagcagct acact                                          25

<210>  30
<211>  25
<212>  DNA
<213>  Homo sapiens

<220>
<221>  misc_feature
<223>  Gene ID: ELF3; Taken from Table 4 of the Specification

<400>  30
caagcacgcg cccagaggca cccac                                          25

<210>  31
<211>  25
<212>  DNA
<213>  Homo sapiens

<220>
<221>  misc_feature
<223>  Gene ID: ERBB3; Taken from Table 4 of the Specification

<400>  31
gcaactctca ggcagtgtgt cctgg                                          25

<210> 32
<211> 25
<212> DNA
<213> Homo sapiens

<220>
<221> misc_feature
<223> Gene ID: KLF5; Taken from Table 4 of the Specification

<400> 32
actgcgatta ccctggttgc acaaa 25

<210> 33
<211> 25
<212> DNA
<213> Homo sapiens

<220>
<221> misc_feature
<223> Gene ID: MAP7; Taken from Table 4 of the Specification

<400> 33
gcgaagcgaa acagcacccg acagc 25

<210> 34
<211> 25
<212> DNA
<213> Homo sapiens

<220>
<221> misc_feature
<223> Gene ID: NQO1; Taken from Table 4 of the Specification

<400> 34
gagccatggt cggcagaaga gcact 25

<210> 35
<211> 25
<212> DNA
<213> Homo sapiens

<220>

<221>  misc_feature
<223>  Gene ID: RBM35B; Taken from Table 4 of the Specification

<400>  35
gtgctgcagc agttctcaca gctgg                                          25

<210>  36
<211>  25
<212>  DNA
<213>  Homo sapiens

<220>
<221>  misc_feature
<223>  Gene ID: TMPRSS2; Taken from Table 4 of the Specification

<400>  36
tcggtgtgtt cgcctctacg gacca                                          25

<210>  37
<211>  1331
<212>  DNA
<213>  Homo sapiens

<220>
<221>  misc_feature
<223>  Gene ID: HPRT1*; Taken from Table 4 of the Specification

<400>  37
tcttgctgcg cctccgcctc ctcctctgct ccgccaccgg cttcctcctc ctgagcagtc    60
agcccgcgcg ccggccggct ccgttatggc gacccgcagc cctggcgtcg tgattagtga   120
tgatgaacca ggttatgacc ttgatttatt ttgcatacct aatcattatg ctgaggattt   180
ggaaagggtg tttattcctc atggactaat tatggacagg actgaacgtc ttgctcgaga   240
tgtgatgaag gagatgggag gccatcacat tgtagccctc tgtgtgctca aggggggcta   300
taaattcttt gctgacctgc tggattacat caaagcactg aatagaaata gtgatagatc   360
cattcctatg actgtagatt ttatcagact gaagagctat tgtaatgacc agtcaacagg   420
ggacataaaa gtaattggtg gagatgatct ctcaacttta actggaaaga atgtcttgat   480
tgtggaagat ataattgaca ctggcaaaac aatgcagact ttgctttcct tggtcaggca   540
gtataatcca aagatggtca aggtcgcaag cttgctggtg aaaaggaccc cacgaagtgt   600
tggatataag ccagactttg ttggatttga aattccagac aagtttgttg taggatatgc   660

ccttgactat aatgaatact tcagggattt gaatcatgtt tgtgtcatta gtgaaactgg 720
aaaagcaaaa tacaaagcct aagatgagag ttcaagttga gtttggaaac atctggagtc 780
ctattgacat cgccagtaaa attatcaatg ttctagttct gtggccatct gcttagtaga 840
gctttttgca tgtatcttct aagaatttta tctgttttgt actttagaaa tgtcagttgc 900
tgcattccta aactgtttat ttgcactatg agcctataga ctatcagttc cctttgggcg 960
gattgttgtt taacttgtaa atgaaaaaat tctcttaaac cacagcacta ttgagtgaaa 1020
cattgaactc atatctgtaa gaaataaaga gaagatatat tagtttttta attggtattt 1080
taatttttat atatgcagga aagaatagaa gtgattgaat attgttaatt ataccaccgt 1140
gtgttagaaa agtaagaagc agtcaatttt cacatcaaag acagcatcta agaagttttg 1200
ttctgtcctg gaattatttt agtagtgttt cagtaatgtt gactgtattt tccaacttgt 1260
tcaaattatt accagtgaat ctttgtcagc agttcccttt taaatgcaaa tcaataaatt 1320
cccaaaaatt t 1331

<210> 38
<211> 2212
<212> DNA
<213> Homo sapiens

<220>
<221> misc_feature
<223> Gene ID: ATP1B1 ... NM_0010001787; Taken from Table 4 of the
      Specification

<400> 38
cagcggcgcg tcctgcctgc agagagccag gccggagaag ccgagcggcg cagaggacgc 60
cagggcgcgc gccgcagcca cccaccctcc ggaccgcggc agctgctgac ccgccatcgc 120
catggcccgc gggaaagcca aggaggaggg cagctggaag aaattcatct ggaactcaga 180
gaagaaggag tttctgggca ggaccggtgg cagttggttt aagatccttc tattctacgt 240
aatattttat ggctgcctgg ctggcatctt catcggaacc atccaagtga tgctgctcac 300
catcagtgaa tttaagccca catatcagga ccgagtggcc ccgccaggat taacacagat 360
tcctcagatc cagaagactg aaatttcctt tcgtcctaat gatcccaaga gctatgaggc 420
atatgtactg aacatagtta ggttcctgga aaagtacaaa gattcagccc agagggatga 480
catgattttt gaagattgtg gcgatgtgcc cagtgaaccg aaagaacgag gagactttaa 540
tcatgaacga ggagagcgaa aggtctgcag attcaagctt gaatggctgg gaaattgctc 600
tggattaaat gatgaaactt atggctacaa agagggcaaa ccgtgcatta ttataaagct 660
caaccgagtt ctaggcttca aacctaagcc tcccaagaat gagtccttgg agacttaccc 720
agtgatgaag tataacccaa atgtccttcc cgttcagtgc actggcaagc gagatgaaga 780
taaggataaa gttggaaatg tggagtattt tggactgggc aactcccctg gttttcctct 840
gcagtattat ccgtactatg gcaaactcct gcagcccaaa tacctgcagc ccctgctggc 900
cgtacagttc accaatctta ccatggacac tgaaattcgc atagagtgta aggcgtacgg 960
tgagaacatt gggtacagtg agaaagaccg ttttcaggga cgttttgatg taaaaattga 1020

agttaagagc tgatcacaag cacaaatctt tcccactagc catttaataa gttaaaaaaa 1080
gatacaaaaa caaaaaccta ctagtcttga acaaactgtc atacgtatgg gacctacact 1140
taatctatat gctttacact agctttctgc atttaatagg ttagaatgta aattaaagtg 1200
tagcaatagc aacaaaatat ttattctact gtaaatgaca aaagaaaaag aaaaattgag 1260
ccttgggacg tgcccatttt tactgtaaat tatgattccg taactgactt gtagtaagca 1320
gtgtttctgg cccctaagta ttgctgcctt gtgtatttta tttagtgtac agtactacag 1380
gtgcatactc tggtcatttt tcaagccatg ttttattgta tctgttttct actttatgtg 1440
agcaaggttt gctgtccaag gtgtaaatat tcaacgggaa taaaactggc atggtaattt 1500
tttttttttt tttttttttg ttttttggct ctttcaaagg taatggccca tcgatgagca 1560
tttttaacat actccatagt cttttcctgt ggtgttaggt ctttattttt atttttttcc 1620
tgggggctgg ggtgggggtt tgtcatgggg gaactgccct ttaaatttta agtgacacta 1680
cagaaaaaca caaaaaggtg atgggttgtg ttatgcttgt attgaatgct gtcttgacat 1740
ctcttgcctt gtcctccggt atgttctaaa gctgtgtctg agatctggat ctgcccatca 1800
ctttggctag tgacagggct aattaatttg ctttatacat tttcttttac tttccttttt 1860
tcctttctgg aggcatcaca tgctggtgct gtgtctttat gaatgtttta accattttca 1920
tggtggaaga attttatatt tatgcagttg tacaatttta tttttttctg caagaaaaag 1980
tgtaatgtat gaaataaacc aaagtcactt gtttgaaaat aaatctttat tttgaacttt 2040
ataaaaagca atgcagtacc ccatagactg gtgttaaatg ttgtctacag tgcaaaatcc 2100
atgttctaac atatgtaata attgccagga gtacagtgct cttgttgatc ttgtattcag 2160
tcaggttaaa acaacggaca ataaaagaat gaacacattc aaaaaaaaaa aa 2212

<210> 39
<211> 1050
<212> DNA
<213> Homo sapiens

<220>
<221> misc_feature
<223> Gene ID: ERBB3 ... NM_001005915

<400> 39
acacacacac acccctcccc tgccatccct ccccggactc cggctccggc tccgattgca 60
atttgcaacc tccgctgccg tcgccgcagc agccaccaat tcgccagcgg ttcaggtggc 120
tcttgcctcg atgtcctagc ctaggggccc ccgggccgga cttggctggg ctcccttcac 180
cctctgcgga gtcatgaggg cgaacgacgc tctgcaggtg ctgggcttgc ttttcagcct 240
ggcccggggc tccgaggtgg gcaactctca ggcagtgtgt cctgggactc tgaatggcct 300
gagtgtgacc ggcgatgctg agaaccaata ccagacactg tacaagctct acgagaggtg 360
tgaggtggtg atggggaacc ttgagattgt gctcacggga cacaatgccg acctctcctt 420
cctgcagtgg attcgagaag tgacaggcta tgtcctcgtg gccatgaatg aattctctac 480
tctaccattg cccaacctcc gcgtggtgcg agggacccag gtctacgatg ggaagtttgc 540
catcttcgtc atgttgaact ataacaccaa ctccagccac gctctgcgcc agctccgctt 600

gactcagctc accggtcagt tcccgatggt tccttctggc ctcacccctc agccagccca 660
agactggtac ctccttgatg atgacccaag actgctcact ctaagtgcct cttccaaggt 720
gcctgtcacc ttggccgctg tctaaaggtc cattgctccc taagcaatag agggccccca 780
gtagggggag ctaggggcat ctgctccagg gaaaggaacc ctgtgtcctt gtggggctgg 840
agtcagagct ggatctgtta accgtttttc taatttcaaa gtacagtgta ccggaggcca 900
ggcctgatgg cttacacctg taatcccagc attttgggag gccaaggagg gcagatcact 960
tgagatcagg agtttgagac cagcctggcc aacatggcga aaccctgtct ctactaaaaa 1020
tacaaaaaaa taaaataaaa taaaaaatta 1050

## Description

### BACKGROUND OF THE INVENTION

There are about 876,000 new stomach cancer cases each year worldwide (Shibuya, Mathers et al. 2002). In the United States, the estimated new cases and deaths in 2005 for stomach cancer are 21,860 and 11,550 respectively (Jemal, Murray et al. 2005). Gastric carcinoma is the most common type (90% to 95%) of stomach cancer, which is followed in the order of incidence by lymphomas, carcinoids and mesenchymal spindle cell tumors (Kumar, Cotran et al. 2003).

Complete local regional control is the key treatment for surviving gastric cancer, which includes total or subtotal gastrectomy, sentinel lymph node dissection, extended lymph node dissection and resection of adjacent tissues affected by metastasis such as splenectomy and pancreatectomy (Hartgrink and van de Velde 2005; Kitagawa, Fujii et al. 2005). Out of all the patients who suffer relapse after curative surgery, up to 87.5% of them are caused by local recurrence or remained regional lymph node metastasis (Songun, Bonenkamp et al. 1996).

In a study covering 148 patients with gastro-esophageal junction (GEJ) carcinomas, it was concluded that the presence of micro metastasis in local lymph nodes is a key indicator of a poor prognosis (Heeren, Kelder et al. 2005). The current prevalent staging/diagnosis of lymph node metastasis from gastric cancer is achieved by palpation and intra-operative frozen section examination. Frozen section examination of lymph nodes can exhibit sensitivity of 74%(Kitagawa, Fujii et al. 2005).

With the appropriate gene markers, gene expression analysis and detection technology such as RT-PCR or quantitative PCR could provide an alternative method for the diagnosis or prognosis of gastric cancer. These methods have potential to provide better sensitivity and accuracy for metastasis detection test. The previously reported gene markers include CK19 (Matsuda, Kitagawa et al. 2004), Carcinoembryonic antigen (CEA), cytokeratin-20 (CK-20), and MAGE-3 (Okada, Fujiwara et al. 2001), CK18, MUC1, hTRT (Ajisaka and Miwa 2003). Unfortunately, as published data shows, most of them are not consistently expressed in all of the tumor tissues and tumor cell lines tested. Markers could not be detected by RT-PCR in 15% (CEA) to over 70% (MAGE-3) of the lymph nodes with metastasis from gastric cancer confirmed by histological examination (Okada, Fujiwara et al. 2001). CK20 was used as a marker for RT-PCR tests for disseminated cancer cells in blood from pancreas, colon, stomach, and lung carcinoma (Chausovsky, Luchansky et al. 1999; Chen, Chen et al. 2004).

Here, markers are identified that are consistently expressed in lymph node with metastasis from gastric carcinoma at easily detectable levels which can be differentiated from their background expression in normal lymph nodes. These markers are useful for the detection of disseminated carcinoma cells from gastric carcinoma, or carcinoma of similar cell origin, such as carcinoma of the esophagus, pancreas, and intestine.

### SUMMARY OF THE INVENTION

In one aspect of the invention, a set of 18 gene markers are identified for detection of lymph node metastasis from gastric carcinoma. These marker genes are expressed at high levels in normal stomach tissues, and the expressions are maintained well in gastric carcinoma with different differentiation status while the background expression levels of these genes are relatively low in normal lymph nodes. In lymph nodes with metastasis from gastric carcinoma, the expression levels of these gene markers are distinctively higher than that of normal lymph nodes.

In another aspect of the invention, methods of diagnosing lymph node metastasis from gastric carcinoma involve obtaining biological samples from a patient and measuring the expression levels of genes selected from a group of Markers, where the gene expression levels above pre-determined cut-off levels are indicative of metastasis from gastric carcinoma. Preferably, the biological samples are lymph nodes samples.

In another aspect of the invention, Markers can be used individually or in combination in assays to detect lymph node metastasis from gastric carcinoma, which may be used to help clinical decision-making.

In another aspect of the invention, Mene markers can be used individually or in combination in assays to identify the origin of metastasis of carcinoma that has a similar origin as gastric carcinoma, such as carcinoma of the pancreas, esophagus, or intestine.

In another aspect of the invention, Markers could be used individually or in combination to collect, detect and identify disseminated cancer cells in blood from gastric carcinoma, or carcinoma with similar tissue origins, such as cancer of the pancreas, esophagus, or intestine.

In another aspect of the invention, Markers are used individually or in combination in immunocytochemistry study for detection of metastasis from gastric carcinoma, or carcinoma with similar tissue origins, such as cancer of the pancreas, esophagus, or intestine.

In yet another aspect of the invention, Markers can be assayed intraoperatively. The intraoperative assays can be conducted by a method that includes the steps of: preparing RNA from a lymph node that drains from the stomach; performing a quantitative RT-PCR method specific to one or more Markers and determining if the presence of the Marker exceeds a predetermined cut-off. The cut-off value can be an absolute value or a value relative to the expression of a control gene(s).

The present invention encompasses microarray or gene chips for performing the methods provided herein.

### BRIEF DESCRIPTION OF THE DRAWINGS

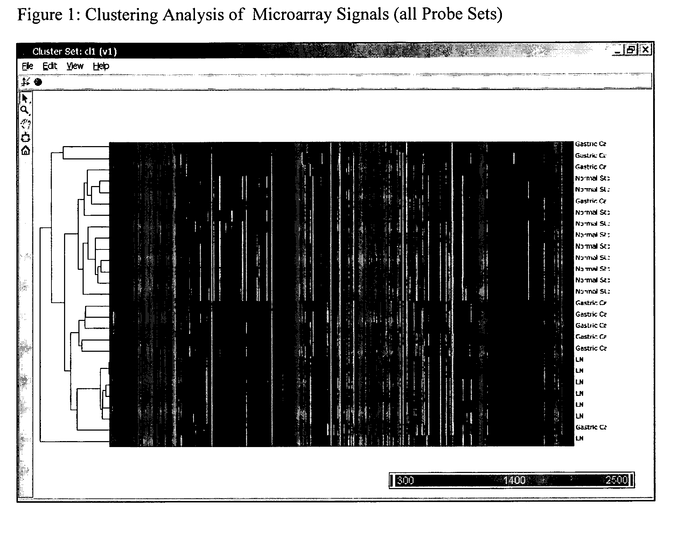

Figure 1 is a representation of the hierarchical clustering analysis of microarray results (HG U133A, signal from all probe sets) from 10 gastric carcinoma, 10 normal stomach tissue and 6 normal lymph nodes (shown as LN) (7 chips for normal lymph nodes, one sample was run twice on chips) from Example 1, Marker Identification.

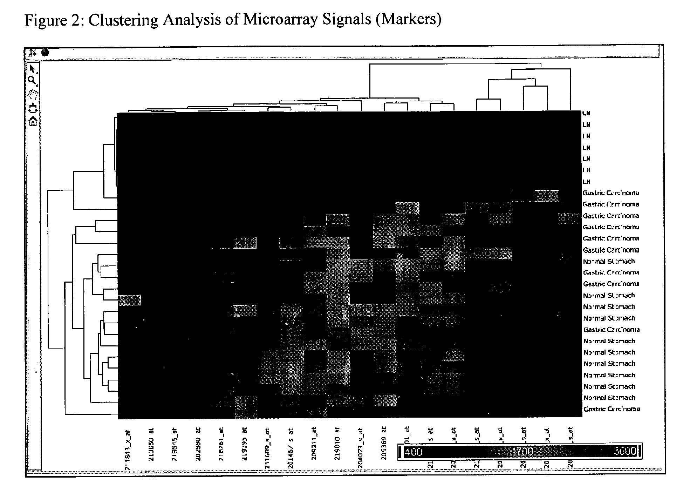

Figure 2 is a representation of the hierarchical clustering analysis of microarray results (HG U133A, signal from 20 selected marker gene probe sets) from 10 gastric carcinoma, 10 normal stomach tissue and 6 normal lymph nodes (7 chips for normal lymph nodes, one sample was run twice on chips). The plot shows the chip signal levels of 20 probe sets (representing 18 genes) among samples of gastric carcinoma, normal stomach tissue and normal lymph node. The distinctive difference in gene signal levels between the normal lymph node (shown as LN) group and the gastric carcinoma/normal stomach tissues is illustrated from Example 1, Marker Identification.

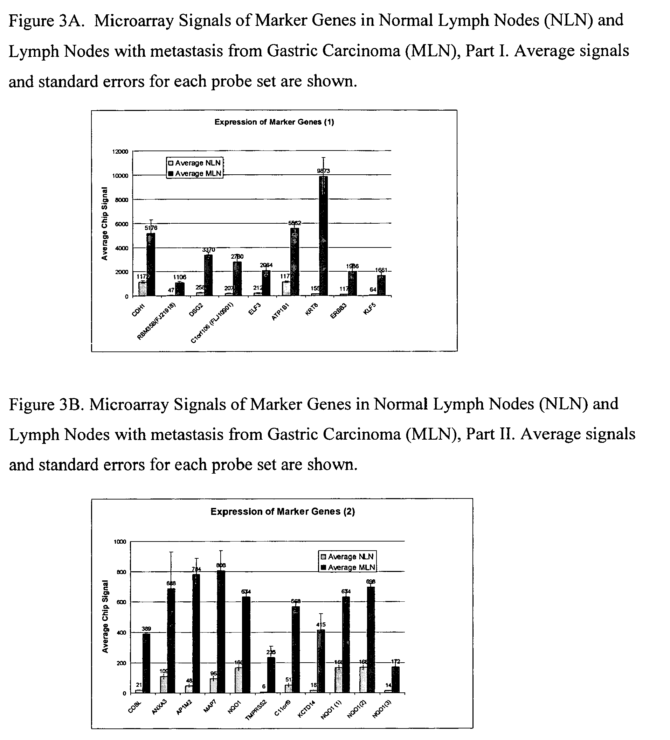

Figure 3A and 3B show the average and standard error of the identified marker gene signals for Confirmation Study (1), which covers the microarray study of 6 normal lymph nodes and 3 lymph nodes with confirmed metastasis from gastric carcinoma described in Example 2, Confirmation Study (1) --- microarray test.

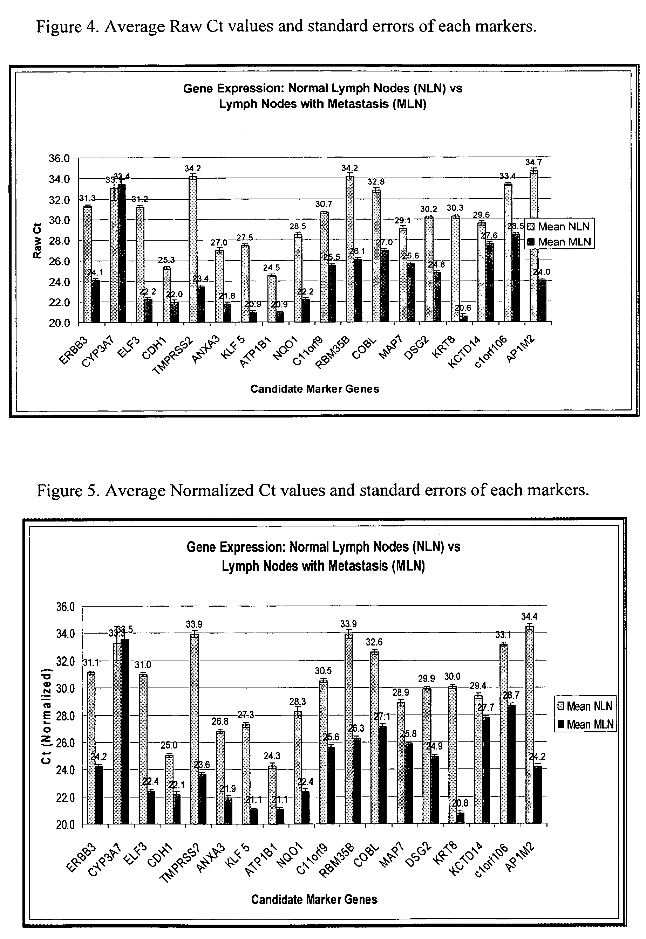

Figure 4 is a graphical portrayal of raw Ct results of a PCR study that covers a total of 10 normal lymph nodes from 6 different patients, and 16 lymph nodes with histologically confirmed metastasis from gastric carcinoma from 13 different patients. The average raw Ct and standard error for each marker genes are shown. The data was obtained as described in Example 2, Confirmation Study (2) --- Realtime RT-PCR Test.
Figure 5 is a graphical portrayal of normalized Ct results of a PCR study that covers a total of 10 normal lymph nodes from 6 different patients, and 16 lymph nodes with histologically confirmed metastasis from gastric carcinoma from 13 different patients. Ct values were normalized based on the house keeping gene HPRT 1 Ct results. The average normalized Ct and standard error for each marker genes are shown. The data was obtained according to Example 2, Confirmation Study (2) --- Realtime RT-PCR Test.

### DETAILED DESCRIPTION

The inventive methods, compositions, articles, and kits described and claimed in this specification include one or more Markers. "Marker" is used throughout this specification to refer to:
a) Genes and gene expression products such as mRNA and corresponding cDNA, peptides, proteins, fragments and complements of each of the foregoing, and
b) Compositions such as probes, antibodies, ligands, haptens, and labels that, through physical or chemical interaction with a) indicate the expression of the gene or presence of the gene expression product and wherein the gene, gene expression product or compositions correspond with:
   i) any of the genes set forth in Table 2;
   ii) a combination of genes, each of which is set forth in Table 2; and
   iii) a combination of all of the genes set forth in Table 2.

A gene corresponds to the sequence designated by a SEQ ID NO when it contains that sequence. A gene segment or fragment corresponds to the sequence of such gene when it contains a portion of the referenced sequence or its complement sufficient to distinguish it as being the sequence of the gene. A gene expression product corresponds to such sequence when its RNA, mRNA, or cDNA hybridizes to the composition having such sequence (e.g. a probe) or, in the case of a peptide or protein; it is encoded by such mRNA. A segment or fragment of a gene expression product corresponds to the sequence of such gene or gene expression product when it contains a portion of the referenced gene expression product or its complement sufficient to distinguish it as being the sequence of the gene or gene expression product.

Markers for ii and iii are preferred. Markers for iv and v are most preferred.

### Sample Preparation.

The distribution of metastases and micrometastases in tissues is not uniform in nodes or other tissues. Therefore, a sufficiently large sample should be obtained so that metastases will not be missed. One approach to the sampling issue in the present method is to homogenize a large tissue sample, and subsequently an appropriate portion of the homogenate is to be used to prepare RNA for molecular testing.

Preferably, the samples used in the methods of the invention are lymph node samples from lymph nodes that drain from the stomach. A useful procedure for preparing such a sample for RT-PCR assay follows; an Omni homogenizer and disposable probe for homogenization are used followed by purification of RNA with RNeasy (Qiagen) kit reagents:

### Homogenization

a) Determine the sample weight in milligrams, if not previously recorded. Place a fresh piece of wax paper on a balance, tare, and weigh the sample. Preferably, nodes samples are greater than 30 mg.
b) Using a fresh scalpel, mince all tissue from the same node into pieces approximately 4 mm in diameter. Care is taken to avoid contamination of the tissue during processing.
c) Add homogenization (RLT) buffer to the polypropylene homogenization tube. Preferably, use 2 mL homogenization buffer per 100 mg tissue.
d) Using a clean forceps, transfer the tissue into the homogenization buffer.
e) Place a new homogenization probe onto the manual homogenizer.
f) Homogenize each node completely.
g) Process the homogenate per the RNA Purification procedures in standard Qiagen protocol, RNA is stored at -65°C or below before use.
h) Dispose of the homogenization probe.
i) Store any remaining homogenate at -65°C or below for possible later use.

### Diagnostic Assays

The Markers of this invention are useful for providing important clinical information such as whether the gastric carcinoma has metastasized to lymph node, which is indicative to the clinical staging of gastric carcinoma and the prognosis for the patient.

Collectively, this information is referred to as "Diagnostic Information" in this specification. Additionally, such Diagnostic Information includes its use as a component in providing therapy. For example, a physician having such Diagnostic Information can base his therapy (e.g., adjuvant therapy) on an indication that the cancer has metastasized to lymph nodes.

Preferred methods for measuring the Markers of the invention include determining the amount of mRNA that is produced by a gene that can code for a protein or peptide. This can be accomplished by competitive reverse transcriptase PCR (RT-PCR), real time RT-PCR, Northern Blot analysis or other tests with similar capabilities. When a large number of Markers of the invention are to be measured simultaneously, it is preferable to generate amplified complementary RNA (cRNA) from mRNA (RNA) and then apply it to DNA microarray for testing and analysis. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology allows for the measurement of the steady-state mRNA level of thousands of genes simultaneously thereby presenting a powerful tool for identifying effects such as the onset, arrest, or modulation of uncontrolled cell proliferation. Although differences exist in the construction of microarray chips, the downstream data analysis and output are similar. The product of these analyses are typically measurements of the intensity of the signal received from a probe set used to detect a target sequence prepared from the sample that hybridizes to a nucleic acid sequence at a known location on the microarray. Typically, the intensity of the signal is proportional to the quantity of target prepared, and thus mRNA, expressed in the sample cells. A large number of such techniques are available and useful. Preferred methods for determining gene expression can be found in US Patents 6,271,002; 6,218,122; 6,218,114; and 6,004,755.

In one embodiment, portfolios of genes are established such that a combination of genes in the portfolio exhibit improved sensitivity and specificity relative to individual genes or randomly selected combinations of genes. In the context of the instant invention, the sensitivity of the portfolio is preferably reflected in the fold differences exhibited by a gene's expression in the diseased state relative to the normal state. Specificity is preferably reflected in statistical measurements of the correlation of the signaling of gene expression with the parameter one is interested in determining (cancerous, metastatic, likely to recur, etc.). For example, standard deviation can be a used as such a measurement. In considering a group of genes for inclusion in a portfolio, a small standard deviation in expression measurements correlates with greater specificity. Other measurements of variation such as correlation coefficients can also be used in this capacity. The invention also encompasses the above methods where the specificity is at least about 70%, preferably at least about 80% and more preferably at least about 90%. The invention also encompasses the above methods where the sensitivity is at least about 80% and preferably at least 90%.

Analysis of the expression levels of genes can be conducted by comparing signal intensities of microarrays. This is best done by generating a ratio matrix of the expression intensities of genes in a test sample versus those in a control sample. For instance, the gene expression intensities from a diseased tissue can be compared with the expression intensities generated from benign or normal tissue of the same type. A ratio of these expression intensities indicates the fold-change in gene expression between the test and control samples.

The invention also encompasses the above methods where the comparison of expression patterns is conducted with pattern recognition methods. One method of the invention involves comparing gene expression profiles for various genes (or portfolios) to ascribe diagnoses. The gene expression profiles of each of the genes comprising the portfolio are fixed in a medium such as a computer readable medium. This can take a number of forms. For example, a table can be established into which the range of signals (e.g., intensity measurements) indicative of disease is input. Actual patient data can then be compared to the values in the table to determine whether the patient samples are normal, benign or diseased. In a more sophisticated embodiment, patterns of the expression signals (e.g., fluorescent intensity) are recorded digitally or graphically. The gene expression patterns from the gene portfolios used in conjunction with patient samples are then compared to the expression patterns.

Pattern comparison software can then be used to determine whether the patient samples have a pattern indicative of the disease. Of course, these comparisons can also be used to determine whether the patient is not likely to experience the disease. The expression profiles of the samples are then compared to the portfolio of a control cell. If the sample expression patterns are consistent with the expression pattern for cancer then (in the absence of countervailing medical considerations) the patient is treated as one would treat a gastric cancer patient. If the sample expression patterns are consistent with the expression pattern from the normal/control cell then the patient is diagnosed negative for cancer.

Numerous well-known methods of pattern recognition are available to compare patterns consistent with metastasis to those produced by patient samples. The following references provide some examples: Weighted Voting: Golub et al. (1999); Support Vector Machines: Su et al. (2001); and Ramaswamy et al. (2001); K-nearest Neighbors: Ramaswamy (2001); and Correlation Coefficients: van't Veer et al. (2002).

Preferably, levels of up and down regulation are distinguished based on fold changes of the intensity measurements of hybridized microarray probes. A 1.5 fold difference is preferred for making such distinctions (or a p-value less than 0.05). That is, before a gene is said to be differentially expressed in diseased versus normal cells, the diseased cell is found to yield at least about 1.5 times more, or 1.5 times less intensity than the normal cells. The greater the fold difference, the more preferred is use of the gene as a diagnostic or prognostic tool. Genes selected for the gene expression profiles of this invention have expression levels that result in the generation of a signal that is distinguishable from those of the normal or non-modulated genes by an amount that exceeds background using clinical laboratory instrumentation.

Statistical values can be used to confidently distinguish modulated from non-modulated genes and noise. Statistical tests find the genes most significantly different between diverse groups of samples. The Student's T-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, one is unlikely to see small p-values just by chance and adjustments for this using a Sidak correction as well as a randomization/permutation experiment can be made. A p-value less than 0.05 by the T-test is evidence that the gene is significantly different. More compelling evidence is a p-value less then 0.05 after the Sidak correction is factored in. For a large number of samples in each group, a p-value less than 0.05 after the randomization/permutation test is the most compelling evidence of a significant difference.

The present invention encompasses microarrays or gene chips for performing the methods provided herein. The microarrays can contain isolated nucleic acid sequences, their complements, or portions thereof corresponding to Markers where the combination is sufficient to characterize gastric cancer or risk of relapse in a biological sample. The microarray preferably measures or characterizes at least about 1.5-fold over- or under-expression, provides a statistically significant p-value over- or under-expression, or a p-value is less than 0.05. Preferably, the microarray contains a cDNA array or an oligonucleotide array and may contain one or more internal control reagents.

Gene expression profiles can also be displayed in a number of ways. One of the most common method is to arrange raw fluorescence intensities or ratio matrix into a graphical dendogram where columns indicate test samples and rows indicate genes. The data are arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRING" from Silicon Genetics, Inc. and "DISCOVERY" and "INFER" software from Partek, Inc.

Modulated genes of the invention are described in the Examples. The genes that are differentially expressed are up regulated in lymph nodes with metastasis from gastric carcinoma relative to those lymph nodes without metastasis conditions. Up regulation is a relative term meaning that a detectable difference (beyond the contribution of noise in the system used to measure it) is found in the amount of expression of the genes relative to some baseline. In this case, the baseline is the measured gene expression of makers in normal lymph nodes without metastasis from gastric carcinoma. The genes of interest in the tested lymph node samples are then either up regulated or unchanged relative to the baseline level using the same measurement method. A diagnosis or prognosis includes the determination of disease/status issues such as determining the likelihood of relapse and type of therapy. Determining the likelihood of relapse involves comparing the patterns with patterns associated with known relapse and/or known non-relapsing events or exceeding a cutoff value (usually a Ct in the case of PCR) set for such a distinction.

Genes can be grouped so that information obtained about the set of genes in the group provides a sound basis for making a clinically relevant judgment such as a diagnosis, prognosis, or treatment choice. These sets of genes make up the portfolios of the invention. As with most diagnostic markers, it is often desirable to use the fewest number of markers sufficient to make a correct medical judgment. This prevents a delay in treatment pending further analysis as well unproductive use of time and resources.

One method of establishing gene expression portfolios is through the use of optimization algorithms such as the mean variance algorithm widely used in establishing stock portfolios. This method is described in detail in US patent publication number 20030194734. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application," referred to as "Wagner Software" throughout this specification, is preferred. This software uses functions from the "Wagner Associates Mean-Variance Optimization Library" to determine an efficient frontier and optimal portfolios in the Markowitz sense is preferred. Use of this type of software requires that microarray data be transformed so that it can be treated as an input in the way stock return and risk measurements are used when the software is used for its intended financial analysis purposes.

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with cancer. Output from the method would be an optimized set of genes that could include some genes that are expressed in peripheral blood as well as in diseased tissue. If samples used in the testing method are obtained from peripheral blood and certain genes differentially expressed in instances of cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data preselection.

Other heuristic rules can be applied that are not necessarily related to the biology in question. For example, one can apply a rule that only a prescribed percentage of the portfolio can be represented by a particular gene or group of genes. Commercially available software such as the Wagner Software readily accommodates these types of heuristics. This can be useful, for example, when factors other than accuracy and precision (e.g., anticipated licensing fees) have an impact on the desirability of including one or more genes.

The gene expression profiles of this invention can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., Cancer Antigen 27.29 ("CA 27.29")). In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Alternatively, tissue samples may be taken from areas adjacent to the tissue from which a tumor was previously removed. This approach can be particularly useful when other testing produces ambiguous results.

The present invention encompasses methods of generating a gastric cancer diagnostic patient report and reports obtained thereby, by obtaining a biological sample from the patient; measuring gene expression of the sample; and using the results obtained thereby to generate the report. The report can also contain an assessment of patient outcome and/or probability of risk relative to the patient population.

The present invention encompasses compositions containing at least one microarray probe set or PCR primer/probe set corresponding to Markers.

Articles of this invention include representations of the gene expression profiles useful for treating, diagnosing, prognosticating, and otherwise assessing diseases along with human or machine-readable instructions for their use. These profile representations are reduced to a medium that can be automatically read by a machine such as computer readable media (magnetic, optical, and the like). For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "DISCOVERY" and "INFER" software from Partek, Inc. mentioned above can best assist in the visualization of such data.

Different types of articles of manufacture according to the invention are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the invention can be fashioned into reagent kits for conducting hybridization, amplification, and signal generation indicative of the level of expression of the genes of interest for detecting cancer.

The present invention also encompasses kits for conducting an assay to determine lymph node metastasis from gastric carcinoma. Kits made according to the invention include formatted assays. These can include all or some of the materials needed to conduct the assays such as reagents and instructions and a medium through which nucleic acid sequences, their complements, or portions thereof are assayed. Where the assay is to be conducted in another format, the kit will have components peculiar to that format. For example, if the format is an amplification format such as PCR, Rolling Circle Amplification methods (RCA), Ligase Chain Reaction methods (LCR), Strand Displacement Amplification methods (SDA), Nucleic Acid Sequence Based Amplification methods (NASBA), then the kit will contain reagents and materials appropriate to such technologies.

In one embodiment, the kit includes reagents for amplifying and detecting Markers. Optionally, the kit includes sample preparation reagents and or articles (e.g., tubes) to extract nucleic acids from tissue such as lymph node tissue. The kits may also include articles to minimize the risk of sample contamination (e.g., disposable scalpel and surface for lymph node dissection and preparation).

In a preferred kit, reagents necessary for a one-tube QRT-PCR process are included such as reverse transcriptase, a reverse transcriptase primer, a corresponding PCR primer set (preferably for Markers and controls), a thermostable DNA polymerase, such as Taq polymerase, and a suitable detection reagent(s), such as, without limitation, a scorpion probe, a probe for a fluorescent 5'nuclease assay, a molecular beacon probe, a single dye primer or a fluorescent dye specific to double-stranded DNA, such as ethidium bromide. The primers are preferably in quantities that yield high concentrations. Thermostable DNA polymerases are commonly and commercially available from a variety of manufacturers. Additional materials in the kit may include: suitable reaction tubes or vials, a barrier composition such as a wax bead, optionally including magnesium; reaction mixtures (typically 10X) for the reverse transcriptase and the PCR stages, including necessary buffers and reagents such as dNTPs; nuclease-or RNase-free water; RNase inhibitor; control nucleic acid (s) and/or any additional buffers, compounds, cofactors, ionic constituents, proteins and enzymes, polymers, and the like that may be used in reverse transcriptase and/or PCR stages of QRT-PCR reactions. Optionally, the kits include nucleic acid extraction reagents and materials.

It is also possible to assay the expression products of the gene Markers (themselves included in the definition of a Marker) such as polypeptides and proteins. A useful method for such an assay involves contacting a sample with antibodies directed to the Markers. These antibodies are useful in the development of very specific assays for the detection of Markers, and allow the tests to be carried out in many different formats. Preferably, the antibodies are labeled monoclonal antibodies. If such a Marker is a secreted protein or polypeptide then expressed between 100 and 1000 times in diseased tissues compared with its normal expression levels will be found. Kits for such assays include reagents typical for such assay formats such as labeled antibody reagents.

The following examples are provided to illustrate but not limit the claimed invention. All references cited herein are hereby incorporated by reference herein.

### Example 1 Marker Identification

Freshly frozen samples of 10 gastric carcinomas, 10 normal stomach tissues and 6 histologically confirmed normal lymph nodes were used to identify gene Markers for detection of lymph node metastasis from gastric carcinoma. RNAs were prepared from these tissues with RNeasy Mini Kit (Qiagen, Catlog# 74106) following standard vendor suggested protocols. Biotin labeled aRNA targets were prepared by RNA linear amplification (Ambion, MessageAmp II aRNA Amplification Kit, Catlog# 1751). Prepared targets were applied to Affymetrix HG U133A chips which were processed following standard Affymetrix suggested protocols. Chips results were collected and globally scaled to Target Signal of 600. Hierarchical clustering of microarray results (HG U133A chip set) was conducted and the chip results were statistically analyzed using Partek Pro 6.0. ANOVA and fold change analysis were applied to identify genes of interest. Genes with the following characteristics were selected as candidate markers for detection of lymph node metastasis from gastric carcinoma: a) expressed stably in normal stomach tissues, b) expression is maintained stably in gastric carcinomas, and c) there is a clear distinction in expression levels between the normal lymph node and gastric carcinoma.

Twenty probe sets (representing 18 genes) met the criteria described above. The distinctive difference in gene signal levels between the normal lymph node group and the gastric carcinoma/normal stomach tissues was statistically significant. The microarray results are summarized in Table 1, average signal and standard deviation of each of the 20 probe sets are shown. More information about the Markers are shown in Table 2 and the clustering analysis is shown in Fig. 1 (all probe sets of HG U133A) and Fig. 2 (markers only). The expression of these Markers is increased in lymph nodes in the case of metastasis from gastric carcinoma. The markers are classified by functions as shown in Table 3.

**Table 1. Summary of microarray results**

| | | Average | | | Standard Deviation | | |
|---|---|---|---|---|---|---|---|
| Affymetrix: PSID | Genes | Lymph Node | Normal Stomach | Gastric Carcinoma | Lymph Node | Normal Stomach | Gastric Carcinoma |
| 209369_at | ANXA3 | 150 | 2077 | 1886 | 28 | 645 | 1193 |
| 218261_at | AP1M2 | 55 | 1061 | 735 | 25 | 237 | 446 |
| 201243_s_at | ATP1B1 | 431 | 10762 | 6659 | 76 | 2585 | 3075 |
| 204073_s_at | c11orf9 | 51 | 2642 | 1003 | 30 | 1099 | 630 |
| 219010_at | c1orf106 | 127 | 1780 | 1856 | 28 | 393 | 986 |
| 201131_s_at | CDH1 | 587 | 7333 | 6444 | 254 | 1054 | 4321 |
| 213050_at | COBL | 26 | 709 | 317 | 6 | 172 | 213 |
| 211843_x_at | CYP3A7 | 18 | 788 | 219 | 4 | 349 | 128 |
| 217901_at | DSG2 | 189 | 3077 | 2162 | 113 | 920 | 1445 |
| 210827_s_at | ELF3 | 183 | 2498 | 2282 | 50 | 315 | 1456 |
| 202454_s_at | ERBB3 | 124 | 2649 | 2411 | 32 | 489 | 1472 |
| 219545_at | KCTD14 | 20 | 727 | 362 | 2 | 184 | 201 |
| 209211_at | KLF5 | 52 | 1227 | 1312 | 23 | 266 | 852 |
| 209008_x_at | KRT8 | 158 | 6815 | 7412 | 20 | 1736 | 4803 |
| 202890_at | MAP7 | 31 | 921 | 756 | 22 | 226 | 335 |
| 201468_s_at | NQO1 | 225 | 7017 | 3107 | 57 | 1242 | 1939 |
| 210519_s_at | NQO1 | 235 | 5276 | 3688 | 51 | 1126 | 2123 |
| 201467_s_at | NQO1 | 15 | 1585 | 1076 | 7 | 293 | 678 |
| 219395_at | RBM35B | 78 | 1145 | 867 | 46 | 337 | 530 |
| 211689_s_at | TMPRSS2 | 9 | 1549 | 632 | 5 | 569 | 432 |

**Table 2. Gene Markers and Their Affymetrix Probe Set Information**

| **Gene** | **Gene Name/Description** | **Affymetrix PSID** | **NCBI Reference Sequences (mRNA)** | **Seq. ID No.** |
|---|---|---|---|---|
| ANXA3 | annexin A3 | 209369_at | NM_005139 | 1 |
| AP1M2 | adaptor-related protein complex 1, mu 2 subunit | 218261_at | NM_005498 | 2 |
| ATP1B1 | ATPase, Na+/K+ transporting, beta 1 polypeptide | 201243_s_at | NM_001677, | 3 |
| | | | NM_001001787 | 38 |
| C11orf9 | chromosome 11 open reading frame 9 | 204073_s_at | NM_013279 | 4 |
| C1orf106 | Hypethetical Protein FLJ10901, chromosome 1 open reading frame 106 | 219010_at | NM_018265 | 5 |
| CDH1 | cadherin 1, type 1, E-cadherin (epithelial) | 201131_s_at | NM_004360 | 6 |
| COBL | cordon-bleu homolog (mouse) | 213050_at | NM_015198 | 7 |
| CYP3A7 | cytochrome P450, family subfamily A, polypeptide 7 | 3, 211843_x_at | NM_000765 | 8 |
| DSG2 | desmoglein 2 | 217901_at | NM_001943 | 9 |
| ELF3 | E74-like factor 3 (ets domain transcription factor, epithelial-specific) | 210827_s_at | NM_004433 | 10 |
| ERBB3 | v-erb-b2 erythroblastic leukemia viral oncogene homolog 3 (avian) | 202454_s_at | NM_001982, | 39 |
| | | | NM_001005915 | 11 |
| KCTD14 | potassium tetramerisation domain containing 14 | channel 219545_at | NM_023930 | 12 |
| KLF5 | Kruppel-like factor 5 (intestinal) | 209211_at | NM_001730 | 13 |
| KRT8 | keratin 8 | 209008_x_at | NM_002273 | 14 |
| MAP7 | microtubule-associated protein 7 | 202890_at | NM_003980 | 15 |
| NQO1 | NAD(P)H dehydrogenase, quinone 1 | 201468_s_at | NM_000903 | 16 |
| | | | NM_001025433 | |
| | | | NM_001025434 | |
| NQO1 | NAD(P)H dehydrogenase, quinone 1 | 210519_s_at | NM_000903 | |
| | | | NM_001025433 | 17 |
| | | | NM_001025434 | |
| NQO1 | NAD(P)H dehydrogenase, quinone 1 | 201467_s_at | NM_000903, | |
| | | | NM_001025433, | |
| | | | NM_001025434 | 18 |
| RBM35B | hypothetical protein FLJ21918 | 219395_at | NM_024939 | 19 |
| TMPRSS 2 | transmembrane protease, serine 2 | 211689_s_at | NM_005656 | 20 |

**Table 3. Markers Classified by Functions**

| **Function** | **Number** | **Genes** |
|---|---|---|
| Signal Transduction | 5 | ANXA3, ATP1B1, AP1M2, KCTD14, ERBB3, |
| Transcription Factor | 2 | ELF3, KLF5 |
| Intercellular Junction | 2 | CDH1, DSG2, |
| Unknown | 4 | RBM35B (FLJ21918), COBL, C1orf106 (FLJ10901), C11orf9 |
| Cell Structure | 2 | KRT8, MAP7 |
| Cytoplasmic Enzyme | 3 | NQ01, CYP3A7, TMPRSS2 |
| **Total** | **18** | |

### Example 2: Confirmation Study (1) --- Microarray Test

Confirmation studies were performed to validate the Markers selected in Example 1. These markers were examined to determine whether their expression is maintained in lymph nodes with metastasis from gastric carcinomas of various differentiation statuses. Affymetrix DNA microarrays (HG U133A) were used in this study. Tissue of 6 normal lymph nodes and 3 lymph nodes with confirmed metastasis from gastric carcinoma were tested. RNAs were prepared from these samples as described above. Biotin-labeled aRNA targets were prepared as in Example 1. Targets were applied to Affymetrix HG U133A chips. Results from the chips were scaled to Target Signal of 600 and analyzed.

The average signal of each probe set and standard error are plotted in Fig 3A, Fig 3B.

### Example 3: Confirmation Study (2) - Realtime RT-PCR Test

Studies were conducted to validate the performance of selected gene markers using real time RT-PCR. 10 normal lymph nodes and 16 lymph nodes with histologically confirmed metastasis from gastric carcinoma were tested (including all the samples tested with DNA microarray in Confirmation Study 1). The 10 histologically confirmed normal lymph nodes were from 7 different patients. The 16 lymph nodes with confirmed metastasis from gastric carcinoma were from 13 different patients. The malignancy statuses of the original carcinoma that metastasized to lymph nodes are: 3 well differentiated (contributed to 6 lymph nodes), 7 poorly differentiated (contributed to 7 nodes), 3 moderately differentiated (contributed to 3 lymph nodes).

All RNAs were prepared as described above. cDNAs were first prepared from 5 µg RNA in 100 uL reaction volume with a High Capacity cDNA Archive Kit (ABI) following the vendor suggested protocol. 2 µL of the cDNA that was synthesized was used in the real time PCR reaction (RT-PCR) in a volume of 50 µL. One cDNA was prepared from each RNA sample. TaqMan Gene Expression Assays were purchased from Applied Biosystems Inc (ABI) for each of the gene markers. RT-PCR tests were performed using these assays with TaqMan Universal PCR Master Mix without AmpErase UNG (ABI) under vendor suggested conditions, one reaction (assay) in each tube. Duplicate or triplicate PCR reactions were performed on each cDNA generated. PCR was run on an ABI 7900 HT analyzer.

Assay ID and Context sequences for each assay (one primer/probe set for a gene) are shown in Table 4:

**Table 4. TaqMan Gene Expression Assay (Applied Biosystems, Inc)**

| **Gene** | **ABI Assay ID No.** | **NCBI Reference Seqences (mRNA)** | **Context Sequence** | **Seq. ID of Context Seq.** |
|---|---|---|---|---|
| ANXA3 | Hs00192983_ml | NM_005139 | TCAGAGGAATTGGAACTGATGAGAA | 21 |
| AP1M2 | HS00194014_ml | NM_005498 | AGGGCAAGCCATTGATCAGCCGCAA | 22 |
| ATP1B1 | Hs00426868_gl | NM_001677, NM_00100178 7 | CAGTGCACTGGCAAGCGAGATGAAG | 23 |
| C11orf9 | Hs00973712_g | NM_013279 | GTCATGGCCTTCAGCGTGGTGTCCA | 24 |
| Clorf106 | Hs00250963_ml | NM_018265 | TGGGACAGCTGGGGAAGCTCCAGGC | 25 |
| CDH1 | Hs00170423_ml | NM_004360 | CCCGCCCCATCAGGCCTCCGTTTCT | 26 |
| COBL | Hs00323596_ml | NM_015198 | CTGAGAAAGGTGCCCTTGCTCGTGT | 27 |
| CYP3A7 | Hs02511627_sl | NM_000765 | TTATTTTGATGGCTCTAACAGTGAC | 28 |
| DSG2 | Hs00170071_ml | NM_001943 | CAGAGAGGAACACAGCAGCTACACT | 29 |
| ELF3 | Hs00231786_ml | NM_004433 | CAAGCACGCGCCCAGAGGCACCCAC | 30 |
| ERBB3 | Hs00176538_ml | NM_001982, NM_00100591 5 | GCAACTCTCAGGCAGTGTGTCCTGG | 31 |
| KCTD14 | Hs01928822_sl | NM_023930 | Not available from Vendor | 12 (Full length) |
| KLF5 | Hs00156145_ml | NM_001730 | ACTGCGATTACCCTGGTTGCACAAA | 32 |
| KRT8 | Hs01595539_gl | NM_002273 | Not available from Vendor | 14 (Full length) |
| MAP7 | Hs00187725_ml | NM_003980 | GCGAAGCGAAACAGCACCCGACAGC | 33 |
| NQO1 | Hs00168547_ml | NM_000903 NM_00102543 3 NM_00102543 4 | GAGCCATGGTCGGCAGAAGAGCACT | 34 |
| RBM35B | Hs00227840_ml | NM_024939 | GTGCTGCAGCAGTTCTCACAGCTGG | 35 |
| TMPRSS2 | Hs00237175_ml | NM_005656 | TCGGTGTGTTCGCCTCTACGGACCA | 36 |
| HPRT1 * | 185278564 | NM_000194 | Not available from Vendor | 37 (Full length) |

Ct values were collected for each of the marker genes for samples from normal lymph nodes (NLN) and lymph nodes with confirmed metastasis from gastric carcinoma (MLN). Raw Ct values for each gene were normalized against the Ct of the house keeping gene, HPRT1. Both the raw Ct and the normalized Ct values across multiple RT-PCR Runs were summarized and analyzed. The average raw Ct and standard error of each gene for both sample groups (NLN, MLN) were calculated as shown in Fig.4. The average normalized Ct and standard error of each marker gene for both sample groups (NLN, MLN) were calculated as shown in Fig.5. For the gene CYP3A7, Ct values were undetermined in 16 out of 26 PCR reactions in NLN group, and 2 out of 35 PCR reactions on MLN group, while typical Ct values of the housekeeping (consitutively expressed) gene HPRT1 were obtained from the same cDNA. The PCR detection rates for gene CYP3A7 are significantly different between NLN and MLN groups (P<0.0001). For CYP3A7 , only determined Ct values were used in the calculation of average, standard deviation, standard error and ANOVA. ANOVA was performed on raw and normalized Ct values to test the significance of difference between the two groups (NLN, MLN), as shown in Table 5 and Table 6. There is no case of undetermined Ct value in PCR reactions on genes tested other than CYP3A7.

**Table 5. Data Summary and ANOVA Results on raw Ct values**

| **Genes** | **p-value** | **Mean(MLN)** | **Std(MLN)** | **Min(MLN)** | **Max(MLN)** | **Mean(NLN)** | **Std(NLN)** | **Min(NLN)** | **Max(NLN)** | **MS(model)** | **MS(Error)** |
|---|---|---|---|---|---|---|---|---|---|---|---|
| **ELF3** | 9.4E-43 | 22.2 | 0.9 | 20.7 | 23.8 | 31.2 | 0.9 | 28.3 | 32.0 | 1204 | 0.9 |
| **AP1M2** | 1.8E-42 | 24.0 | 1.0 | 22.0 | 25.8 | 34.7 | 1.3 | 31.1 | 36.4 | 1689 | 1.2 |
| **ERBB3** | 6.6E-41 | 24.1 | 1.0 | 22.7 | 25.9 | 31.3 | 0.5 | 30.4 | 32.5 | 791 | 0.7 |
| **TMPRSS2** | 1.1E-39 | 23.4 | 1.0 | 21.9 | 25.7 | 34.2 | 1.6 | 30.3 | 36.9 | 1713 | 1.6 |
| **KRT8** | 5.5E-36 | 20.6 | 1.5 | 18.0 | 24.1 | 30.3 | 1.1 | 27.7 | 31.9 | 1406 | 1.8 |
| **C11orf9** | 2.4E-32 | 25.5 | 0.8 | 24.3 | 27.0 | 30.7 | 0.4 | 29.7 | 31.0 | 329 | 0.4 |
| **RBM35B(FLJ21918)** | 3.8E-31 | 26.1 | 1.1 | 24.6 | 28.9 | 34.2 | 1.6 | 30.7 | 38.0 | 971 | 1.8 |
| **DSG2** | 2.4E-30 | 24.8 | 1.1 | 22.5 | 27.1 | 30.2 | 0.6 | 28.9 | 31.3 | 435 | 0.9 |
| **C1orf106(FLJ10901)** | 1.4E-29 | 28.5 | 1.0 | 26.6 | 30.5 | 33.4 | 0.8 | 31.9 | 34.5 | 355 | 0.8 |
| **KLF5** | 2.6E-29 | 20.9 | 1.1 | 19.6 | 23.5 | 27.5 | 0.7 | 25.8 | 28.3 | 520 | 0.9 |
| **COBL** | 4.5E-28 | 27.0 | 1.0 | 24.7 | 28.9 | 32.8 | 1.3 | 29.9 | 34.9 | 516 | 1.3 |
| **NQ01** | 3.5E-26 | 22.2 | 1.3 | 19.0 | 23.7 | 28.5 | 1.4 | 26.6 | 30.8 | 592 | 1.7 |
| **ATP1B1** | 7.7E-26 | 20.9 | 0.8 | 19.1 | 22.3 | 24.5 | 0.7 | 23.6 | 25.8 | 195 | 0.6 |
| **ANXA3** | 5.2E-22 | 21.8 | 1.0 | 20.4 | 24.6 | 27.0 | 1.2 | 24.6 | 28.8 | 332 | 1.2 |
| **MAP7** | 2.8E-17 | 25.6 | 1.0 | 24.2 | 27.2 | 29.1 | 1.3 | 26.6 | 30.6 | 180 | 1.3 |
| **CDH1** | 9.8E-16 | 22.0 | 1.4 | 19.8 | 26.3 | 25.3 | 0.8 | 24.5 | 28.5 | 164 | 1.4 |
| **KCTD14** | 1.4E-09 | 27.6 | 1.0 | 25.5 | 29.1 | 29.6 | 1.2 | 26.7 | 31.5 | 62 | 1.2 |
| **CYP3A7*** | 7.1E-01 | 33.4 | 2.6 | 30.6 | 38.7 | 33.1 | 3.6 | 29.5 | 39.6 | 1 | 7.9 |

**Table 6. Data Summary and ANOVA Results on Normalized Ct values**

| **Genes** | **p-value** | **Mean(MLN)** | **Std(MLN)** | **Min(MLN)** | **Max(MLN)** | **Mean(NLN)** | **Std(NLN)** | **Min(NLN)** | **Max(NLN)** | **MS(Model)** | **MS(Error)** |
|---|---|---|---|---|---|---|---|---|---|---|---|
| **AP1M2** | 5.2E-42 | 24.2 | 1.0 | 22.2 | 25.9 | 34.4 | 1.2 | 31.5 | 37.0 | 1559 | 1.2 |
| **ELF3** | 1.5E-40 | 22.4 | 1.1 | 21.2 | 24.5 | 31.0 | 0.8 | 28.7 | 32.5 | 1095 | 0.9 |
| **TMPRSS2** | 7.4E-40 | 23.6 | 1.2 | 21.9 | 25.8 | 33.9 | 1.2 | 30.8 | 36.1 | 1582 | 1.4 |
| **KRT8** | 7.3E-39 | 20.8 | 1.3 | 18.2 | 23.6 | 30.0 | 0.9 | 28.1 | 31.1 | 1288 | 1.3 |
| **ERBB3** | 1.0E-38 | 24.2 | 0.9 | 22.8 | 25.7 | 31.1 | 0.8 | 30.2 | 33.0 | 703 | 0.7 |
| **RBM35B(FLJ21918)** | 5.4E-33 | 26.3 | 0.7 | 25.4 | 28.4 | 33.9 | 1.6 | 31.2 | 38.3 | 874 | 1.4 |
| **KLF** 5 | 1.7E-31 | 21.1 | 0.8 | 19.6 | 22.9 | 27.3 | 0.8 | 26.2 | 29.3 | 475 | 0.6 |
| **C1orf106(FLJ10901)** | 2.1E-31 | 28.7 | 0.8 | 27.1 | 30.2 | 33.1 | 0.6 | 32.2 | 34.5 | 297 | 0.5 |
| **DSG2** | 7.5E-31 | 24.9 | 1.0 | 22.5 | 26.6 | 29.9 | 0.6 | 28.7 | 30.8 | 370 | 0.7 |
| **COBL** | 9.7E-29 | 27.1 | 1.0 | 25.4 | 28.5 | 32.6 | 1.0 | 30.3 | 34.4 | 445 | 1.0 |
| **C11orf9** | 6.9E-26 | 25.6 | 0.9 | 23.9 | 27.3 | 30.5 | 0.6 | 29.7 | 31.8 | 293 | 0.7 |
| **ANXA3** | 1.0E-20 | 21.9 | 1.3 | 20.1 | 25.6 | 26.8 | 0.7 | 25.6 | 27.8 | 296 | 1.2 |
| **NQ01** | 1.7E-20 | 22.4 | 1.5 | 19.1 | 24.7 | 28.3 | 1.7 | 26.9 | 31.8 | 517 | 2.6 |
| **ATP1B1** | 4.9E-18 | 21.1 | 1.0 | 19.1 | 22.4 | 24.3 | 1.0 | 23.1 | 26.7 | 153 | 1.0 |
| **MAP7** | 3.5E-17 | 25.8 | 0.9 | 24.1 | 27.7 | 28.9 | 1.1 | 25.9 | 30.0 | 139 | 1.0 |
| **CDH1** | 5.8E-13 | 22.1 | 1.5 | 20.1 | 26.2 | 25.0 | 0.7 | 24.2 | 28.0 | 125 | 1.5 |
| **KCTD14** | 5.5E-07 | 27.7 | 1.1 | 25.1 | 29.5 | 29.4 | 1.1 | 25.7 | 30.8 | 39 | 1.2 |
| **CYP3A7*** | 0.82 | 33.5 | 3.0 | 29.9 | 39.7 | 33.3 | 3.9 | 28.5 | 40.0 | 0.5 | 10.2 |

Except for CYP3A7 (undetermined Ct observed in many PCR reactions), significantly increased expression of the 17 Markers were observed in all of the 16 lymph nodes with metastasis from gastric carcinoma of various differentiation states (from 13 patients). The background expression levels of these Marker genes are consistent among the 10 normal lymph nodes tested, which are significantly lower than that of lymph nodes with metastasis from gastric carcinoma. Receiver Operating Characteristic (ROC) curves were generated for each of the 17 Markers (excluding CYP3A7) to show the potential of them to be used in discriminative assays against the two sample groups (MLN, NLN). A summary of the analysis of these curves is shown in Table 7. AUC (Area Under Curve) for each of the gene markers was calculated. Two Cut-off Cts were tested, and the respective sensitivity and specificity for each Cut-off Ct value are shown in Table7.

**Table 7. Summary of ROC Curve for each Gene Marker Ct results**

| | | Situation A | | | Situation B | | |
|---|---|---|---|---|---|---|---|
| **Gene** | **ROC-AUC** | **Cutoff Ct #1** | **Sensitivity #1** | **Specificity #1** | **Cutoff Ct #2** | **Sensitivity #2** | **Specificity #2** |
| DSG2 | 1 | 28.026 | 1.00 | 1.00 | 29.48 | 1.00 | 0.89 |
| RBM35B | 1 | 29.82 | 1.00 | 1.00 | 32.64 | 1.00 | 0.89 |
| COBL | 1 | 29.40 | 1.00 | 1.00 | 31.27 | 1.00 | 0.89 |
| ELF3 | 1 | 26.06 | 1.00 | 1.00 | 30.71 | 1.00 | 0.89 |
| AP1M2 | 1 | 28.41 | 1.00 | 1.00 | 33.63 | 1.00 | 0.89 |
| NQO1 | 1 | 25.17 | 1.00 | 1.00 | 27.08 | 1.00 | 0.89 |
| C1orf106 | 1 | 31.20 | 1.00 | 1.00 | 32.17 | 1.00 | 0.89 |
| ATP1B1 | 1 | 22.90 | 1.00 | 1.00 | 23.66 | 1.00 | 0.89 |
| ERBB3 | 1 | 28.16 | 1.00 | 1.00 | 30.62 | 1.00 | 0.89 |
| C11orf9 | 1 | 28.35 | 1.00 | 1.00 | 30.05 | 1.00 | 0.90 |
| KLF5 | 1 | 24.65 | 1.00 | 1.00 | 26.61 | 1.00 | 0.90 |
| KRT8 | 1 | 25.90 | 1.00 | 1.00 | 29.035 | 1.00 | 0.89 |
| TMPRSS2 | 1 | 27.98 | 1.00 | 1.00 | 32.70 | 1.00 | 0.89 |
| ANXA3 | 0.998 | 24.40 | 0.97 | 1.00 | 25.30 | 1.00 | 0.90 |
| MAP7 | 0.974 | 26.545 | 0.714 | 1.00 | 27.03 | 0.97 | 0.89 |
| CDH1 | 0.945 | 23.83 | 0.94 | 1.00 | 24.69 | 0.94 | 0.89 |
| KCTD14 | 0.898 | 26.52 | 0.17 | 1.00 | 28.30 | 0.69 | 0.89 |

### Example 4: Antibodies (Prophetic)

C-terminal Markers-derived peptides are synthesized, coupled to keyhole limpet hemocyanin, and used to immunize rabbits for production of polyclonal antibodies. The sera are tested for reactivity against the corresponding peptide with ELISA, and the positive batches are affinity-purified. The purified antibody specifically detects the protein that has the peptide epitope in tissue sections. This is verified by complete abolishment of the signal if the corresponding peptide is added simultaneously with the antibody. In addition to this polyclonal antibody, which works well in immunohistochemistry, monoclonal antibodies able to detect the protein in its natural fold are produced. To produce monoclonal antibodies, a purified antigen, produced in mammalian cells to ensure natural fold and posttranslational modifications, are generated. The antigen, Markers-IgG constant part fusion protein, is expressed in mouse myeloma cells, and the secreted protein is purified using the Fc part as bait. This purified antigen is recognized in Western blot by the C-terminal polyclonal antibody, and by other anti-Markers peptide antibodies. The antigen is used to generate mouse monoclonal antibodies against Markers by selecting out of the positive clones those that produced antibodies that react against Markers instead of the IgG constant part.

Kits for the clinical identification of Markers can be readily fashioned employing these and similar antibodies. Such kits would include antibodies directed to Markers identification, appropriate indicator reagents (e.g., enzymes, labels, and the like), and (optionally) other reagents useful in the clinical application of such a kit such as dilution buffers, stabilizers, and other materials typically used in such assays. The kits would be used to detect Markers in body fluids to screen or follow-up Markers expressing cancers, and to screen the presence of Markers protein in tissue samples. Kits that include antibodies as described are also made to conduct immunocytochemistry assays for the tissue of interest. These kits include positive staining for the Marker protein to indicate metastasis.

### Example 5: Enzyme Immunoassay (Prophetic)

Immunoassays are prepared for the Markers. To develop an Enzyme Immunoassay (EIA) procedure, antigen standards comprising a digest of stomach tumor specimens (shown to contain the antigen by immunoperoxidase staining) are used. Human primary stomach cancer specimens are pooled and homogenized in 10 volumes of 10 mM Tris buffer, pH 7.4, containing 0.2% (w/v) sodium deoxycholate at 4C. The homogenate is quickly brought to 37 C and the following reagents (final concentration) are added while stirring: 1 mM cysteine (Sigma), 1 mM EDTA (Sigma), and papain (0.8 unit/ml) (Boehringer-Mannheim, Indianapolis, Ind.). After 5 minutes, digestion is stopped by the addition of 5 mM iodoacetamide (Sigma). The homogenate is centrifuged at 100,000Xg for 1 hour at 4C, then extensively dialyzed against 10 mM Tris/0.9% NaCl solution buffer, pH 7.4, containing phenylmethysulfonyl fluoride and aminocaproic acid, each at 10 mM. The homogenate is frozen in small aliquots at a concentration of 0.5 mg of protein/mi.

The dose response curve that will be generated for the immunoassay procedure measuring Markers will demonstrate linearity at clinically significant low levels of concentration.

Solid-phase preparations of the antibodies described in Example 6 are prepared using CNBr-activated Sepharose (Pharmacia). Microtiter plates (Nunc I Immunoplates; Grand Island Biological Co., Grand Island, N.Y.) are coated with the antibodies (200 µl /well) in 50 mM carbonate-bicarbonate buffer, pH 9.6, for 18 hours at 4C. After removal of the antibody solution, residual protein binding sites on the plastic are blocked by the addition of 200 µl of assay buffer [PBS containing 1% (v/v) rabbit *serum* and 1% (w/v) bovine albumin]. After 1 hour of incubation at room temperature, the coated plates are used immediately for the assay procedure.

To perform the assay, samples, diluted in assay buffer, are applied for 1-5 hours at 37C. After 3 washes using assay buffer, 200 µl of the antibody covalently conjugated to horseradish peroxidase (Sigma, Type VI) is applied to each well for 1.5 hours at 37C. The conjugate is diluted to a concentration of 0.5 µg of immunoglobulin per ml of PBS containing 10% (v/v) murine serum. Following a wash procedure as above, 200 µl of substrate per well are applied for 0.5 hours at room temperature. Substrate solution contains 0.4 mg of o-phenylenediamine per ml of pH 5.0 citrate buffer and 0.003% hydrogen peroxide. The reaction is stopped by addition of 50 µl of 2N sulfuric acid, and absorbance is monitored at 488 nM using an enzyme assay plate reader (Fisher Scientific Co., Pittsburgh, Pa.).

The percentage of bound enzyme conjugate is calculated by the formula: (B-B₀)(Bₜ -B₀) ( 100)
where B=absorbance of the sample, Bₜ =maximal absorbance, and B₀ =absorbance of the blank. Each assay is performed in triplicate using a standard digest and 26-fold diluted serum samples diluted in assay buffer. Specificity of the immunoassay is examined by substituting various antibody reagents at the solid phase, including an antibody to CEA and nonimmune rabbit serum.

Samples obtained from patients with cancers corresponding to those described above are also evaluated. The incidence of elevated Markers values is 90%. Mean values from the group with cancer are significantly higher than control levels (about 250% higher).

Using a limited number of postoperative stomach cancer patients with primary localized disease, a significant decrease in Markers occurs. These data indicate a relationship between Markers levels and tumor load. Such measurements are thus valuable for patient monitoring.

### References:

Ajisaka, H. and K. Miwa (2003). "Micrometastases in sentinel nodes of gastric cancer." Br J Cancer 89(4): 676-80.
Chausovsky, G., M. Luchansky, et al. (1999). "Expression of cytokeratin 20 in the blood of patients with disseminated carcinoma of the pancreas, colon, stomach, and lung." Cancer 86(11): 2398-405.
Chen, X. M., G. Y. Chen, et al. (2004). "Detection of micrometastasis of gastric carcinoma in peripheral blood circulation." World J Gastroenterol 10(6): 804-8.
Hartgrink, H. H. and C. J. van de Velde (2005). "Status of extended lymph node dissection: locoregional control is the only way to survive gastric cancer." J Surg Oncol 90(3): 153-65.
Heeren, P. A., W. Kelder, et al. (2005). "Prognostic value of nodal micrometastases in patients with cancer of the gastro-oesophageal junction." Eur J Surg Oncol 31 (3): 270-6.
Jemal, A., T. Murray, et al. (2005). "Cancer statistics, 2005." CA Cancer J Clin 55(1): 10-30.
Kim, T. M., H. J. Jeong, et al. (2005). "Determination of genes related to gastrointestinal tract origin cancer cells using a cDNA microarray." Clin Cancer Res 11(1): 79-86.
Kitagawa, Y., H. Fujii, et al. (2005). "Recent advances in sentinel node navigation for gastric cancer: a paradigm shift of surgical management." J Surg Oncol 90(3): 147-51; discussion 151-2.
Kumar, V., R. S. Cotran, et al. (2003). Basic Pathology. Philadelphia, Saunders.
Matsuda, J., Y. Kitagawa, et al. (2004). "Significance of metastasis detected by molecular techniques in sentinel nodes of patients with gastrointestinal cancer." Ann Surg Oncol 11 (3 Suppl): 250S-4S.
Okada, Y., Y. Fujiwara, et al. (2001). "Genetic detection of lymph node micrometastases in patients with gastric carcinoma by multiple-marker reverse transcriptase-polymerase chain reaction assay." Cancer 92(8): 2056-64.
Parkin, D. M., F. Bray, et al. (2001). "Estimating the world cancer burden: Globocan 2000." Int J Cancer 94(2): 153-6.
Parkin, D. M., F. I. Bray, et al. (2001). "Cancer burden in the year 2000. The global picture." Eur J Cancer 37 Suppl 8: S4-66.
Shibuya, K., C. D. Mathers, et al. (2002). "Global and regional estimates of cancer mortality and incidence by site: II. Results for the global burden of disease 2000." BMC Cancer 2: 37.
Songun, I., J. J. Bonenkamp, et al. (1996). "Prognostic value of resection-line involvement in patients undergoing curative resections for gastric cancer." Eur J Cancer 32A(3): 433-7.

## Claims

1. A method of diagnosing lymph node metastasis from gastric carcinoma comprising, measuring Marker levels in a biological sample obtained from a patient, wherein the levels above the pre-determined cut-off levels are indicative of lymph node metastasis from gastric carcinoma.

2. The method of claim 1, further comprising the use of another diagnostic method.

3. The method of claim 2, wherein the other diagnostic method comprises conducting a serum assay.

4. A method of determining or adjusting therapy for a gastric carcinoma patient comprising, measuring the levels of a Marker in a biological sample from a gastric cancer patient, wherein levels above or below cut-off levels are consistent with a course of disease or physical condition for which a therapy is determined or adjusted.

5. The method of any one of the preceding claims, wherein the Marker is a gene, a gene fragment, or the complement thereof.

6. The method of any one of the preceding claims, wherein the Marker is a protein, protein fragment, or polypeptide.

7. The method of any one of the preceding claims, further comprising the measurement of a consitutively expressed gene.

8. The method of any one of the preceding claims, wherein the specificity is at least 70%.

9. The method of any one of the preceding claims, wherein the sensitivity is at least 80%.

10. The method of any one of the preceding claims, conducted with a microarray.

11. The method of any one of the preceding claims, conducted by using an amplification process.

12. The method of claim 11, wherein the amplification process is a PCR.

13. The method of claim 12, wherein the PCR s an RT-PCR.

14. A kit for conducting an assay to determine gastric cancer diagnosis in a biological sample comprising materials for detecting a Marker.

15. The kit of claim 14 including a microarray and reagents.

16. The kit of claim 14 including PCR reagents.

17. The kit of claim 14 including instructions.
